# EUROPEAN PATENT APPLICATION

(11) **EP 2 100 966 A1**
(43) Date of publication of application: **16.09.2009**
(21) Application number: 08290238.8
(22) Date of filing: 12.03.2008
(51) Int. Cl.: C12P 19/12, C12P 19/26, C07H 15/10

(54) **Mutants of glycoside hydrolases and uses thereof for synthesizing complex oligosaccharides and disaccharide intermediates**

(71) Applicant: Institut Pasteur, 75724 Paris Cedex 15 (FR)
(72) Inventor: Mulard, Laurence, 94270 Le Kremlin Bicetre (FR); Andre, Isabelle, 31400 Toulouse (FR); Champion, Elise, 31400 Toulouse (FR); Moulis, Claire, 31380 Garidech (FR); Morel, Sandrine, 31320 Auzeville (FR); Monsan, Pierre, 31700 Mondonville (FR); Remaud-Siméon, Magali, 31520 Ramonville (FR); Boutet, Julien, 44770 La Plaine Sur Mer (FR)
(74) Representative: Rançon, Xavier Lucien Abel

(57) **Abstract**

Method for preparing the disaccharide [α-D-Gle*p*(1→3)]-α-L-Rha*p-*YR wherein Y is selected from -O- and -S- and R is selected from the group consisting of: C₁-C₆ alkyl, C₁-C₆ alkenyl, aryl, allyl, -CO-alkyl (C₁-C₆), -CO-alkenyl (C₁-C₆), -CO-aryl, comprising the step of using a mutant of a wild type glycoside hydrolase.

SEQUENCE LISTING

<110>  Institut Pasteur

<120>  MUTANTS OF GLYCOSIDE HYDROLASES AND USES THEREOF FOR SYNTHESIZING
        COMPLEX OLIGOSACCHARIDES AND DISACCHARIDE INTERMEDIATES

<130>  F226EP150

<160>  27

<170>  PatentIn version 3.3

<210>  1
<211>  11
<212>  PRT
<213>  Artificial

<220>
<223>  Consenus motif (1)

<400>  1

Leu Gly Val Asn Tyr Leu His Leu Met Pro Leu
1               5                   10


<210>  2
<211>  6
<212>  PRT
<213>  Artificial

<220>
<223>  Consensus motif (2)

<400>  2

Asp Gly Gly Tyr Ala Val
1               5


<210>  3
<211>  6
<212>  PRT
<213>  Artificial

<220>
<223>  Consenus motif (3)

<400>  3

Asp Phe Val Phe Asn His
1               5


<210>  4
<211>  13
<212>  PRT
<213>  Artificial

<220>
<223>  Consenus motif (4)

<400>  4

Leu Arg Glu Ile Phe Pro Asp Thr Ala Pro Gly Asn Phe
1               5                   10

<210> 5
<211> 9
<212> PRT
<213> Artificial

<220>
<223> Consensus motfi (5)

<400> 5

Phe Asn Ser Tyr Gln Trp Asp Leu Asn
1               5


<210> 6
<211> 12
<212> PRT
<213> Artificial

<220>
<223> Consensus motif (6)

<400> 6

Ile Leu Arg Leu Asp Ala Val Ala Phe Leu Trp Lys
1               5                   10


<210> 7
<211> 4
<212> PRT
<213> Artificial

<220>
<223> Consenus motif (7)

<400> 7

Glu Ala Ile Val
1


<210> 8
<211> 8
<212> PRT
<213> Artificial

<220>
<223> Consenus motif (8)

<400> 8

Tyr Val Arg Cys His Asp Asp Ile
1               5


<210> 9
<211> 11
<212> PRT
<213> Artificial

<220>
<223> Consensus motif (9)

<400> 9

Arg Ile Ser Gly Thr Leu Ala Ser Leu Ala Gly

1               5                   10


<210>  10
<211>  10
<212>  PRT
<213>  Artificial

<220>
<223>  Consensus motif (9)

<400>  10

Gly Ile Pro Leu Ile Tyr Leu Gly Asp Glu
1               5                   10


<210>  11
<211>  6
<212>  PRT
<213>  Artificial

<220>
<223>  Consenus motif (10)

<400>  11

Arg Trp Val His Arg Pro
1               5


<210>  12
<211>  85
<212>  PRT
<213>  Artificial

<220>
<223>  Consensus sequence

<400>  12

Leu Gly Val Asn Tyr Leu His Leu Met Pro Leu Asp Gly Gly Tyr Ala
1               5                   10                  15


Val Asp Phe Val Phe Asn His Leu Arg Glu Ile Phe Pro Asp Thr Ala
            20                  25                  30


Pro Gly Asn Phe Phe Asn Ser Tyr Gln Trp Asp Leu Asn Ile Leu Arg
        35                  40                  45


Leu Asp Ala Val Ala Phe Leu Trp Lys Glu Ala Ile Val Tyr Val Arg
    50                  55                  60


Cys His Asp Asp Ile Gly Ile Pro Leu Ile Tyr Leu Gly Asp Glu Arg
65                  70                  75                  80


Trp Val His Arg Pro
                85


<210>  13
<211>  628
<212>  PRT

<213>  Neisseria polysaccharea

<400>  13

Ser Pro Asn Ser Gln Tyr Leu Lys Thr Arg Ile Leu Asp Ile Tyr Thr
1               5                   10                  15

Pro Glu Gln Arg Ala Gly Ile Glu Lys Ser Glu Asp Trp Arg Gln Phe
            20                  25                  30

Ser Arg Arg Met Asp Thr His Phe Pro Lys Leu Met Asn Glu Leu Asp
        35                  40                  45

Ser Val Tyr Gly Asn Asn Glu Ala Leu Leu Pro Met Leu Glu Met Leu
    50                  55                  60

Leu Ala Gln Ala Trp Gln Ser Tyr Ser Gln Arg Asn Ser Ser Leu Lys
65                  70                  75                  80

Asp Ile Asp Ile Ala Arg Glu Asn Asn Pro Asp Trp Ile Leu Ser Asn
                85                  90                  95

Lys Gln Val Gly Gly Val Cys Tyr Val Asp Leu Phe Ala Gly Asp Leu
            100                 105                 110

Lys Gly Leu Lys Asp Lys Ile Pro Tyr Phe Gln Glu Leu Gly Leu Thr
        115                 120                 125

Tyr Leu His Leu Met Pro Leu Phe Lys Cys Pro Glu Gly Lys Ser Asp
    130                 135                 140

Gly Gly Tyr Ala Val Ser Ser Tyr Arg Asp Val Asn Pro Ala Leu Gly
145                 150                 155                 160

Thr Ile Gly Asp Leu Arg Glu Val Ile Ala Ala Leu His Glu Ala Gly
                165                 170                 175

Ile Ser Ala Val Val Asp Phe Ile Phe Asn His Thr Ser Asn Glu His
            180                 185                 190

Glu Trp Ala Gln Arg Cys Ala Ala Gly Asp Pro Leu Phe Asp Asn Phe
        195                 200                 205

Tyr Tyr Ile Phe Pro Asp Arg Arg Met Pro Asp Gln Tyr Asp Arg Thr
    210                 215                 220

Leu Arg Glu Ile Phe Pro Asp Gln His Pro Gly Gly Phe Ser Gln Leu
225                 230                 235                 240

Glu Asp Gly Arg Trp Val Trp Thr Thr Phe Asn Ser Phe Gln Trp Asp
                245                 250                 255

Leu Asn Tyr Ser Asn Pro Trp Val Phe Arg Ala Met Ala Gly Glu Met
            260                 265                 270

Leu Phe Leu Ala Asn Leu Gly Val Asp Ile Leu Arg Met Asp Ala Val
        275                 280                 285

Ala Phe Ile Trp Lys Gln Met Gly Thr Ser Cys Glu Asn Leu Pro Gln
    290                 295                 300

Ala His Ala Leu Ile Arg Ala Phe Asn Ala Val Met Arg Ile Ala Ala
305                 310                 315                 320

Pro Ala Val Phe Phe Lys Ser Glu Ala Ile Val His Pro Asp Gln Val
                325                 330                 335

Val Gln Tyr Ile Gly Gln Asp Glu Cys Gln Ile Gly Tyr Asn Pro Leu
            340                 345                 350

Gln Met Ala Leu Leu Trp Asn Thr Leu Ala Thr Arg Glu Val Asn Leu
        355                 360                 365

Leu His Gln Ala Leu Thr Tyr Arg His Asn Leu Pro Glu His Thr Ala
    370                 375                 380

Trp Val Asn Tyr Val Arg Ser His Asp Asp Ile Gly Trp Thr Phe Ala
385                 390                 395                 400

Asp Glu Asp Ala Ala Tyr Leu Gly Ile Ser Gly Tyr Asp His Arg Gln
                405                 410                 415

Phe Leu Asn Arg Phe Phe Val Asn Arg Phe Asp Gly Ser Phe Ala Arg
            420                 425                 430

Gly Val Pro Phe Gln Tyr Asn Pro Ser Thr Gly Asp Cys Arg Val Ser
        435                 440                 445

Gly Thr Ala Ala Ala Leu Val Gly Leu Ala Gln Asp Asp Pro His Ala
    450                 455                 460

Val Asp Arg Ile Lys Leu Leu Tyr Ser Ile Ala Leu Ser Thr Gly Gly
465                 470                 475                 480

Leu Pro Leu Ile Tyr Leu Gly Asp Glu Val Gly Thr Leu Asn Asp Asp
                485                 490                 495

Asp Trp Ser Gln Asp Ser Asn Lys Ser Asp Asp Ser Arg Trp Ala His
            500                 505                 510

Arg Pro Arg Tyr Asn Glu Ala Leu Tyr Ala Gln Arg Asn Asp Pro Ser
        515                 520                 525

Thr Ala Ala Gly Gln Ile Tyr Gln Asp Leu Arg His Met Ile Ala Val
    530                 535                 540

Arg Gln Ser Asn Pro Arg Phe Asp Gly Gly Arg Leu Val Thr Phe Asn
545                 550                 555                 560

Thr Asn Asn Lys His Ile Ile Gly Tyr Ile Arg Asn Asn Ala Leu Leu
                565                 570                 575

Ala Phe Gly Asn Phe Ser Glu Tyr Pro Gln Thr Val Thr Ala His Thr
            580                 585                 590

Leu Gln Ala Met Pro Phe Lys Ala His Asp Leu Ile Gly Gly Lys Thr
        595                 600                 605

Val Ser Leu Asn Gln Asp Leu Thr Leu Gln Pro Tyr Gln Val Met Trp
    610                 615                 620

Leu Glu Ile Ala
625

<210>  14
<211>  29
<212>  DNA
<213>  Artificial

<220>
<223>  primer

<220>
<221>  misc_feature
<222>  (13)..(15)
<223>  n is a, c, g, or t

<400>  14
accctgcgcg aannnttccc cgaccagca                                        29

<210>  15
<211>  29
<212>  DNA
<213>  Artificial

<220>
<223>  primer

<220>
<221>  misc_feature
<222>  (15)..(17)
<223>  n is a, c, g, or t

<400>  15
tgctggtcgg ggaannnttc gcgcagggt                                        29

<210>  16
<211>  33
<212>  DNA
<213>  Artificial

<220>
<223>  primer


<220>
<221>  misc_feature
<222>  (14)..(16)
<223>  n is a, c, g, or t

<400>  16
tatggatgcg gttnnnttta tttggaaaca aat                                    33


<210>  17
<211>  33
<212>  DNA
<213>  Artificial

<220>
<223>  primer


<220>
<221>  misc_feature
<222>  (18)..(20)
<223>  n is a, c, g, or t

<400>  17
atttgtttcc aaataaannn aaccgcatcc ata                                    33


<210>  18
<211>  33
<212>  DNA
<213>  Artificial

<220>
<223>  primer


<220>
<221>  misc_feature
<222>  (17)..(19)
<223>  n is a, c, g, or t

<400>  18
tatggatgcg gttgccnnna tttggaaaca aat                                    33


<210>  19
<211>  33
<212>  DNA
<213>  Artificial

<220>
<223>  primer


<220>
<221>  misc_feature
<222>  (15)..(17)
<223>  n is a, c, g, or t

<400>  19
atttgtttcc aaatnnnggc aaccgcatcc ata                                    33

<210>  20
<211>  34
<212>  DNA
<213>  Artificial

<220>
<223>  primer


<220>
<221>  misc_feature
<222>  (15)..(17)
<223>  n is a, c, g, or t

<400>  20
tcaaatccga agccnnngtc caccccgacc aagt                                    34


<210>  21
<211>  34
<212>  DNA
<213>  Artificial

<220>
<223>  primer


<220>
<221>  misc_feature
<222>  (21)..(23)
<223>  n is a, c, g, or t

<400>  21
acttggtcgg ggtggacgat nnnttcggat ttga                                    34


<210>  22
<211>  34
<212>  DNA
<213>  Artificial

<220>
<223>  primer


<220>
<221>  misc_feature
<222>  (18)..(20)
<223>  n is a, c, g, or t

<400>  22
tcaaatccga agccatcnnn caccccgacc aagt                                    34


<210>  23
<211>  34
<212>  DNA
<213>  Artificial

<220>
<223>  primer


<220>
<221>  misc_feature
<222>  (15)..(17)
<223>  n is a, c, g, or t

<400>  23
acttggtcgg ggtgnnngat ggcttcggat ttga                                    34


<210>  24
<211>  32
<212>  DNA
<213>  Artificial

<220>
<223>  primer


<220>
<221>  misc_feature
<222>  (15)..(17)
<223>  n is a, c, g, or t

<400>  24
tccgcagcca cgacnnnatc ggctggacgt tt                                      32


<210>  25
<211>  32
<212>  DNA
<213>  Artificial

<220>
<223>  primer


<220>
<221>  misc_feature
<222>  (16)..(18)
<223>  n is a, c, g, or t

<400>  25
aaacgtccag ccgatnnngt cgtggctgcg ga                                      32


<210>  26
<211>  27
<212>  DNA
<213>  Artificial

<220>
<223>  primer


<220>
<221>  misc_feature
<222>  (13)..(15)
<223>  n is a, c, g, or t

<400>  26
acaggcgact gcnnngtcag tggtaca                                            27


<210>  27
<211>  27
<212>  DNA
<213>  Artificial

<220>
<223>  primer


<220>

<221>  misc_feature
<222>  (13)..(15)
<223>  n is a, c, g, or t

<400>  27
tgtaccactg acnnngcagt cgcctgt                                          27

## Description

The present invention relates to mutants of glycoside hydrolases and uses thereof in chemo-enzymatic synthesis of complex oligosaccharides, in particular fragments of *S*. *flexneri* 2b, 3a, 5a, 5b, and X O-antigens.

Carbohydrates displayed at the surface of cells and pathogens are involved in a wide range of biological processes, among which several intercellular recognition events, as well as host-pathogen interactions possibly resulting in microbial or viral infections. The understanding of the molecular events involved in carbohydrate-mediated interactions has long been impaired by the difficult access to relevant oligosaccharides and glycoconjugates in pure form and sufficient amounts. The exquisite diversity of possible structures, varying in monosaccharide composition, linkage and branching pattern (ref. 1), is indeed a major roadblock to easy availability. In recent years however, important developments in the preparation of carbohydrate derivatives, based on (i) multistep chemical synthesis, (ii) enzymatic strategies using recombinant glycosyltransferases (*i.e.*, enzymes catalyzing the transfer of a monosaccharide residue from an activated sugar phosphate to an acceptor molecule; EC 2.4) with *in situ* regeneration of sugar nucleotides, (iii) combinations thereof, or (iv) biosynthesis using metabolically engineered cell-factories (ref. 2), have opened the way to significant progress in the fields of glycobiology and glycotherapeutics (ref. 3-5). A number of efficient and elegant synthetic methods such as one-pot oligosaccharide synthesis (ref. 6) or automated synthetic tools (ref. 7) have been developed to provide more straightforward access to structurally-defined carbohydrates. The use of lightly protected precursors and the regioselective one-pot protection of monosaccharides were recently emphasized (ref. 8).

Nevertheless, despite accomplished advancements, chemical approaches towards specific usable microbial oligosaccharides still need considerable effort. They remain, for the most part, highly dependent on the design of appropriate combinations of multiple protection, deprotection, and efficient glycosylation steps, which often involve numerous tedious chromatographic separations (ref. 9). Avoiding the need for protecting groups, organisms engineered to express several glycosyltransferase genes have been used to produce a nice range of biologically active complex carbohydrates, but they remain to date limited to the synthesis of short oligosaccharides which can passively cross the cell membrane (ref. 2, 10).

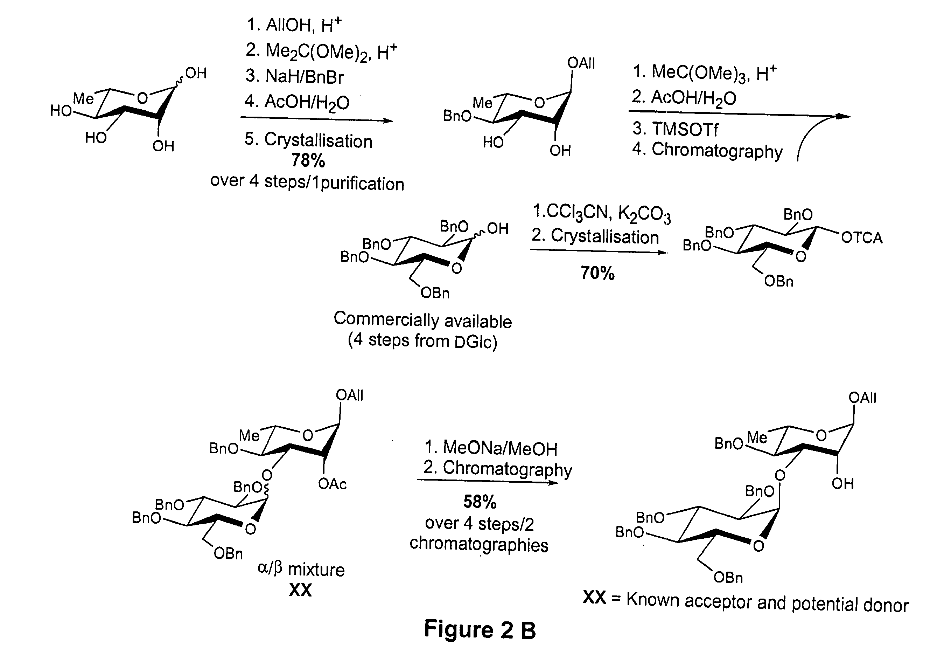

Following the early success of polysaccharide vaccines in the second half of the 20^{th} century, polysaccharide-protein conjugate vaccines were seen as a major progress in antibacterial vaccination (ref. 11, 12). Indeed, made from bacterial polysaccharides purified from pathogen cell cultures, eventually shortened following partial-chemical hydrolysis or enzymatic depolymerisation of the native antigen, and subsequently covalently coupled to a protein carrier, these second generation carbohydrate vaccines are suitable for use in human (ref. 12). Potential extrapolations are numerous since for a large number of pathogens, surface carbohydrates behave as key "protective antigens". Interestingly, this long known property of a range of bacterial polysaccharides was extended in recent years to other microbial carbohydrates of fungal (ref. 13) and parasitic origin (ref. 14). Besides, the disclosure that cancer cells could among other features be differentiated from healthy ones by the presence of surface glycoconjugates, often termed tumor-associated carbohydrate antigens, contributed to additional interest in carbohydrate antigens (ref. 15). Overall, interest in synthetic carbohydrate-based vaccines has emerged as one amongst the many exploding fields of carbohydrate medical applications (ref. 15, 16). The development of synthetic microbial carbohydrate-protein conjugates, thus termed third generation carbohydrate vaccines, was proposed as an alternative to conventional polysaccharide vaccines, compatible with the increasingly demanding requirements in terms of safety, efficiency, product definition, and needs for multivalent vaccines (ref. 17). Most interestingly, use of the natural polysaccharide, and consequently risks associated to antigens of biological origin, are avoided. However, chemical synthesis of carbohydrate-protein conjugates, more precisely of appropriate carbohydrate haptens is seen as a drawback. By way of example, Figure 2B shows the first steps of a known chemical synthetic route to fragments of *S*. *flexneri* 5a O-antigen (ref. 61). Construction of the α-D-Glc*p*(1→3)-α-L-Rha*p* glycosidic linkage, a 1,2-cis-glucosidic linkage, by chemical means was found the most demanding of all glycosylation steps involved in the overall process, due to the lack of any possible anchimeric assistance, a feature which was not observed for 1,2-*trans* linkages present in the linear backbone. Consequently, a poorly separable mixture of α-D- and β-D-glucosylation products is always obtained, resulting in time-consuming purifications and lower yields of the target isomer.

The use of enzymes as catalysts has emerged as a practical alternative to a number of limitations encountered in chemical synthesis (ref. 18, 19). Leloir-type glycosyltransferases and transglycosidases constitute the two major classes of enzymes that can be used for the synthesis of glycosidic linkages. Both are enzymes transferring a glycosyl group from a donor to an acceptor. Glycosyltransferases require nucleotide sugar as donor substrate whereas glycosidases (also called glycoside hydrolases) usually employ mono- and/or oligosaccharides as donor substrates.

The term "donor" refers to a molecule that provides a glycosyl moiety which will be transferred to an acceptor molecule.

The term "acceptor" refers to a molecule that will receive the glycosyl moiety through the formation of a chemical bond, preferentially C-O-linkage.

However, despite the increasing number of available transglycosidases and glycosyltransferases (Leloir type), the lack of appropriate enzymatic tools with requisite substrate specificity has prevented extensive exploration of chemo-enzymatic strategies when dealing with complex bacterial carbohydrate antigens. The use of multiple overexpressed native glycosyltransferases was shown to be highly rewarding for the synthesis of the upstream pentasaccharide terminus of the *Neisseria meningitidis* lipo-oligosaccharide (ref. 20), but examples of enzymatic synthesis of complex carbohydrates remain scarce (ref. 21). Indeed, certain membrane Leloir-type glycosyltransferases are not easily available. Their nucleotide activated sugar substrates are expensive. They may be generated *in situ,* but the process necessitates additional enzymes (ref. 21).

Replacement of glycosyltransferases by transglycosidases has been proposed to proceed from different types of glycosyl donors, and to be compatible with a larger variety of acceptors (ref. 22). Interestingly, modified donors were occasionally used successfully (ref. 23). Nonetheless, the availability of these enzymes is often critical, especially when considering the appropriate regio- and stereospecificity required for a given target (ref. 22, 24 and 25).

Protein engineering based on rational, semi-rational or fully combinatorial approaches (directed evolution) has also proven to be extremely useful to generate catalysts with improved natural properties but also to create new substrate specificities (ref. 26, 27). In the field of carbohydrate-enzymes, glycosyltransferase substrate specificity has been successfully modified by site-directed mutagenesis assisted by computational modelling or directed evolution for the synthesis of biologically relevant carbohydrate structure (ref. 27). Promiscuous glycosidases showing altered and new specificities towards the donor or the acceptor sugar have been generated (ref. 28-31). Engineering of new glycosynthases (*i.e*., enzymes catalyzing the condensation of sugar residues for synthesizing a glycoside) from β-glycosidases (classified into EC 3.2.1) also emerged as a powerful way to generate modified transferases, even if they use only fluoride donors (ref. 26, 32, 33). However, this methodology is difficult to apply to α-retaining enzymes. Interestingly, only one active glycosynthase derived from an α-retaining enzyme has been described up to now, and there is yet no description of substrate acceptance enlargement for α-retaining transglycosidases (or glycosynthases) (ref. 79).

In addition, enzymatic glycosylation towards complex carbohydrates, relying on compatibility between enzyme and acceptor, mostly involves fully deprotected acceptors and thus comes at the latest stages in a chemo-enzymatic synthetic process (ref. 21, 34 and 35).

Interestingly, the use of intermediates issued from enzymatic glycosylation in the subsequent generation of glycoconjugates of higher complexity has also been reported (ref. 36-38). In all cases, the building blocks, conceived by action of native glycosyltransferases, were converted to donors and used as such, following peracylation.

The term "building block" refers to a suitably protected carbohydrate intermediate occurring in the chemical pathway of synthesis of complex oligosaccharides, e.g., said carbohydrate can be a disaccharide.

The term "intermediate" refers to a compound, protected or not, issued from an enzymatic and/or synthetic step, and involved in the multi-step synthesis of a specific target, *e.g*., said compound can be a disaccharide.

The design of an appropriate enzymatic glycosylation tool that would allow an optimal combination of the chemical and enzymatic steps involved in the synthesis of complex oligosaccharides has never been attempted although it would be of major interest to develop new synthetic pathways.

The Inventors have thus investigated the applicability of enzymatic glycosylation for the synthesis of building blocks compatible with chemical chain extension both at the reducing and non-reducing ends that is compatible with subsequent conversion into donors as well as acceptors. In the course of their investigation, the Inventors have surprisingly demonstrated the applicability of engineered amylosucrases (AS) - which are glycoside hydrolases - to the synthesis of oligosaccharide fragments of *Shigella flexneri* lipopolysaccharide by *in-vitro* chemo-enzymatic synthetic methodologies, implicating an enzymatic step at an early stage in the synthesis.

Amylosucrases (EC 2.4.1.4) as well as sucrose hydrolases (EC 3.2.1.-) belong to the family 13 of the glycoside hydrolases (GH13), and more particularly the subfamily 4 (GH 13.4) as defined per the CAZY nomenclature (ref. 66-69). Amylosucrases and sucrose hydrolases operate on the same substrate (sucrose) with the same molecular mechanism (ref. 80). The difference between the amylosucrases and the sucrose hydrolases is only different transglycosylation abilities (ref. 69).

The structure of amylosucrase from *Neisseria polysaccharea* is the only known structure of enzymes from family GH13.4 (ref. 81). The single polypeptide chain (628 amino acid residues) of amylosucrase from *Neisseria polysaccharea* is folded into a tertiary structure consisting of five domains named N (residues 1-90), A (residues 98-184; 261-395; 461-550), B (residues 185-260), B' (residues 395-460) and C (residues 555-628). Domains A, B and C are common domains found in family GH13. Domains N and B' are specific to family GH13.4. Domain N is the N-terminal domain composed of 6 α-helices. Domain A is made up of eight alternating β-sheets (β1- β8) and α-helices (α1-α8) building up the catalytic core: the (β/α)₈ barrel common to family GH13. It contains also eight loops connecting helices to strands (labelled loop1 to loop8). Domain B, or loop 3, is an extension of domain A, containing two short antiparallel β-sheets flanked by two α-helices. Domain B', or loop 7, is another extension of domain A, composed of two α - helices followed by a β-sheet and another short β -helice. Domain C is an eight-stranded β-sandwich found C-terminal to the (β/α)₈ barrel.

Unexpectedly, the Inventors have now found eleven consensus amino acid sequences to characterize glycoside hydrolases: eight consensus motifs defined hereafter (SEQ ID NO: 1; SEQ ID NO: 2; SEQ ID NO: 3; SEQ ID NO: 6; SEQ ID NO: 7; SEQ ID NO: 8; SEQ ID NO: 10; SEQ ID NO: 11) are localised in said β-sheets (6) or said loops (2) constituting domain A, two consensus motifs (SEQ ID NO: 4; SEQ ID NO: 5) are found in said domain B and one consensus motifs (SEQ ID NO: 9) is found is said domain B'.

*Shigella* is the causal agent of shigellosis, or bacillary dysentery. In developing countries, it induces about 1 million deaths per year, most of which involve children under five years of age (ref. 39). In countries where disease is endemic, a number of *S*. *flexneri* serotypes and to a lesser extent *S*. *sonnei* are isolated, emphasizing the need for a multivalent vaccine. Noteworthy, despite numerous clinical trials (ref. 40), no vaccine is available so far. Epidemiological as well as experimental data point to the polysaccharide part, or O-antigen (O-Ag), of the bacterial lipopolysaccharide as an important virulence factor (ref. 41) and the major target of protective humoral response against reinfection (ref. 42). *S. flexneri* is divided into 14 serotypes based on known O-Ag structures. Interestingly, protein-conjugates of short synthetic oligosaccharides mimicking *S*. *flexneri* 2a O-Ag induced in mice a potent anti-O-Ag humoral immune response, which was shown to be protective in a murine model of infection (ref. 43). The diversity, associated to a close resemblance in composition, of the known *S*. *flexneri* O-Ag repeating units was found of utmost interest to challenge the investigation. Indeed, except for serotype 6, all *S*. *flexneri* O-Ag repeating units share a linear tetrasaccharide backbone (ref. 41). Diversity resides in the branching pattern, which involves *O*-acetyl and/or α-D-glucopyranosyl decorations (ref. 41, 44). Interestingly, at least 4 different patterns of α-D-glucosylation, have been characterized for this family of bacterial polysaccharides. L-Rhamnopyranose residue is implicated as branching acceptor. By way of example, *S*. *flexneri* 2b, 3a, 5a, 5b, and X O-Ags have the α-D-glucopyranosyl-(1→3)-α-L-rhamnopyranosyl (EA) branching pattern in common:
2b O-antigen: **2)-[α-D-Glcp(1→3)]-α-L-Rha*p*-(1**→2)-α-L-Rha*p-*(1→3)-[α-D-G1c*p*(1→4)]-α-L-Rha*p*-(1→3)-β-D-Glc*p*NAc-(1→
3a O-antigen: **2)-[α-D-Glc*p*(1→3)]-α-L-Rha*p*-(1**→2)-α-L-Rha*p-*(1→3)-[2Ac]-α-L-Rha*p*-(1→3)-β-D-Glc*p*NAc-(1→
5a O-antigen: 2)-α-L-Rha*p*-(1→**2)-[α-D-Glc*p*(1→3)]-**α**-L-Rha*p-*(1**→3)-α-L-Rha*p*-(1→3)-β-D-Glc*p*NAc-(1→
5b O-antigen: **2)-[α-D-Glc*p*(1→3)]-α-L-Rha*p*-(1→2)-[α-D-Glc*p*(1→3)]-α-L-Rha*p*-(1**→3)-α-L-Rha*p*-(1→3)-β-D-Glc*p*NAc-(1→
X O-antigen: **2)-[α-D-Glc*p*(1→3)]-α-L-Rha*p*-(1**→2)-α-L-Rha*p-*(1→3)-α-L-Rha*p*-(1→3)-β-D-Glc*p*NAc-(1→

Abbreviations: Glc*p* = Glucopyranosyl, Rha*p* = Rhamnopyranosyl, GlcNAc*p* = *N*-acetyl-Glucosaminopyranosyl, Ac = acetyl

Here-under are examples of repeating units and/or cores of bacterial surface polysaccharides containing the disaccharide motives synthesized by glucansucrases and that can be obtained by the method of the invention (ref. 38, 43, 44, 45):

| | | |
|---|---|---|
| | | →2)-**[α-D-Glcp-(1→3)]-α-L-Rha*p*-**(1→2)-α- |
| α-D-Glc*p*-(1→3)-L- | *S.flexneri* type 2b | L-Rha*p*-(1→3)-[α-D-Glcp-(1→4)]-α-L-Rha*p*- |
| Rha*p* | | (1→3)-β-D-Glc*p*NAc-(1→ |
| | | →2)**-[α-D-Glcp-(1→3)]-α-L-Rha*p***-(1→2)-α- |
| | *S. flexneri* type 3a | L-Rha*p*-(1→3)-[2Ac]-α-L-Rha*p*-(1→3)-β-D- |
| | | Glc*p*NAc-(1→ |
| | | →2)-α-L-Rha*p*-(1→2)-**[α-D-Glcp-(1→3)]-**α**-** |
| | *S. flexneri* type 5a | **L-Rha*p***-(1→3)-α-L-Rha*p*-(1→3)-β-D-Glc*p*NAc- |
| | | (1→ |
| | | →2)-**[α-D-Glcp-(1→3)]-α-L-Rha*p***-(1→2)-[α- |
| | *S. flexneri* type 5b | **D-Glcp-(1→3)]-α-L-Rha*p*-(**1→3)-α-L-Rha*p*- |
| | | (1→3)-β-D-Glc*p*NAc-(1→ |
| | | →2)-**[α-D-Glcp-(1-3)]-**α**-L-Rha*p***-(1→2)- |
| | S. *flexneri* type X | |
| | | (1→3)-α-L-Rha*p*-(1→3)-β-D-Glc*p*NAc-(1→ |
| | *S*. *pneumoniae* type | →2)-α-D-Gal*p*-(1→3)-**α-D-Glc*p*-(1→3)-α-L-** |
| | 6B | **Rha*p***-(1→4)-D-Rib-ol-(5→P→ |
| | | →4)-β-D-Glc*p*-(1→4)-[α-D-Glc*p*(6OAc)- |
| | *S. pneumoniae* type | |
| | 18C | (1→2)]-[Gro-(1→P→3)-β-D-Gal*p*-(1→4)-**α-D-** |
| | | **Glc*p*-(1→3)-β-L-Rha*p***-(1→P→ |
| | *S. pneumoniae* type | →4)-β-D-Man*p*NAc-(1→4)-α-D-Glc*p*-(1→3)- |
| | 19A | **α-L-Rha*p***-(1→P→ |

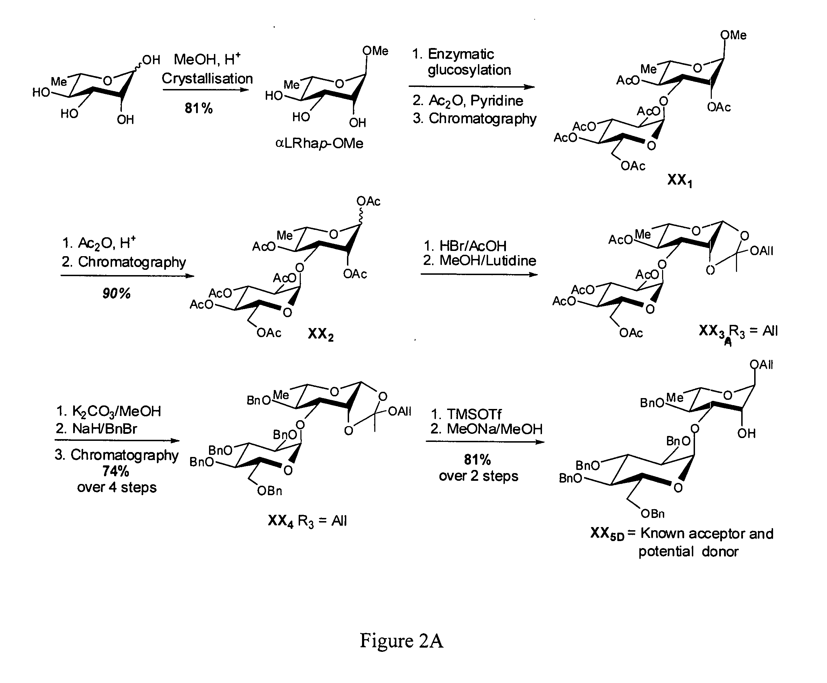

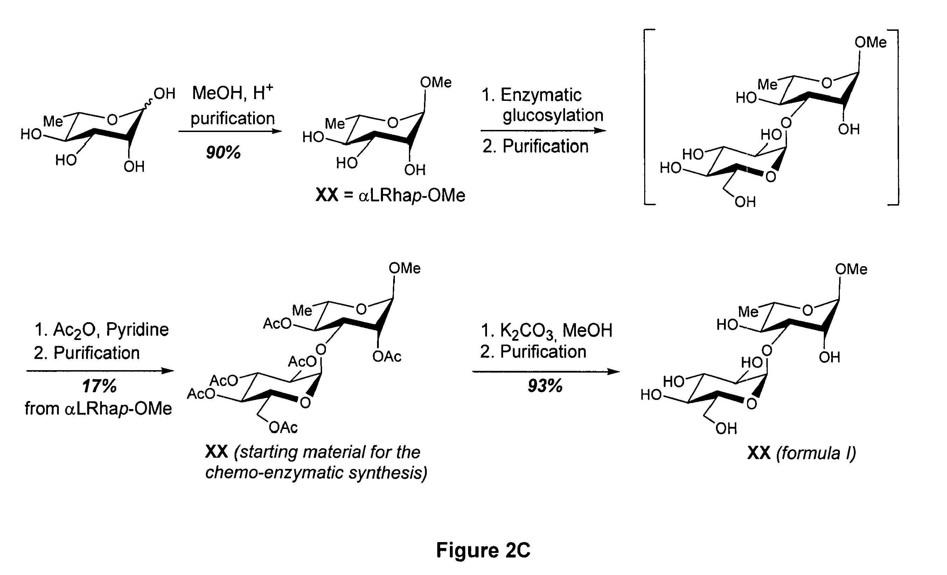

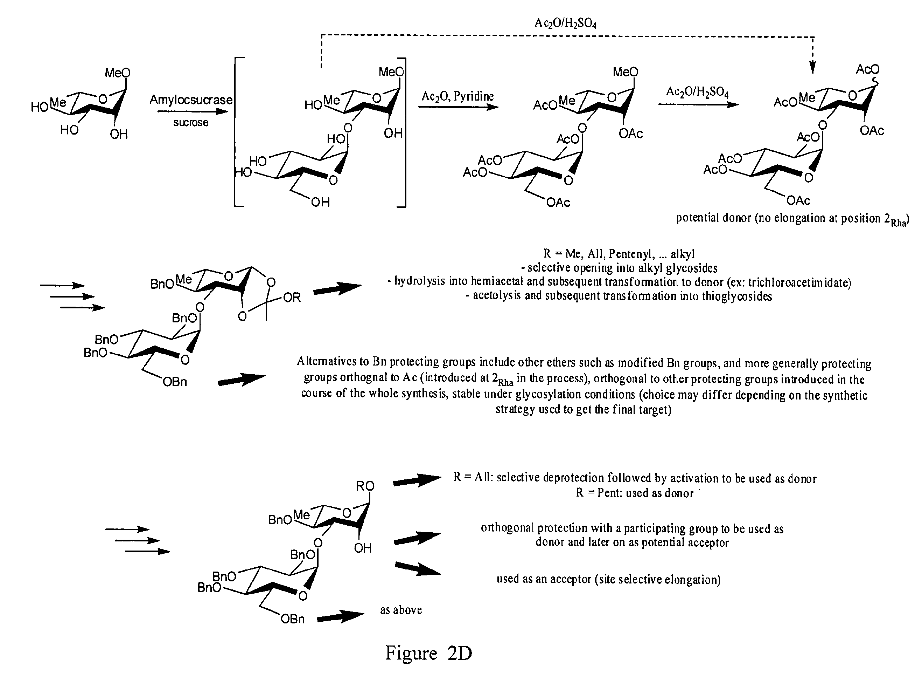

Within the framework of research that has lead to the present invention, the Inventors have demonstrated the chemo-enzymatic synthesis of S. *flexneri* serotype-specific oligosaccharides (Figure 2A) by initially selecting a lightly protected L-Rhamnopyranose residue as substrate acceptor, and a recombinant amylosucrase (an α-retaining transglucosidase from family 13 of glycoside-hydrolases that uses sucrose as glucosyl donor) (ref. 45, 46). New amylosucrase specificity was then successfully generated to glucosylate efficiently and regiospecifically methyl α-L-rhamnopyranoside (α-L-Rha*p*-OMe) (ref. 78) to provide a disaccharide intermediate (Example 1, Figure 2C) which could be chemically converted into a building block compatible with chemical chain elongation (Example 3, Figure 2A & 2D). Other examples include the successful enzymatic glucosylation at position 3 of allyl α-L-rhamnopyranoside (α-L-Rha*p*-OAllyl) (ref. 82) and the easiest accessible precursor L-rhamnopyranose (L-Rha*p*) (Example 2).

Accordingly, the present invention provides a method for preparing the synthetic intermediate corresponding to the disaccharide [α-D-Glc*p*(1→3)]-α-L-Rhap-OMe of formula (I) (ref. 74 and 62), and more generally to the disaccharide [α-D-Glcp(1→3)]-α-L-Rha*p*-YR of formula (Ia), wherein Y is selected from -0- and -Sand R is selected from the group consisting of: C₁-C₆ alkyl, C₁-C₆ alkenyl, aryl, allyl, -CO-alkyl (C₁-C₆), -CO-alkenyl (C₁-C₆), -CO-aryl; aryl designating an aromatic group like phenyl, benzyl, possibly substituted by one or several of the following groups: C₁-C₄ alkyl, -NO₂, a halogen atom, -O-alkyl (C₁-C₆). 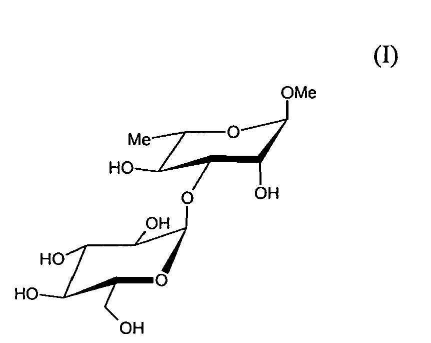 said method being characterized in that it comprises the step of using a mutant of a wild type glycoside hydrolase, wherein said wild type glycoside hydrolase has 450 to 850 amino acids, preferably 580 to 735 amino acids, and comprises from the N- to C-terminus eleven motifs defined by the following consensus motifs:
(1) the amino acid sequence LGVNYLHLMPL (SEQ ID NO: 1), which is located in the β-strand 2 of said wild type glycoside hydrolase;
(2) the amino acid sequence DGGYAV (SEQ ID NO: 2), which is located in the loop 2 of the (β/α)₈-barrel of said wild type glycoside hydrolase ;
(3) the amino acid sequence DFVFNH (SEQ ID NO: 3) which is located in the β-strand 3 of said wild type glycoside hydrolase;
(4) the amino acid sequence LREIFPDTAPGNF (SEQ ID NO: 4), which is located in the domain B of said wild type glycoside hydrolase;
(5) the amino acid sequence FNSYQWDLN (SEQ ID NO: 5), which is located in the C-terminal part of the domain B of said wild type glycoside hydrolase;
(6) the amino acid sequence ILRLDAVAFLWK (SEQ ID NO: 6), which is located in the β-strand 4 of said wild type glycoside hydrolase;
(7) the amino acid sequence EAIV (SEQ ID NO: 7), which is located in the β-strand 5 of said wild type glycoside hydrolase;
(8) the amino acid sequence YVRCHDDI (SEQ ID NO: 8), which is located in the β-strand 7 of said wild type glycoside hydrolase;
(9) the amino acid sequence RISGTLASLAG (SEQ ID NO: 9), which is located in the domain B' of said wild type glycoside hydrolase;
(10) the amino acid sequence GIPLIYLGDE (SEQ ID NO: 10), which is located in the β-strand 8 of said wild type glycoside hydrolase;
(11) the amino acid sequence RWVHRP (SEQ ID NO: 11), which is located in the loop 8 of the (β/α)₈-barrel,
   and the sequence formed by said eleven motifs joined end-to-end from motif (1) to motif (11) of said wild type glycoside hydrolase has at least 65%, preferably at least 70%, and by order of increasing preference, at least 75%, 80%, 85%, 90%, 95%, 95%, 97%, 98%, and 99%, or 100% sequence identity or at least 80%, preferably at least 85%, and by order of increasing preference, at least 90%, 95%, 95%, 97%, 98%, and 99%, or 100% sequence similarity with the amino acid sequence SEQ ID NO: 12, which is formed by the concatenation of the eleven consensus motifs ordered from (1) to (11);
wherein said mutant has a mutation consisting of:
- the substitution of the amino acid residue at position 4 in said motif (4) with any amino acid selected from the group consisting of alanine (A), cysteine (C), glycine (G), histidine (H), lysine (K), asparagine (N), arginine (R), serine (S), threonine (T), tryptophan (W) and tyrosine (Y), or
- the substitution of the amino acid residue at position 9 in said motif
(6) with any amino acid selected from the group consisting of cysteine (C), glutamic acid (E), isoleucine (I) and valine (V).

A "wild type glycoside hydrolase" refers to an amylosucrase (EC 2.4.1.4) or sucrose hydrolase (EC 3.2.1.-), preferably an amylosucrase. A wild type glycoside hydrolase belongs to the family 13, subfamily 4, of the glycoside hydrolases (GH 13.4) as defined per the CAZY nomenclature (ref. 66-69, http://www.cazy.org).

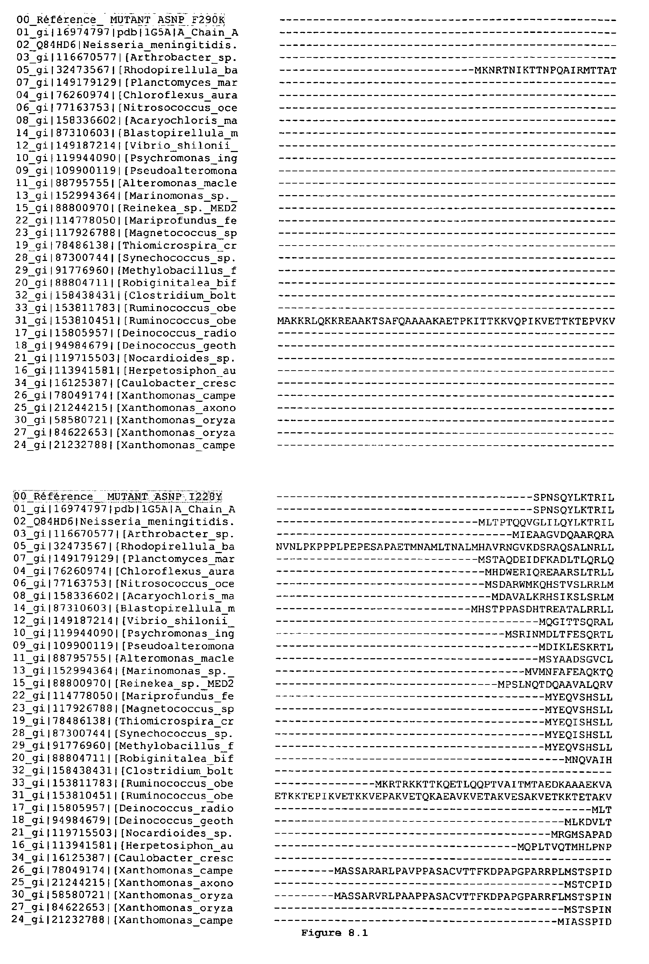

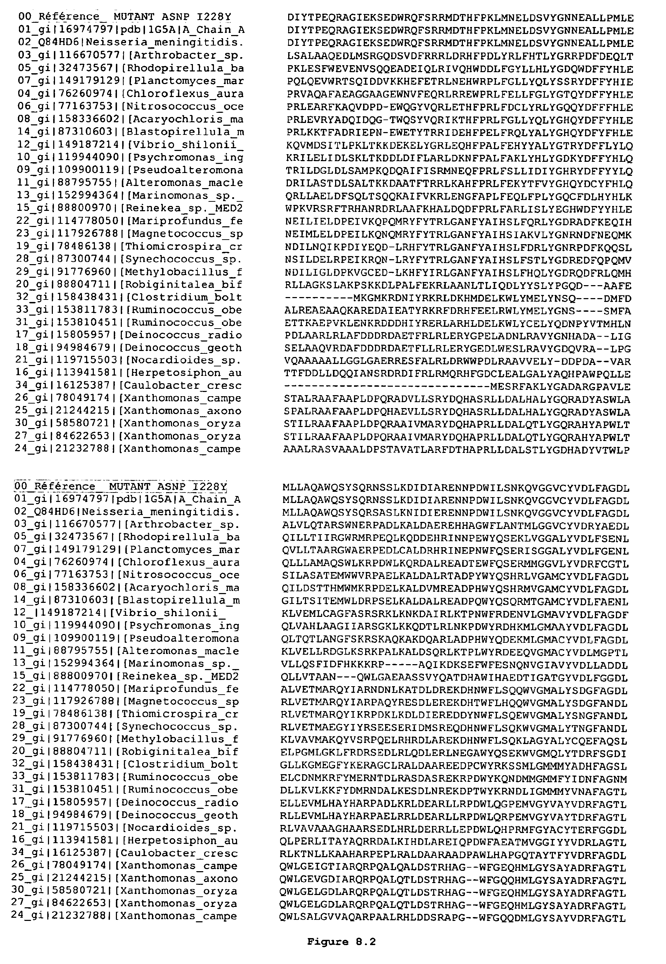

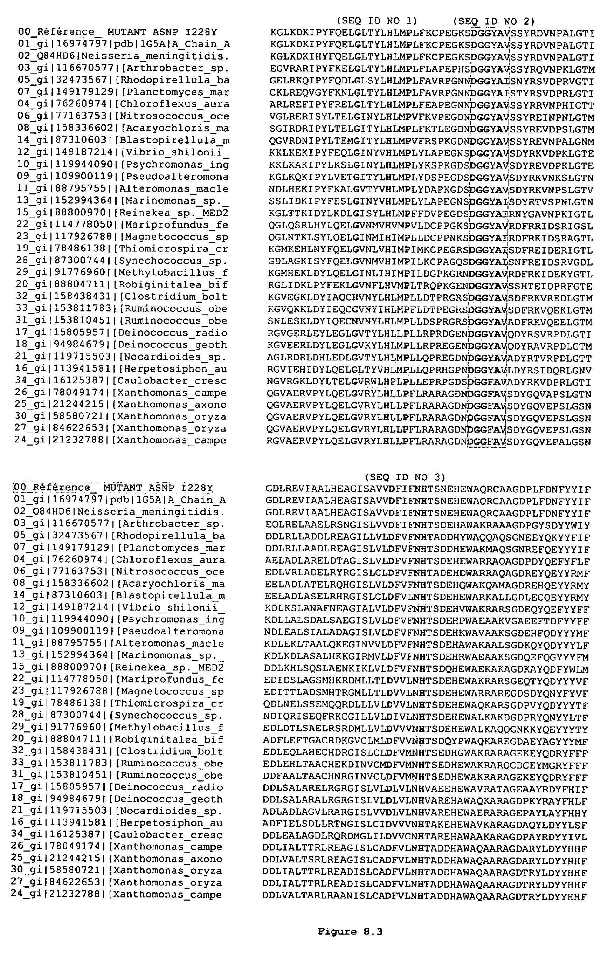

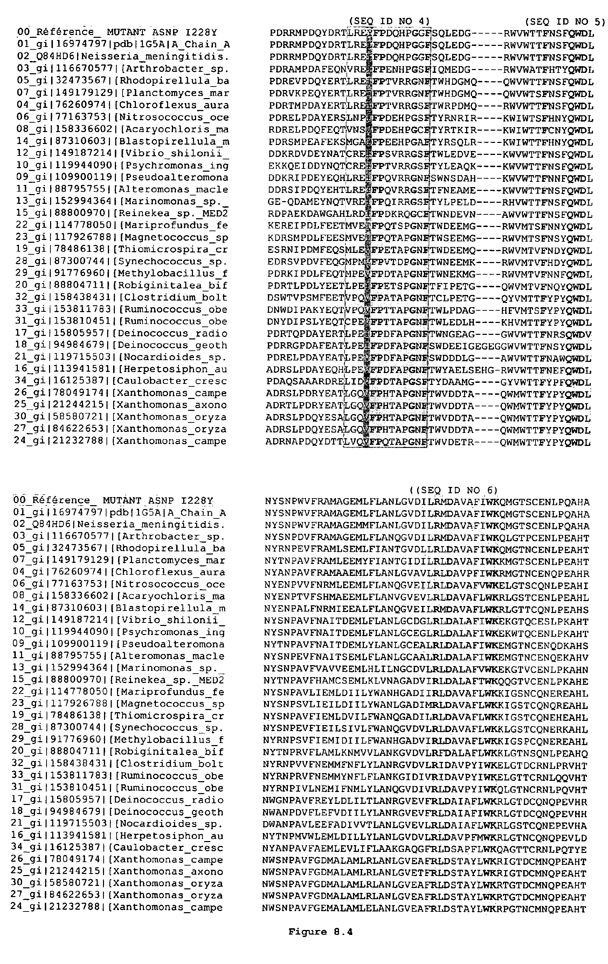

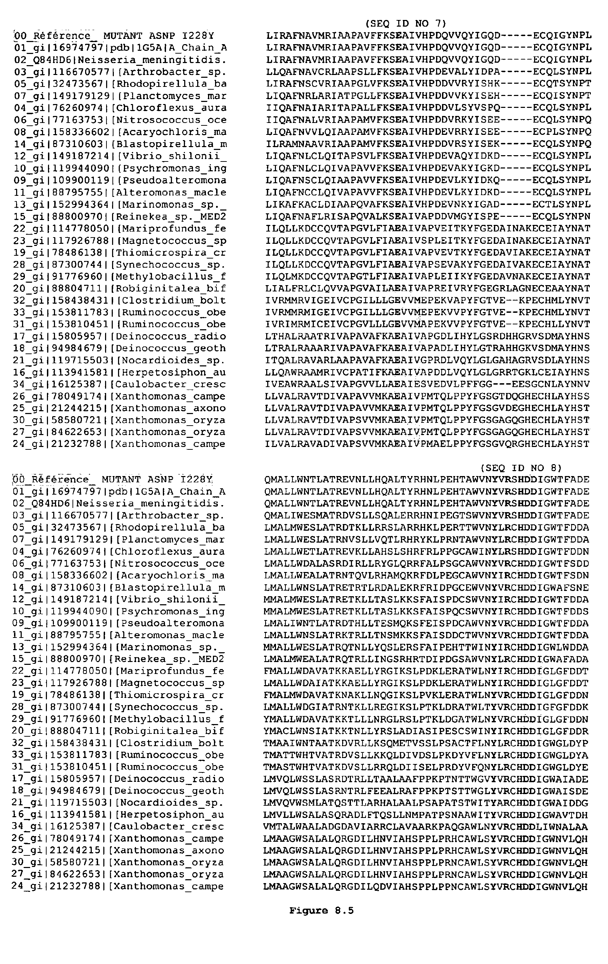

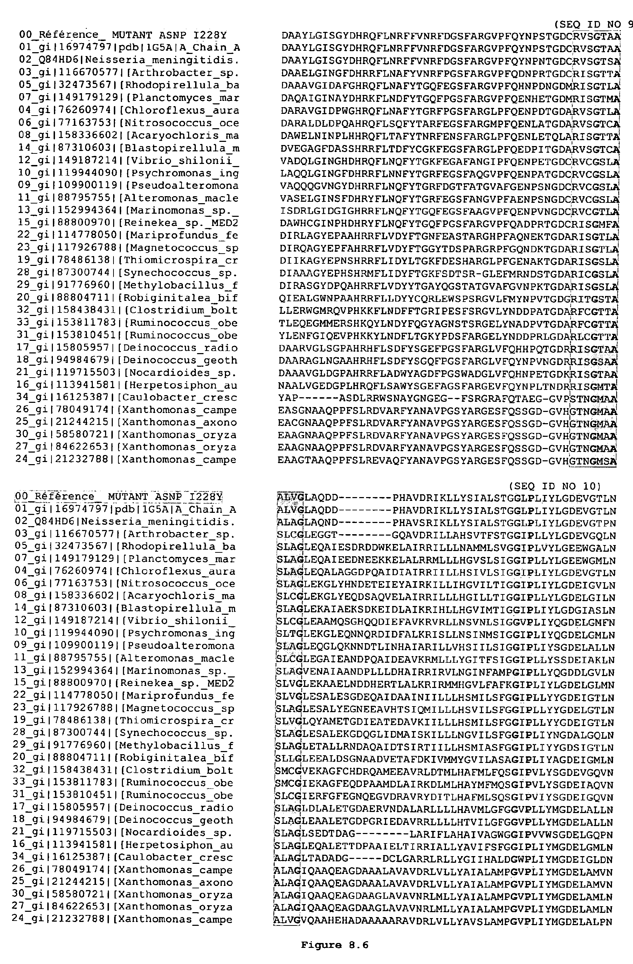

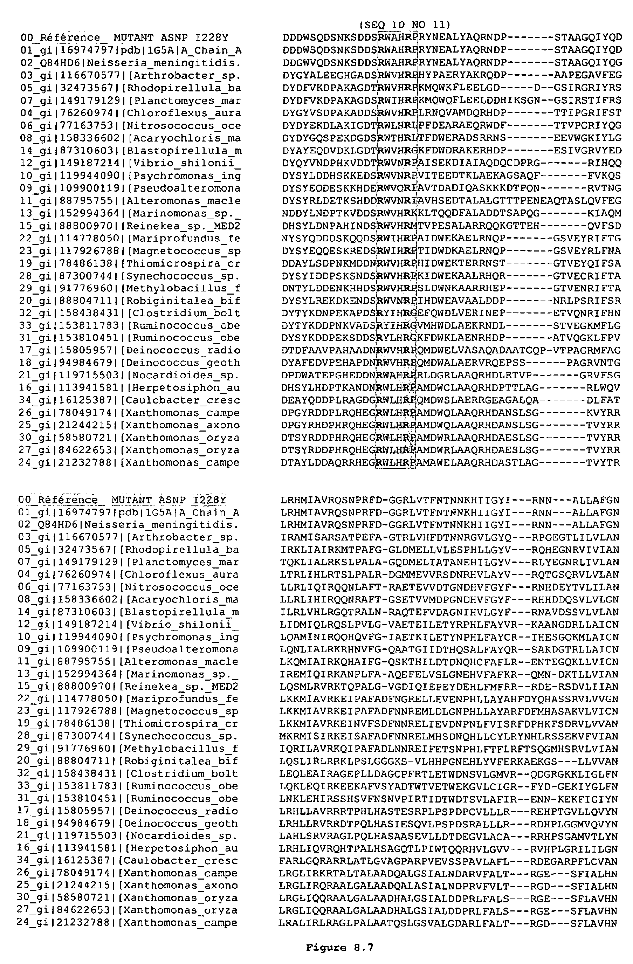

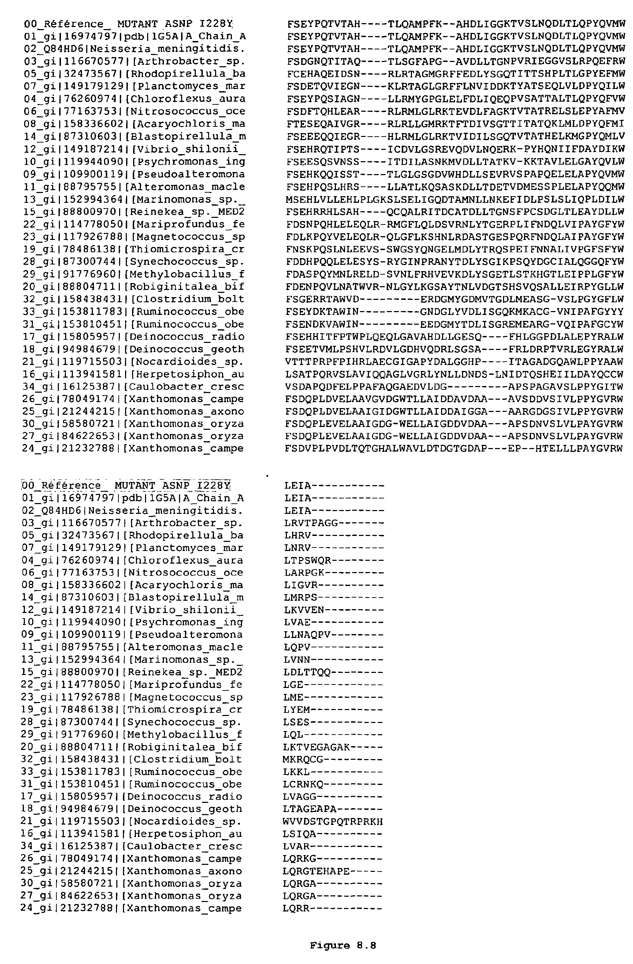

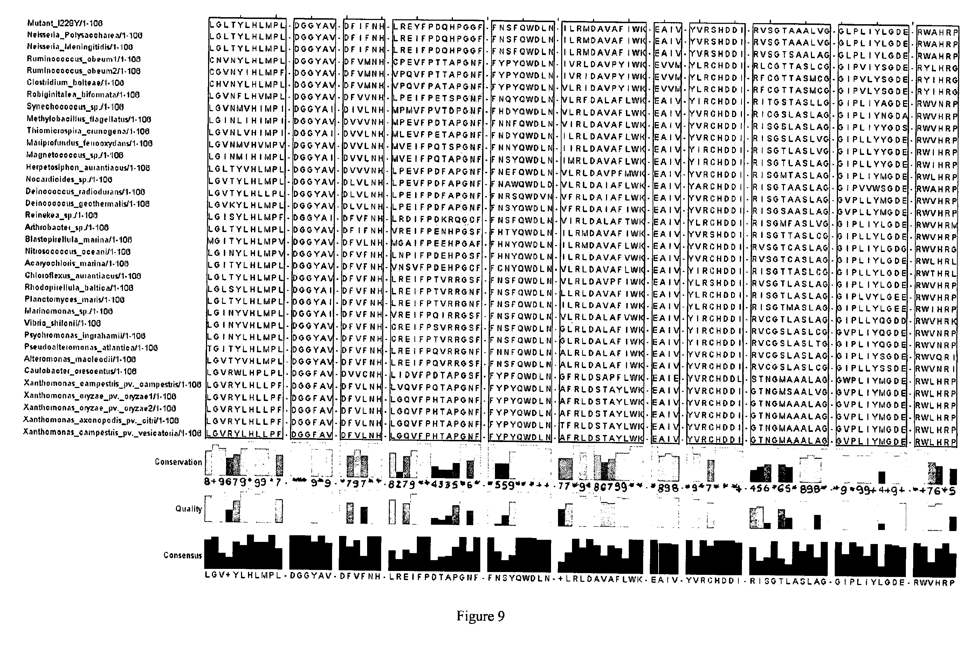

The eleven consensus motifs of the wild type glycoside hydrolases have been found by the inventors by aligning 34 wild type glycoside hydrolases as shown in Figures 8 and 9.

By way of example, the glycoside hydrolase 1G5A (gi|16974797, SEQ ID NO: 13) comprises, from the N- to C-terminus, the eleven following motifs: (1) 125-134, (2) 144-149, (3) 182-187, (4) 225-237, (5) 250-258, (6) 282-293, (7) 328-331, (8) 388-395, (9) 446-456, (10) 480-489 and (11) 509-514 of SEQ ID NO: 13. These eleven motifs joined end-to-end form motif (1) to motif (11) form an amino acid sequence which has 83% sequence identity and 92% sequence similarity with the sequence SEQ ID NO: 12.

In order to identify the eleven motifs from a wild type glycoside hydrolase, one of skilled in the art can align the amino acid sequence of this wild type glycoside hydrolase against the amino acid sequence of the glycoside hydrolase 1G5A (SEQ ID NO: 13) for example, and therefore identify the eleven motifs thereof matching the eleven motifs of 1 G5A as described above.

Unless otherwise specified, sequence alignments are performed using the well-known MUSCLE program under default parameters (http://phylogenomics.berkeley.edu/cgi-bin/muscle/input_muscle.py). Jalview software can be used for visualizing the alignment and generating the eleven motifs joined end-to-end. The sequence identity and similarity values provided herein are calculated using the Vector NTI AlignX program (V9.1.0, Invitrogen, USA) on a comparison window including the whole set of eleven consensus motifs ordered from 1 to 10 as defined above.

In a particular embodiment of said wild type glycoside hydrolase, it is an amylosucrase selected from the group consisting of the proteins available in the GENBANK database under the following accession number: gi|16974797 (named 1G5A and also reproduced herein as SEQ ID NO: 13), gi|99031739 (named 1ZS2), gi|27574003 (1MVY), gi|27574004 (named 1MW0), gi|47169012 (named 1S46), gi|16974938 (named 1JGI), gi|27574006 (named 1MW2), gi|27574007 (named 1MW3), gi|27574005 (named 1MW1), gi|16974937 (named 1JG9), gi|27728142 (named Q84HD6), gi|116670577, gi|32473567, gi|149179129, gi|76260974, gi|77163753, gi|158336602, gi|87310603, gi|149187214, gi|119944090, gi|109900119, gi|88795755, gi|152994364, gi|88800970, gi|114778050, gi|117926788, gi|78486138, gi|87300744, gi|91776960, gi|88804711, gi|158438431, gi|153811783, gi|153810451, gi|15805957, gi|94984679, gi|119715503, gi|113941581, gi|16125387, gi|119477809 and gi|89092061.

In a more preferred embodiment of said wild type glycoside hydrolase, it is an amylosucrase from *Neisseria polysaccharea,* and is preferably selected from the group consisting of 1G5A, 1ZS2, 1MVY, 1MW0, 1S46, 1JGI, 1MW2, 1MW3, 1MW1 and 1JG9 proteins.

In another preferred embodiment of said wild type glycoside hydrolase, it is a sucrose hydrolase from *Xanthomonas,* and is preferably selected from the group consisting of the proteins available in the GENBANK database under the following accession number: gi|8049174, gil21244215, gi|58580721, gi|84622653, gi|21232788.

The Table I below shows the sequence identity and similarity percent of the eleven motifs joined end-to-end for each of 34 glycoside hydrolases as described above with the sequence SEQ ID NO: 12.

**Table I:**

| GI number in the GenBank Database | Organisms | % identity | % similarity |
|---|---|---|---|
| gi\|16974797 | *Neisseria polysaccharea* | 83 | 92 |
| gi\|27728142 | *Neisseria meningitidis* | 84 | 92 |
| gi\|116670577 | *Arthrobactersp. FB24* | 83 | 90 |
| gi\|76260974 | *Chloroflexus aurantiacus J-10-fl* | 85 | 93 |
| gi\|32473567 | *Rhodopirellula baltica SH 1* | 86 | 91 |
| gi\|77163753 | *Nitrosococcus oceani ATCC 19707* | 85 | 92 |
| gi\|149179129 | *Planctomyces maris DSM 8797* | 85 | 91 |
| gi\|158336602 | *Acaryochloris marina MBIC11017* | 81 | 93 |
| gi\|109900119 | *Pseudoalteromonas atlantica T6c* | 81 | 93 |
| gi\|119944090 | *Psychromonas ingrahamii* 37 | 81 | 91 |
| gi\|88795755 | *Alteromonas macleodii 'Deep ecotype'* | 79 | 90 |
| gi\|149187214 | *Vibrio shilonii AK1* | 79 | 88 |
| gi\|152994364 | *Marinomonas sp. MWYL1* | 79 | 86 |
| gi\|87310603 | *Blastopirellula marina DSM3645* | 80 | 91 |
| gi\|88800970 | *Reinekea sp. MED297* | 80 | 91 |
| gi\|113941581 | *Herpetosiphon aurantiacus ATCC 23779* | 82 | 92 |
| gi\|15805957 | *Deinococcus radiodurans R1* | 83 | 93 |
| gi\|94984679 | *Deinococcus geothermalis DSM 11300* | 85 | 93 |
| gi\|78486138 | *Thiomicrospira crunogena XCL-2* | 83 | 91 |
| gi\|88804711 | *Robiginitalea biformata HTCC2501* | 82 | 90 |
| gi\|119715503 | *Nocardioides sp. JS614* | 81 | 91 |
| gi\|114778050 | *Mariprofundus ferrooxydans PV-1* | 82 | 91 |
| gi\|117926788 | *Magnetococcus sp. MC-1* | 84 | 91 |
| gi\|21232788 | *Xanthomonas campestris pv. campestris str.* | 73 | 88 |
| gi\|21244215 | *Xanthomonas axonopodis pv. citri str. 306* | 74 | 88 |
| gi\|78049174 | *Xanthomonas campestris pv. vesicatoria str. 85-10* | 74 | 88 |
| gi\|84622653 | *Xanthomonas oryzae pv. oryzae MAFF 311018* | 74 | 88 |
| gi\|87300744 | *Synechococcus sp. WH 5701* | 79 | 91 |
| gi\|91776960 | *Methylobacillus flagellatus KT* | 82 | 88 |
| gi\|58580721 | *Xanthomonas oryzae pv. oryzae KACC10331* | 74 | 88 |
| gi\|153810451 | *Ruminococcus obeum ATCC 29174* | 73 | 88 |
| gi\|158438431 | *Clostridium bolteae ATCC BAA-613* | 68 | 84 |
| gi\|153811783 | *Ruminococcus obeum ATCC 29174* | 67 | 83 |
| gi\|16125387 | *Caulobacter crescentus CB15* | 68 | 86 |

According to a preferred embodiment of said wild type glycoside hydrolase, it contains an isoleucine (I) or valine (V) residue at position 4 in said motif (4), preferably an isoleucine.

According to another preferred embodiment of said wild type glycoside hydrolase, it contains a phenylalanine (F) or tyrosine (Y) residue at position 9 in said motif (6), preferably a phenylalanine.

By way of example, the amino acid residues at position 4 in said motif (4) and at position 9 in said motif (6) of the amylosucrase 1G5A, corresponds respectively to an isoleucine (I) at position 228 and a phenylalanine (F) at position 290 in the amino acid sequence of 1G5A (SEQ ID NO: 13).

According to a preferred embodiment of said mutant of a wild type glycoside hydrolase, the amino acid residue at position 4 in said motif (4) is substituted with any amino acid selected from the group consisting of histidine, tryptophan and tyrosine.

According to another preferred embodiment of said mutant of a wild type glycoside hydrolase, the amino acid residue at position 9 in said motif (6) is substituted with any amino acid selected from the group consisting of glutamic acid and isoleucine.

Unexpectedly, the mutants of a wild type glycoside hydrolase, in particular mutants of an amylosucrase, as defined in the present invention show a specific activity towards L-Rha*p*, α-L-Rha*p*-OMe, α-L-Rha*p*-OAllyl. The use of an appropriate combination of a mutant of wild type glycoside hydrolase, in particular an amylosucrase, with a donor and an acceptor as defined in the present invention at an earlier stage of a multi-step synthesis leads to the synthesis of any complex oligosaccharides, such as fragments of *S. flexneri* 3a, X, 5a, 5b, and 2b O-antigens.

Especially, the invention is directed to a method for preparing the synthetic intermediate corresponding to the disaccharide [α-D-Glcp(1→3)]-α-L-Rha*p-*OMe of formula (I): 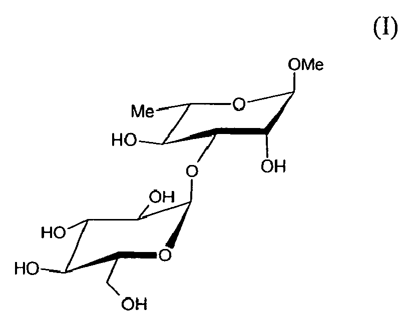 and more generally to the disaccharide [α-D-Glc*p*(1→3)]-α-L-Rha*p-*YR of formula (Ia),
wherein Y is selected from -O- and -S- and R is selected from the group consisting of: C₁-C₆ alkyl, C₁-C₆ alkenyl, aryl, allyl, -CO-alkyl (C₁-C₆), -CO-alkenyl (C₁-C₆), -CO-aryl: 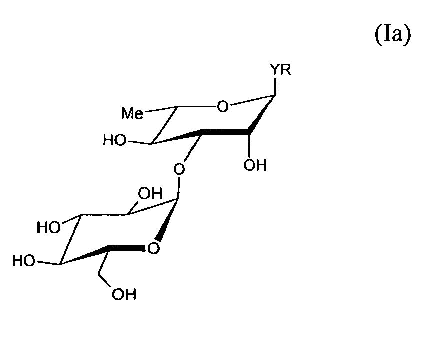 wherein aryl designates an aromatic group like phenyl, benzyl, possibly substituted by one or several of the following groups: C₁-C₄ alkyl, -NO₂, a halogen atom, -O-alkyl (C₁-C₆),
said method being characterized in that it comprises the step of reacting a mutant of the wild type glycoside hydrolase as defined above, with the acceptor of formula (II) (methyl α-L-rhamnopyranoside (ref. 78)) or with the acceptor of formula (IIa), respectively: 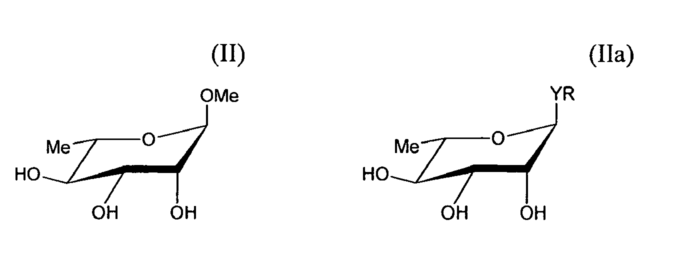 and with a donor of formula (IIIa) : 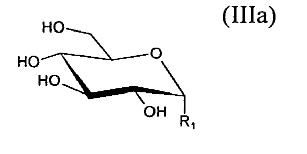 wherein R₁ represents a group selected from: 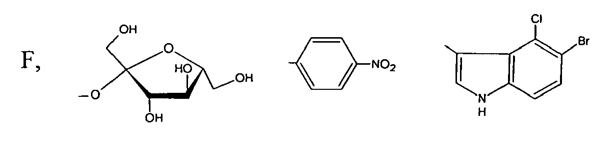 and preferably with the sucrose donor of formula (III):

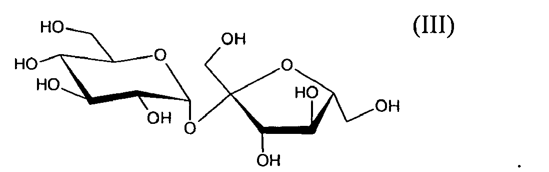

Advantageously, the method further comprises at least one step of acetylation by treatment with Ac₂O so that the disaccharide of formula (XX₁) or (XX₁ₐ): 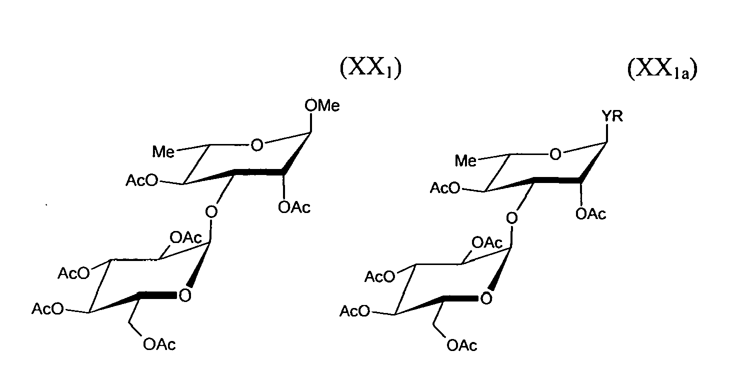 is obtained as intermediate molecule.

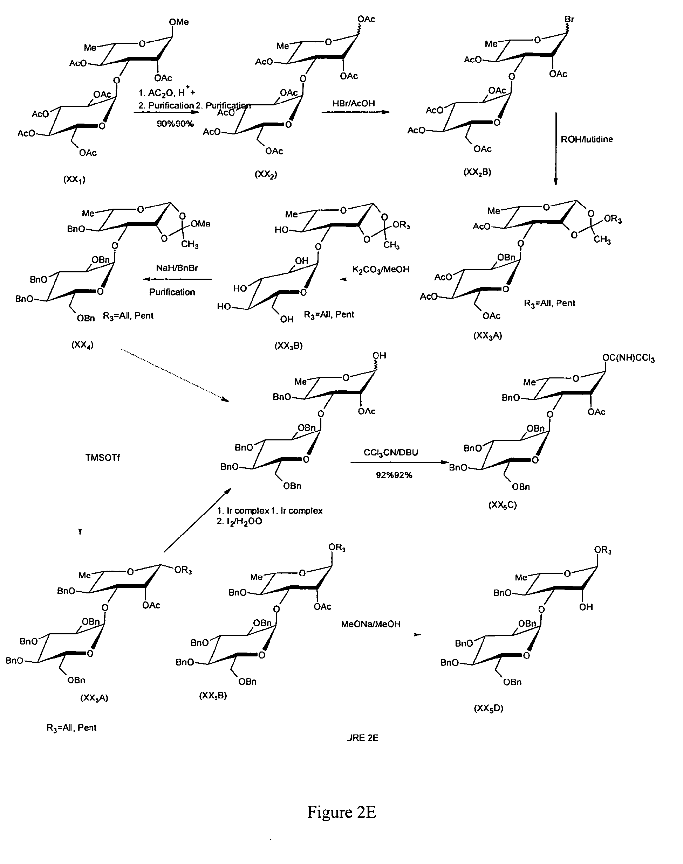

Another object of the invention is a method comprising an enzymatic glucosylation step for the preparation of the building block corresponding to the disaccharide of formula (XX₅): 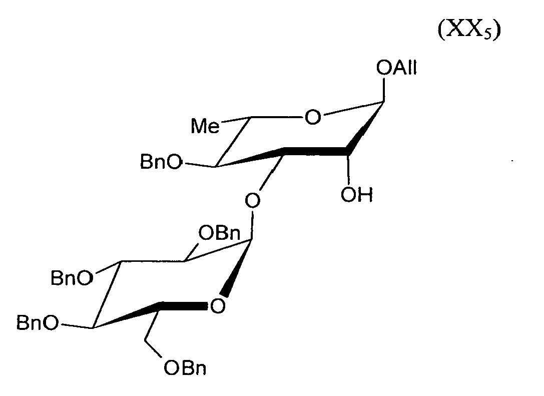 wherein it follows the steps according to scheme 1 below. Said method is illustrated in the examples and in figure 2E with more details and variants. 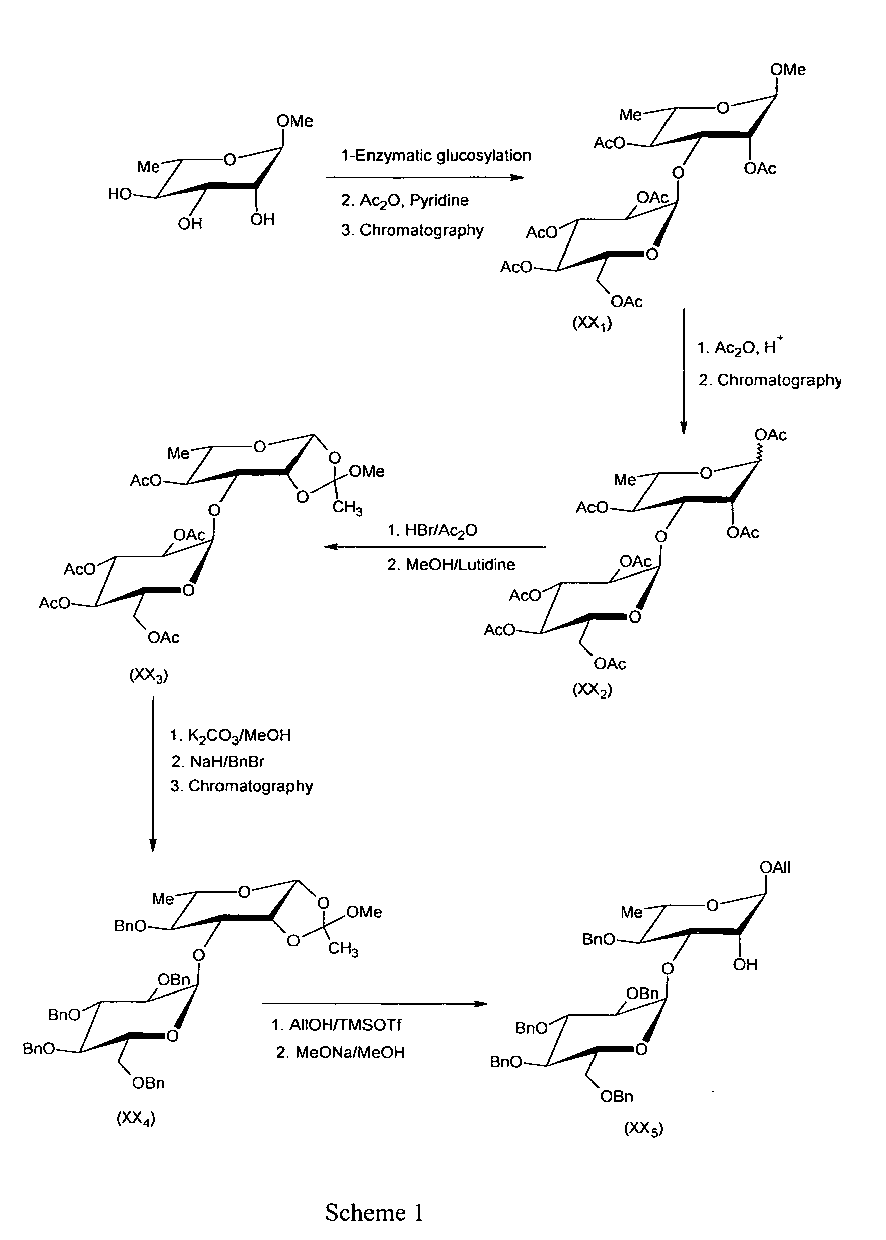
- methyl glycosidation of L-Rhamnose,
- enzymatic glucosylation by treatment of methyl α-L-rhamnopyranoside used as an acceptor with a mutant of wild type glycoside hydrolase according to the present invention,
- advantageously rough purification, preferably by flash chromatography
- acetylation
- advantageously purification by chromatography
- acetolysis, but direct anomeric bromination or chlorination from XX₁ is possible
- advantageously purification by chromatography
- anomeric bromination, or anomeric deacetylation and conversion into a trichloroacetamidate donor
- cyclization into an orthoester preferably with allyl alcohol, but it can be done with MeOH, pentenyl alcohol
- deacetylation, possibly under basic conditions
- protection of the hydroxyl groups, preferentially as ethers. This protection is described here with benzyl but the same could be done with substituted benzyl or benzoyl or lipophilic protecting groups (like those disclosed in reference 76 or fluorinated protective groups like those described in reference 77)
- purification preferably by crystallisation.
- orthoester opening under acid catalysis to give a glycoside or hemiacetal, which for example can be converted into a trichloroacetimidate donor (TCA) by reaction with trichloroacetonitrile in the presence of a base,
- selective deacetylation.

Another object of the invention is a method comprising an enzymatic glucosylation step as illustrated on figure 2E, for preparing any of the disaccharide of formula (XX₁), the disaccharide of formula (XX₂), the disaccharide of formula (XX_{2B}), the disaccharide of formula (XX₃), the disaccharide of formula (XX_{3A}), the disaccharide of formula (XX_{3B}), the disaccharide of formula (XX₄), the disaccharide of formula (XX₅), the disaccharide of formula (XX_{5A}), the disaccharide of formula (XX_{5B}), the disaccharide of formula (XX_{5C}), the disaccharide of formula (XX_{5D}), or the disaccharide of formula (XX_{5E}): 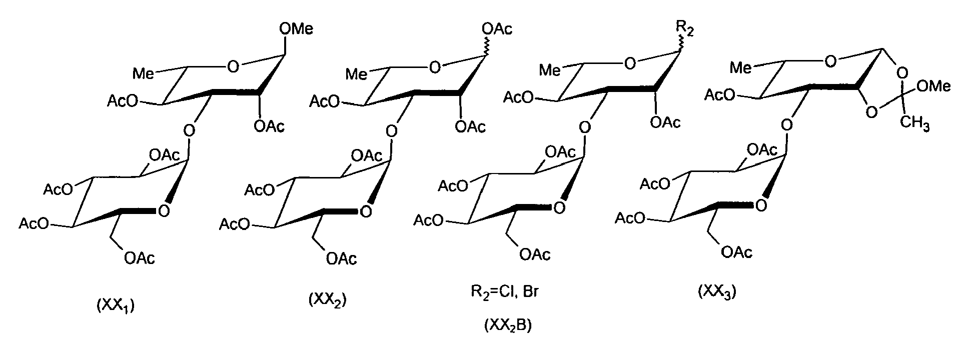 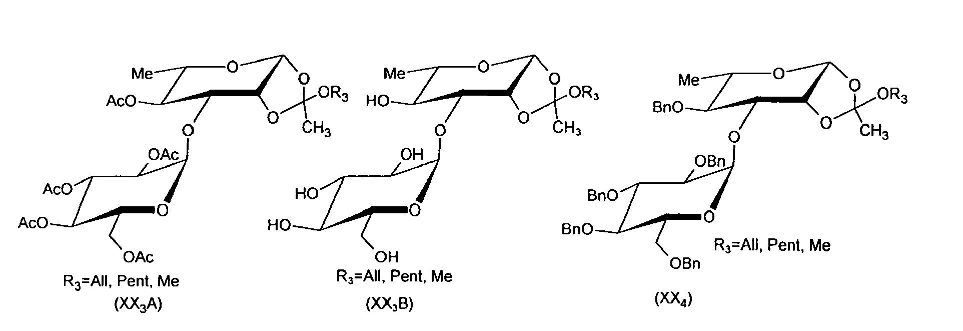 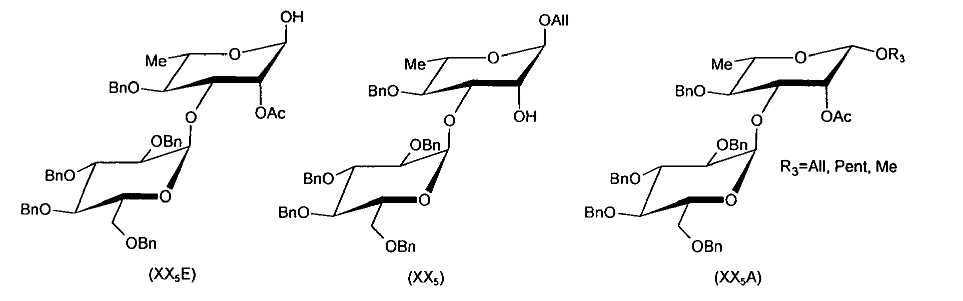 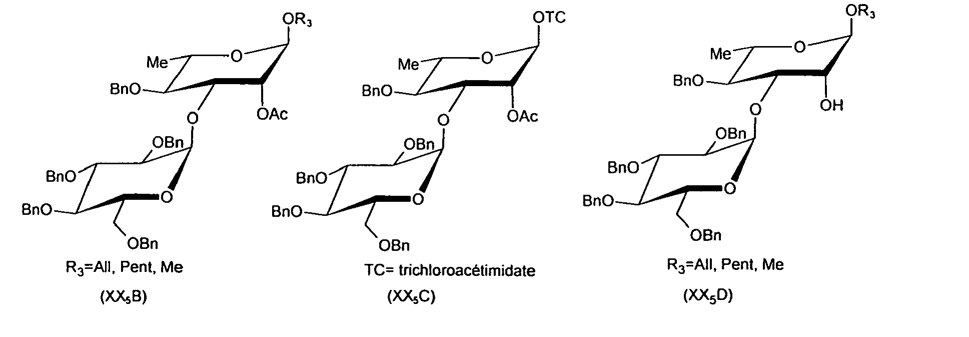 comprising at least one step of reacting a mutant of the wild type glycoside hydrolase as defined above in the presence of the acceptor of formula (IIa), advantageously with the acceptor of formula (II): 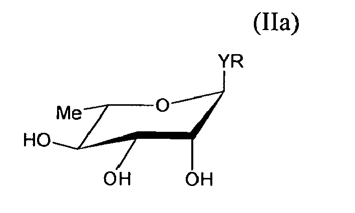 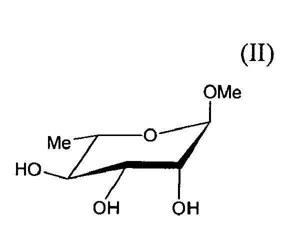 and with a donor of formula (IIIa) : 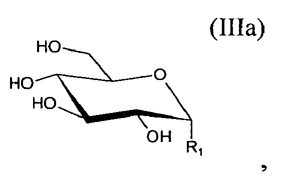 wherein R₁ can represent a group selected from: 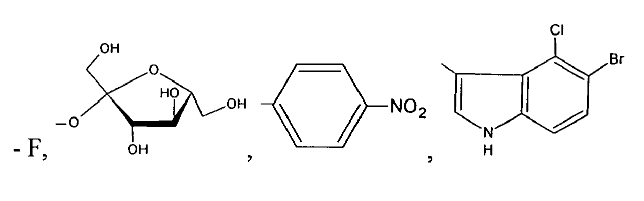 preferably the donor of formula (III):

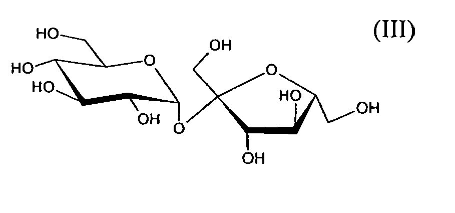

Some of the molecules obtained by the method of the invention are new and are another object of the invention. Among these are the following molecules:

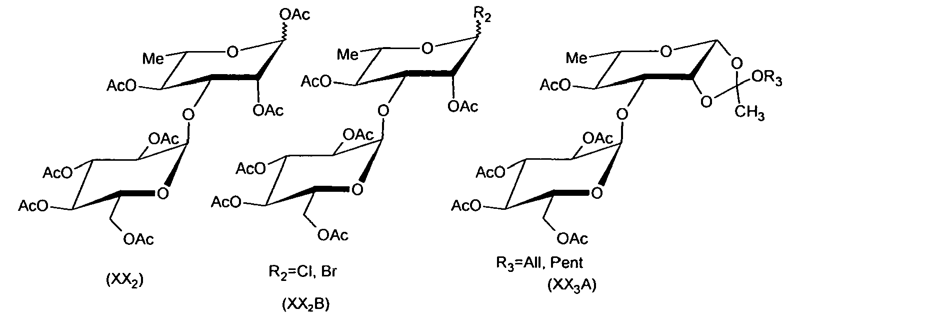

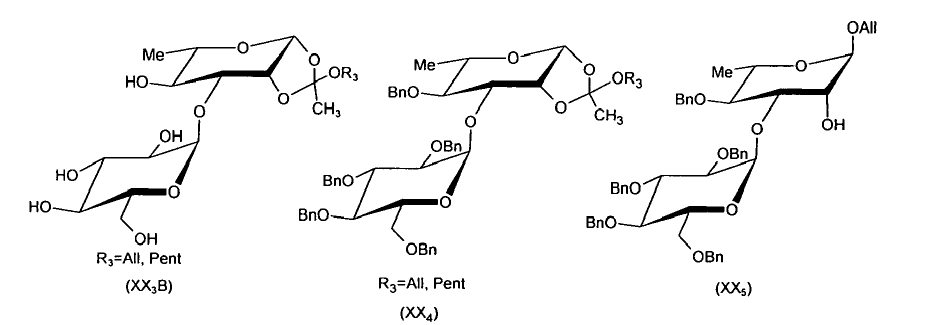

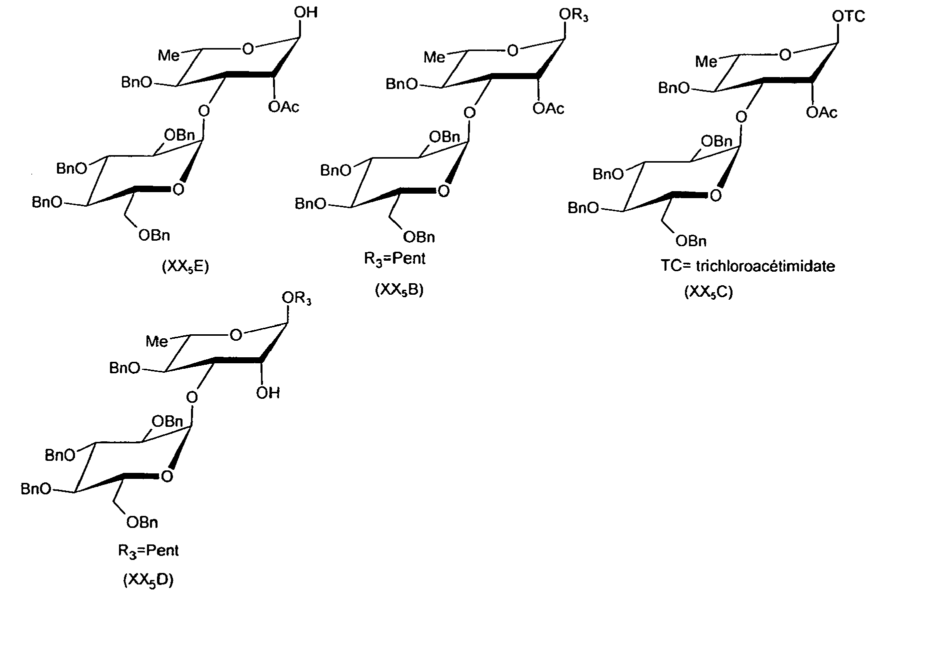

Another object of the invention is a mutant of a wild type glycoside hydrolase, said wild type glycoside hydrolase being defined as above, and said mutant having a mutation consisting of:
- the substitution of the amino acid residue at position 4 in said motif
(4) with any amino acid selected from the group consisting of lysine (K) and arginine (R), or
- the substitution of the amino acid residue at position 9 in said motif
(6) with any amino acid selected from the group consisting of cysteine (C), glutamic acid (E), isoleucine (I) and valine (V).

In a preferred embodiment of said mutant of a wild type glycoside hydrolase, it is a mutant of an amylosucrase from *Neisseria polysaccharea* having the amino acid sequence SEQ ID NO: 13, wherein said mutant, has in reference to SEQ ID NO: 13, a mutation consisting of:
- the substitution of the isoleucine (I) residue at position 228 (I228) with any amino acid selected from the group consisting of lysine (K) and arginine (R),
   or
- the substitution of the phenylalanine (F) residue at position 290 (F290) with any amino acid selected from the group consisting of cysteine (C), glutamic acid (E), isoleucine (I) and valine (V).
   The present invention also provides polynucleotides encoding a mutant of a glycoside hydrolase according to the present invention.
   Polynucleotides of the invention may be obtained by the well-known methods of recombinant DNA technology and/or of chemical DNA synthesis. These methods also allow introducing the desired mutations in a naturally occurring DNA sequence.
   The invention also provides recombinant DNA constructs comprising a polynucleotide of the invention, such as expression cassettes wherein said polynucleotide is linked to appropriate control sequences allowing the regulation of its transcription and translation in a host cell and optionally to a sequence encoding a GST tag allowing a rapid purification of the mutant enzymes and recombinant vectors comprising a polynucleotide or an expression cassette of the invention.
   In addition to the preceding features, the invention further comprises other features which will emerge from the following description, which refers to examples illustrating the present invention, as well as to the appended figures.

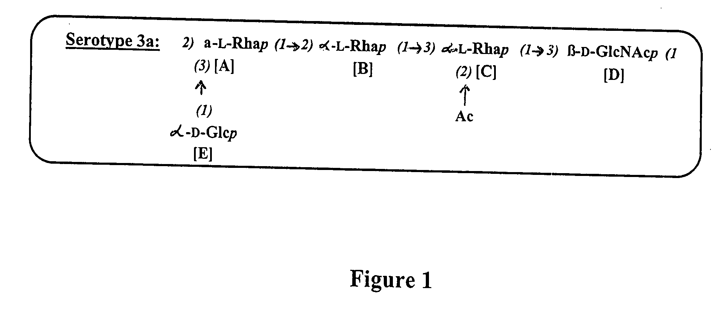

   Figure 1 shows the Repeating unit of *S*. *flexneri* serotype 3a O-Ag. Rha*p* = Rhamnopyranosyl - GlcNAc*p* = *N*-acetyl-glucosaminopyranosyl - Glc*p* = Glucopyranosyl, Ac = acetyl.
   Figure 2A shows a way to the chemo-enzymatic route to oligosaccharide fragments of *S*. *flexneri* 3a O-antigen.
   Figure 2B shows a way to the chemical synthetic route to oligosaccharide fragments of *S. flexneri* 3a O-antigen.
   Figure 2C shows a chemo-enzymatic synthesis of α-D-Glc*p*-(1→3)-α-L-Rha*p*-OMe.
   Figure 2D shows a chemo-enzymatic synthesis of oligosaccharides containing the R-(1→2)-[α-D-Glc*p*-(1→3)]-α-L-Rha*p* pattern.
   Figure 2E shows a a chemo-enzymatic synthesis of oligosaccharides containing the R-(1→2)-[α-D-Glc*p*-(1→3)]-α-L-Rha*p* pattern and some intermediates of interest.
   Figure 3 shows the reaction catalyzed by glucansucrases. Glucansucrases follow a double displacement retaining mechanism, in which an α-glucosyl enzyme covalent intermediate is first formed from sucrose substrate. In a second step, the glucosyl moiety is transferred to an acceptor which depending on the conditions of reaction may be (*i*) water to give glucose, *(ii)* fructose to form sucrose isomers, *(iii)* glucose released from hydrolysis to form soluble oligosaccharides, or *(iv)* an exogeneous hydroxylated acceptor.
   Figure 4 shows the architecture of the active site in complex with maltoheptaose (G7).
   Figure 5 shows the comparison of docking modes: (a) Maltose moiety from the crystallographic maltoheptaose (PDB: 1MW0) occupying binding subsites (-1) and (+1) of amylosucrase from *Neisseria polysaccharea* and (b) **α-D-Glc*p*-(1→3)-α-L-Rhap-OMe** in the active site of AS. The seven amino acid residues (I228, A289, F290, I330, V331, D394 and R446) selected for mutagenesis are shown on the figures. Hydrogen atoms have been omitted on the figures for clarity purpose.

Figure 6 shows the screening of the library for their ability to synthesize the desired disaccharide: **α-D-Glc*p*-(1→3)-α-L-Rha*p*-OMe.** Rows indicate the seven mutated positions and columns represent the twenty possible amino acid mutations including the wild type glycoside hydrolase.

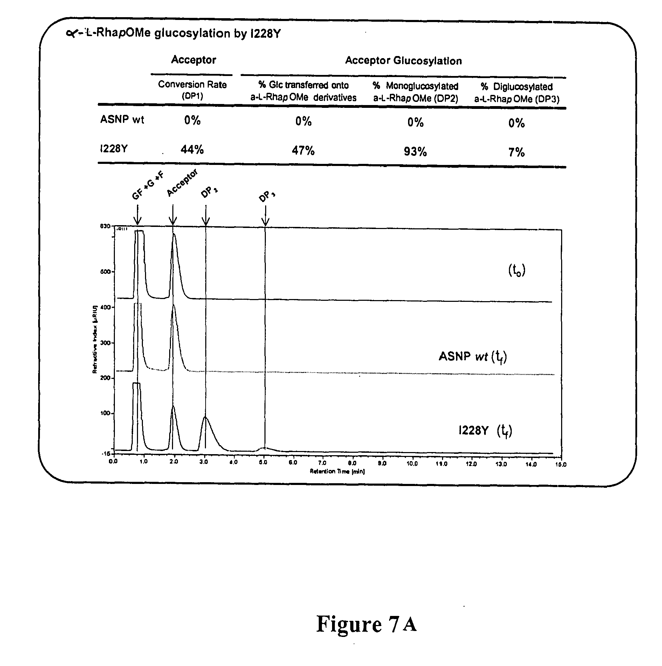

Figure 7A shows the transglucosylation of methyl α-L-rhamnopyranoside (formula II) using the most improved 1G5A variant I228Y and corresponding HPLC chromatogram (with RI detection) comparing I228Y and ASNPwt. Initial reaction conditions: Sucrose=Acceptor=146mM. At final time, sucrose was fully consumed. Conversion Rate= (Q(Acceptor)ₜ₀-Q(Acceptor)_{tf})/ Q(Acceptor)ₜ₀ where Q(X)= Quantity of X in moles. % Glc transferred onto acceptor derivatives = Q(Glucosyl units transferred onto acceptor derivatives)/ Q(Glucosyl units transferrable from initial sucrose). % Monoglucosylated acceptor = Q(Monoglucosylated acceptor)/ Q (acceptor derivatives). % Diglucosylated acceptor = Q(Diglucosylated acceptor)/ Q (acceptor derivatives). G=Glucose; F=Fructose; GF=Sucrose; DP₁= Acceptor; DP₂= Monoglucosylated acceptor; DP₃= Diglucosylated acceptor.

Figure 7B shows the comparison of Dionex HPAEC product profiles obtained at the end of the reaction (*t* = 24 h) with wild-type AS (1G5A) and the variant I288Y using 146 mM sucrose.

Figure 7C shows the comparison of Dionex HPAEC product profiles obtained at the end of the reaction (*t* = 24 h) with wild-type AS (1G5A) and the variant I288Y using 146 mM sucrose supplemented with 146 mM acceptor (methyl α-L-rhamnopyranoside).

Figure 7D shows the determination of kinetic parameters for the variant I228Y catalyzed reactions: (a) varied acceptor (b) varied donor. Figure 7D(a), varied donor: sucrose, constant acceptor: 250 mM. Figure 7D(b), varied acceptor: methyl α-L-rhamnopyranoside, constant donor: 250mM.

Figure 7E shows the comparison of kinetic parameters between AS wt (1G5A) and the variant I228Y.

Figures 8.1 to 8.8 show the sequence alignment of 34 wild type glycoside hydrolases using the CLUSTALW program under default parameters.

Figure 9 shows the alignment of the eleven different motifs found in 34 wild type glycoside hydrolases.

### EXAMPLE 1: Engineering transglucosidase for the synthesis the [α-D-Glcp(1→3)1-α-L-Rhap disaccharide

### 1) Materials and Methods

### Bacterial strains, plasmids and chemicals

Plasmid pGST-AS, derived from the pGEX-6P-3 (GE Healthcare Biosciences) and containing the *N. polysaccharea* amylosucrase encoding gene (ref. 45) was used for the construction of the AS single mutant library.

*E. coli* JM109 was used as host for the plasmid library transformation, gene expression and large-scale production of the selected mutants.

Sucrose, N-acetyl-D-glucosamine and glycogen were purchased from Sigma-Aldrich (Saint-Louis, MO, USA).

Methyl α-L-rhamnopyranoside (ref. 78), and the disaccharides of reference α-D-Glc*p*-(1→3)-α-L-Rha*p*-OMe (ref. 74) and α-D-Glc*p*-(1→4)-α-L-Rha*p-*OMe (ref. 63) were chemically synthesised at the Institut Pasteur (Paris, France).

Ampicillin, lysozyme and isopropyl β-D-thiogalactopyranoside (IPTG) were purchased from Euromedex (Souffelweyersheim, France), and *Dpn*I restriction enzyme from New England Biolabs (Beverly, MA, USA).

Oligonucleotides were synthesised by Eurogenetec (Liege, Belgium).

DNA extraction (QIASpin) and purification (QIAQuick) columns were purchased from Qiagen (Chatsworth, CA).

Wild type glycoside hydrolase: amylosucrase (ASNPwt) 1G5A of sequence SEQ ID NO: 13.

### Selection of mutation position by molecular modelling

*Starting models for the disaccharide and for AS:* The disaccharide α-D-Glc*p*-(1→3)-α-L-Rha*p*OMe (formula I) was constructed with the monosaccharide obtained from a database of carbohydrate three-dimensional structures. All molecular modelling calculations were performed using the SYBYL 7.3 software. The coordinates of amylosucrase were taken from the 2.0 Å resolution crystal structures of amylosucrase from *N. polysaccharea* in complex with sucrose (PDB: 1JGI) and maltoheptaose, a reaction product (PDB: 1MW0). All hydrogen atoms were added to the enzyme and their position optimized with the Tripos force field.

*Systematic conformational search for the disaccharides:* α-D-Glcp-(1→3)-α-L-Rha*p*OMe (formula I) was subjected to a systematic grid search study of the glycosidic linkage conformation. Starting from minimized disaccharide, a two-dimensional systematic conformational search was performed by rotating the two torsion angles defining the glycosidic linkages, Φ and Ψ by 20°steps: Φ = O5'-C1'-O3-C3' and Ψ = C1'-O3-C3'-C2' for α-D-Glc*p*-(1→3)-α-L-Rha*p*OMe. The MM3 force field implemented in SYBYL 7.3 software was used for this purpose together with the energy parameters appropriate for carbohydrates. Different maps were constructed with the dielectric constant set to 4.0 and 78.0 (to mimic gas phase and water environment, respectively). The geometries were optimized at each point of the grid with the driver option that keeps fixed the atoms defining the torsion angles. The solvent specific relaxed conformational maps obtained for the disaccharide were then used to locate the different energy minima that were subsequently fully relaxed.

*Docking of disaccharide in the binding site of AS:* The lowest energy conformations identified on the disaccharide potential energy maps were used as starting structures to be docked in the binding site of amylosucrase. This was performed by superimposing the glucosyl unit of the disaccharide at (-1) subsite onto the glucosyl unit of the crystallographic maltoheptaose. Each of these AS-disaccharide complexes was optimized by means of the appropriate energy parameters. The annealing method implemented in SYBYL 7.3 software was used to optimize the complexes. Two shells of amino acids were considered: a 12Å shell centred on the binding site was taken into account for the energy calculations. A 6Å shell region closest to the carbohydrate was defined as the hot region to be optimized. The position of all atoms included in this region was optimized using Powell's method.

### Construction of mutant library

Single mutagenesis, focused on +1 subsite amino acids retained from ligand docking, was carried out with the QuickChange Site-Directed Mutagenesis Kit (Stratagene, La Jolla, CA) according to the manufacturer's instructions, and using pGST-AS G537D as vector template. It was checked that this mutation had no impact on the native enzyme catalytic properties. The complementary primers listed below were used to obtain the single mutant library (Table II below). XXX codon indicates the bases which were used to obtain the replacement by the desired amino acids and are listed in Table III below.

**Table II: Primers used to generate the 19 monomutants for the positions 228, 289,290, 330, 331, 394 and 446.**

| SEQ ID NO: | Primer Name | Nucleotide Sequence |
|---|---|---|
| 14 | I228for | 5'-ACC CTG CGC GAA XXX TTC CCC GAC CAG CA-3' |
| 15 | I228rev | 5'-TG CTG GTC GGG GAA XXX TTC GCG CAG GGT-3' |
| 16 | A289for | 5'-T ATG GAT GCG GTT XXX TTT ATT TGG AAA CAA AT-3' |
| 17 | A289rev | 5'-AT TTG TTT CCA AAT AAA XXX AAC CGC ATC CAT A-3' |
| 18 | F290for | 5'-T ATG GAT GCG GTT GCC XXX ATT TGG AAA CAA AT-3' |
| 19 | F290rev | 5'-AT TTG TTT CCA AAT XXX GGC AAC CGC ATC CAT A-3' |
| 20 | I330for | 5'-TC AAA TCC GAA GCC XXX GTC CAC CCC GAC CAA GT-3' |
| 21 | I330rev | 5'-AC TTG GTC GGG GTG GAC GAT XXX TTC GGA TTT GA-3' |
| 22 | V331for | 4'-TC AAA TCC GAA GCC ATC XXX CAC CCC GAC CAA GT-3' |
| 23 | V331rev | 5'-AC TTG GTC GGG GTG XXX GAT GGC TTC GGA TTT GA-3' |
| 24 | D394for | 5'-TC CGC AGC CAC GAC XXX ATC GGC TGG ACG TTT-3' |
| 25 | D394rev | 5'-AAC CGT CCA GCC GAT XXX GTC GTG GCT GCG GA-3' |
| 26 | R446for | 5'-ACA GGC GAC TGC XXX GTC AGT GGT ACA-3' |
| 27 | R446rev | 5'-TGT ACC ACT GAC XXX GCA GTC GCC TGT-3' |

**Table III: Sequence of XXX codons used to replace each selected amino acids by the 19 other ones**

| | | Ala | Cys | Asp | Glu | Phe | Gly | His | Ile | Lys | Leu | Met | Asn | Pro | Gln | Arg | Ser | Thr | Val | Trp | Tyr |
|---|---|---|---|---|---|---|---|---|---|---|---|---|---|---|---|---|---|---|---|---|---|
| | for | GCC | TGC | GAC | GAG | TTC | GGC | CAC | wt | AAG | CTC | ATG | AAC | CCC | CAG | CGC | AGC | ACC | GTC | TGG | TAC |
| 228 | rev | GGC | GCA | GTC | CTC | GAA | GCC | GTG | wt | CTT | GAG | CAT | GTT | GGG | CTG | GCG | GCT | GGT | GAC | CCA | GTA |
| | for | wt | TGC | GAC | GAA | TTC | GGC | CAC | ATC | AAA | CTC | ATG | AAC | CCC | CAA | CGC | AGC | ACC | GTC | TGG | TAC |
| 289 | rev | wt | GCA | GTC | TTC | GAA | GCC | GTG | GAT | TTT | GAG | CAT | GTT | GGG | TTG | GCG | GCT | GGT | GAC | CCA | GTA |
| | for | GCT | TGT | GAT | GAA | wt | GGT | CAT | ATT | AAA | CTT | ATG | AAT | CCT | CAA | CGT | TCT | ACT | GTT | TGG | TAT |
| 290 | rev | AGC | ACA | ATC | TTC | wt | ACC | ATG | AAT | TTT | AAG | CAT | ATT | AGG | TTG | ACG | AGA | AGT | AAC | CCA | ATA |
| | for | GCC | TGC | GAC | GAA | TTC | GGC | CAC | wt | AAA | CTC | ATG | AAC | CCC | CAA | CGT | TCT | ACC | GTC | TGG | TAC |
| 330 | rev | GGC | GCA | GTC | TTC | GAA | GCC | GTG | wt | TTT | GAG | CAT | GTT | GGG | TTG | ACG | AGA | GGT | GAC | CCA | GTA |
| | for | GCC | TGC | GAC | GAA | TTC | GGC | CAT | ATC | AAA | CTC | ATG | AAC | CCC | CAA | CGC | TCC | ACC | wt | TGG | TAC |
| 331 | rev | GGC | GCA | GTC | TTC | GAA | GCC | ATG | GAT | TTT | GAG | CAT | GTT | GGG | TTG | GCG | GGA | GGT | wt | CCA | GTA |
| | for | GCC | TGC | wt | GAG | TTC | GGC | CAC | ATC | AAA | CTC | ATG | AAC | CCC | CAA | CGC | AGC | ACC | GTC | TGG | TAC |
| 394 | rev | GGC | GCA | wt | CTC | GAA | GCC | GTG | GAT | TTT | GAG | CAT | GTT | GGG | TTG | GCG | GCT | GGT | GAC | CCA | GTA |
| | for | GCT | TGT | GAT | GGT | TTT | GGT | CAT | ATT | AAA | CTT | ATG | AAT | CCT | CAA | wt | AGT | ACT | GTT | TGG | TAT |
| 446 | rev | AGC | ACA | ATC | ACC | AAA | ACC | ATG | AAT | TTT | AAG | CAT | ATT | AGG | TTG | wt | ACT | AGT | AAC | CCA | ATA |

PCR amplification was carried out with Pfu DNA polymerase (2.5 U) for 16 cycles (95°C, 30s; 55°C, 30s; 72°C, 12min). The DNA was digested with Dpnl to eliminate methylated parental template and purified using Qiaquick spin column, following manufacturer's recommendations. E. coli JM109 was transformed with the plasmid and plated on LB agar supplemented with 100µg/mL ampicillin. For each construction, two clones were isolated and their corresponding plasmids stored at -20°C. 17 mutants (1228A₁, I228V₁, I228Y1, A289D₁, F290D₁, F290K1, F290Q₁, I330A₁, 1330D₁, I330E₁, I330F₁, I330T₁, I330W₁, V331A₁, V331S₁, D394V₁ and R446K₁) were sequenced on the entire gene and showed no other mutations by Millegen (Labège, France) or Cogenics (Meylan, France).

### Expression of mutant library

The protocol was established to enable the rapid identification of clones able to glucosylate α-L-Rha*p*-OMe (formula II). To obtain higher amounts of enzymes and facilitate detection of glucosylated compounds upon HPLC screening, mutants were produced in 96-DeepWell Format plates. Storage microplates containing monomutants were thawed and replicated to inoculate a starter culture in 96-well microplates containing, in each well, 150µL LB medium supplemented with ampicillin (100µg/mL). After growth for 24h at 30°C under agitation (200 rpm), plates were duplicated into 96-Deep Well plates containing, in each well, 1.1 mL LB medium supplemented with ampicillin (100µg/mL) and IPTG (1mM) to induce GSTAS expression. Cultures were then grown for 24h at 30°C under agitation (200 rpm). Plates were centrifuged (20 min, 3000g, 4°C) and the supernatant was removed. The cell pellet was resuspended in 200µL of lysozyme (0.5 mg/mL), followed by freezing at -80°C for 8 to 12h. After thawing at room temperature, 100µL of sucrose and 100µL of acceptor (each at a final concentration of 73 mM) were added to each well. Enzymatic reaction was incubated at 30°C during 24h under agitation. The DeepWell plates were then centrifuged (20 min, 3000g, 4°C) and 300µL of the supernatant was transferred to a filter micro-plate (PVDF 0.2 µm) to be clarified. Supernatant filtration was carried out by centrifugation of the filter micro-plate (5 min, 2000g, 4°C) into a novel microplate for screening.

### Screening of mutant library

Efficiency of the glucosylation reaction was evaluated by HPLC analysis of the acceptor reaction product synthesized when using α-L-Rha*p*-OMe as acceptor using a Dionex P 680 series pump, a Shodex RI 101 series refractometer, a Dionex UVD 340 UVNis detector and an autosampler HTC PAL. HPLC analyses were performed using two columns: 1) a Biorad HPLC Carbohydrate Analysis column (HPX-87K column (300x7.8 mm)) maintained at 65°C, using ultra-pure water as eluent with a flow rate of 0.6 mL/min ; 2) a reversed phase analytical column (Synergi C18RPFusion, 4µm, 30x4.6 mm) kept at room temperature, with 1ml/min of ultra-pure water as eluent. HPX-87K column was used to determine sucrose consumption by RI detection. C18RPfusion column served to detect the production of α-D-Glc*p-*(1→3)-α-L-Rha*p*OMe.

### Production and Purification of the Selected Variant: I228Y

Production and purification of AS variant were performed as previously described (ref. 45). Since pure GST/AS fusion protein possesses the same function and the same efficiency as pure AS, enzymes were purified to the GST/AS fusion protein stage (96 kDa). The enzyme was desalted by size exclusion chromatography using P6DG columns (GE Healthcare Biosciences) at +4°C and stored in elution buffer (50 mM Tris-HCl, pH 7.0, 150 mM NaCl) at -80°C. The protein content was determined by micro-Bradford method, using bovine serum albumin as standard (ref. 55).

### Biochemical Characterization of the Selected Variant: I228Y

All assays were performed at 30°C in 50 mM Tris buffer, pH=7.0. For the acceptor reactions, I228Y and wild type AS were tested on α-L-Rha*p*-OMe (formula II) used as acceptor.

*Standard activity determination.* Specific activity of the purified enzymes was determined by measuring the initial rate of released fructose under standard conditions (146mM sucrose). Fructose concentration was determined using the dinitrosalycilic acid (DNS) method (ref. 56). One unit of AS variant corresponds to the amount of enzyme that catalyses the production of 1µmole fructose per minute in the assay conditions.

### Comparison of products synthesized by wild type and AS variant.

Reactions were performed in the presence of 146 mM sucrose alone or supplemented with 146 mM acceptor. The purified wild-type or mutated GST/AS were employed at 0.5 U/mL. The reactions were stopped by heating at 95°C for 5 min. The final mixture was centrifuged at 18 000g for 5 min. Different carbohydrate analyses were performed to compare the product profiles synthesized by ASNPwt and AS variant (I228Y): Soluble and insoluble oligosaccharides produced during the reaction were identified by HPAEC using a Dionex Carbo-Pack PA100 column at 30°C. Before analysis, the insoluble fraction was solubilised in KOH at a final total sugar concentration of 10g/kg. Mobile phase (150mM NaOH) was set at 1mL/min flow rate with a sodium acetate gradient (going from 6 to 500 mM within 120 min). Detection was performed using a Dionex ED40 module with a gold working electrode and an Ag/AgCl pH reference. Note that α-L-Rha*p*-OMe (acceptor) and its derivatives are not oxidable products and thus are not detectable by HPAEC. Sucrose, glucose, fructose, α-L-Rha*p*-OMe (acceptor) and its derivatives (glucosylation products) were quantified by HPLC, as previously described.

### Determination of kinetic parameters.

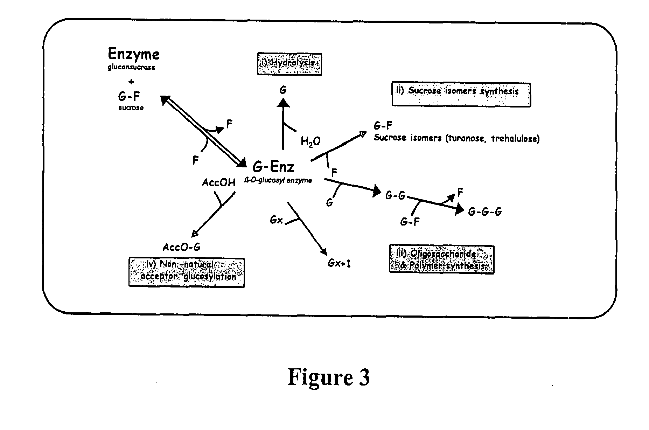

Enzyme assays were carried out in a total volume of 2 mL containing pure enzyme (0.115 mg and 2.6mg when using ASNPwt and I228Y, respectively). Catalytic efficiency (Eff=k_{cat}/K_{M}) of ASNPwt and I228Y variant were determined with both sucrose (D=Donor) and methyl α-L-rhamnopyranoside (A=Acceptor) as variable substrates. For the determination of the catalytic efficiency for D, A was held constant at 250 mM and D was varied between 0 and 600 mM. For the determination of the catalytic efficiency for A, D was held constant at 250 mM and A was varied between 0 and 500 mM. Experiments were performed until A and D solubility limits were reached. For each experiment, two distinct reaction velocities were determined: (1) the donor consumption determined by the release of fructose (called Vi (F)), corresponding to the overall kinetics of the different reactions described in Fig.3 and (2) the acceptor glucosylation determined by the formation of α-D-Glc*p*-(1→3)-α-L-Rha*p*OMe (called Vi(Dp2)), corresponding to the kinetics of the reaction of interest.

Initial velocities were fitted to the Michaelis-Menten equation using Sigma-Plot. As saturation was not achieved with the mutant, efficiency was calculated by linear regression analysis of the velocity versus substrate concentration plot. Aliquots (200µL) were removed between 0 and 60 min (at which time product formation was still linear with respect to time), heated at 95°C for 5 min and centrifuged at 18 000g for 5 min. The final mixtures were filtered on a 0.22 µm membrane and analyzed using HPLC material previously described. HPLC analyses were performed using two columns: 1) a Biorad HPLC Carbohydrate Analysis column (HPX-87H column (300x7.8 mm)) maintained at 30°C, using ultra-pure water as eluent with a flow rate of 0.6 mL/min ; 2) a reversed phase analytical column (Synergi C18RPFusion, 4µm, 30x4.6 mm) kept at room temperature, with 1ml/min of ultra-pure water as eluent. HPX-87H column was used to determine the released fructose by RI detection. C18RPfusion column was used to detect the formation of α-D-Glcp-(1→3)-α-L-Rha*p*OMe.

### Preparative synthesis of the acceptor reaction product (cf. Figure 2C)

**Methyl α-L-rhamnopyranoside (ref. 78):** To a solution of 5 g (27.4 mmol) of L-Rhamnose monohydrate in 50 mL of MeOH was added 5g of Dowex X8-200 ion exchange resin (H⁺). The reaction mixture was refluxed for 24h, cooled to room temperature, and filtered. The residue was purified by flash chromatography on silica gel (DCM/MeOH, 88/12) to give 4.4 g of known methyl α-L-rhamnopyranoside as a yellow syrup (90%) as the first eluting product, and 400 mg of methyl β-L-rhamnopyranoside (8%) as the second eluting one. Rf = 0.6 (8:2 DCM-MeOH); HRMS (ESI⁺) of C₇H₁₄O₅Na (M+Na, 201.0739) m/z 201.0753 ([M+Na]⁺). NMR data were identical to literature data.

A 500 ml mixture containing 146 mM sucrose, 100 mM methyl α-L-rhamnopyranoside (formula II) (ref. 78), and 0.25 U/ml of non-purified I228Y mutant extract was incubated at 30°C for 24h. Then, the reaction mixture was centrifuged (4800 rpm, 20 min, 4°C) to remove proteins and filtered on a 0.22 µm membrane

### Purification and characterisation of the target glucosylation product (Figure 2C): Methyl α-D-glucopyranosyl-(1→3)-α-L-rhamnopyranoside (formula I) (ref. 74).

*Methyl (2,3,4,6-tetra-O-acetyl-*α*-D-glucopyranosy*/*)-(1→3)-2,4-di-O-acetyl-*α*-L-rhamnopyranoside **(XX₁)*** (ref. 74). The crude mixture issued from the enzymatic glucosylation of methyl α-L-rhamnopyranoside (formula I, 9.1 g) by use of non-purified I228Y extract was purified by column chromatography (CH₃CN-H₂O, 9:1) to give contaminated unreacted acceptor (7.7 g) as the first eluting product, followed by the target disaccharide (formula I) contaminated by the more polar fructose (17.5 g). Remaining sucrose was identified as the last eluting product. Acetic anhydride (100 mL) was added dropwise to a solution of the whole mixture (17.5 g) in anhydrous pyridine (100 mL) stirred at 0°C. The solution was stirred overnight at room temperature. TLC (Toluene-EtOAc, 6:4) showed the complete disappearance of the starting materials and the presence of 3 less polar products. The mixture was concentrated under reduced pressure, and volatiles were eliminated by repeated coevaporation with toluene. The residue was purified by column chromatography (Toluene-EtOAc, 7:3) to give 4.9 g, (17% from methyl α-L-rhamnopyranoside) of methyl α-D-glucopyranosyl-(1→3)-α-L-rhamnopyranoside as a colourless amorphous solid. Rf = 0.45 (6:4 Toluene-EtOAc); ¹H NMR (CDCl₃) δ 5.30 (dd, 1H, J_{3,4} = 9.4 Hz, H-3_{E}), 5.18 (dd, 1H, J_{2,3} = 3.2 Hz, H-2_{A}), 5.11 (d, 1H, H-1_{E}), 5.08 (dd, 1H, J_{3,4} = 9.8 Hz, H-4_{A}), 5.05 (dd, 1 H, J_{4,5} = 9.4 Hz, H-4_{E}), 4.91 (dd, 1H, J_{1,2} = 3.4 Hz, J_{2,3} = 10.3 Hz, H-2_{E}), 4.61 (d, 1H, J_{1,2} = 1.7 Hz, H-1_{A}), 4.15 (dd_{overlaped}, 1H, J_{5,6aE} = 4.3 Hz, J_{6aE,6bE} = 12.4 Hz, H-6a_{E}), 4.13 (dd_{overlaped}, 1H, J_{5,6bE} = 2.5 Hz, H-6b_{E}), 4.07-4.02 (m, 2H, H-5_{E}, H-3_{A}), 3.72 (dq, 1H, J_{4,5} = 9.8 Hz, H-5_{A}), 3.33 (s, 1H, OCH₃), 2.13, 2.12, 2.06, 2.00, 1.98, 1.94 (6s, 18H, 6Ac), 1.21 (d, 3H, J_{5,6} = 6.3 Hz, H-6_{A}); ¹³C NMR (CDCl₃) δ 170.5, 170.0, 169.9, 169.8, 169.6, 169.3 (6C, 6C=O), 98.5 (C-1_{A}, ¹J_{CH} = 166.5 Hz), 93.6 (C-1_{E,} ¹J_{CH} = 174.6 Hz), 72.1 (C-3_{A}), 71.8 (C-4_{A}), 69.7 (2C, C-2_{E}, C-3_{E}), 68.5 (C-4_{E}), 67.9 (C-2_{A}), 67.7 (C-5_{E}), 66.6 (C-5_{A}), 61.6 (C-6_{E}), 55.0 (OCH₃), 20.9, 20.8, 20.7, 20.6, 20.5, 20.4 (6C, 6Ac), 17.5 (C-6_{A}). HRMS (ESI⁺) of C₂₅H₃₆O₁₆Na (M+Na, 615.1901) *m*/*z* 615.1861 ([M+Na]⁺); HRMS (ESI⁺) of C₂₅H₃₆O₁₆NH₄ (M+NH₄, 610.2347) m/z 610.2309 ([M+NH₄]⁺).

*Methyl α-D-glucopyranosyl-(1→3)-α-L-rhamnopyranoside (formula I)* (ref. 74). K₂CO₃ (21 mg, 0.15 mmol), was added to peracetylated XX₁ (300 mg, 0.51 mmol) in methanol (5 mL) and the suspension was stirred at room temperature. After 24h, TLC (DCM-MeOH, 8:2) showed the presence of a single more polar product. The suspension was filtered and volatiles were evaporated. The residue was purified by reverse phase chromatography to give the target disaccharide (formula I) as a lyophilized powder (160 mg, 93%). HRMS (ESI⁺) of C₁₃H₂₄O₁₀Na (M+Na, 363.1267) *m*/*z* 363.1304 ([M+Na]⁺). NMR data were identical to that of a reference (see above) compound issued from chemical synthesis.

### 2) Results

### Choice of acceptor substrates

The choice of a suitable rhamnosyl residue to be used as acceptor in the enzymatic glucosylation step was crucial. Three major features were taken into account: (i) light protecting pattern compatible with the limited ability of selected glucansucrases to modulate their acceptor binding site, (ii) easy synthetic access, and (iii) the possible conversion of the glucosylation product into a protected disaccharide building block known to be compatible with additional chemical elongation. In view of previous work on *S*. *flexneri* 5a synthetic oligosaccharides (ref. 64), methyl α-L-rhamnopyranoside (ref. 78), a crystalline material easily available in one step from commercially available L-rhamnose in 90% yield, was defined as the acceptor of choice, since it matched the above-defined major features. Indeed, the intermediate XX₁ resulting from the peracetylation of the enzymatic glucosylation product α-D-Glc*p*-(1→3)-α-L-Rha*p*-OMe (formula I) was converted into the known (ref. 61) allyl (2,3,4,6-tetra-*O*-benzyl-α-D-glucopyranosyl)-(1→3)-4-*O*-benzyl-α-L-rhamnopyranoside **(XX_{5D})** in 7 steps involving 4 purifications, and 54% overall yield. Noteworthy, disaccharide **XX_{5D},** acting as an acceptor and potential donor, is a known key intermediate in the synthesis of a large panel of *S*. *flexneri* 5a oligosaccharides (ref. 65). Analogously, it was more recently selected as an exquisite building block in the construction of several *S*. *flexneri* 3a O-antigen fragments. Alternatively, the peracetylated **XX₁** was converted to the analogous pentenyl glycosides **(XX_{5D}, R₃** = **Pent**). Interestingly, a number of intermediates involved in the conversion of α-D-Glc*p*-(1→3)-α-L-Rhc*p*-OMe (formula I) into the allyl or pentenyl glycosides **(XX_{5D}, R₃** = **All**) and **(XX_{5D}, R₃** = Pent), respectively, may also act as donors, as orthoesters **(XX₃, XX₄),** bromide **(XX_{2B}, R = Br**), or anomeric acetate **(XX₂),** and eventually potential acceptors. Additionnally, the acetolysis product **(XX₂)** may be converted to thioglycosides opening the way to new intermediates of potential interest.

### Screening for native glucansucrases able to synthesize the starting building block

Glucansucrases are α-retaining transglucosidases found in families 13 and 70 of glycoside-hydrolases (ref. 51). They catalyze the synthesis of α-glucan polymers by successive transfers of α-D-glucopyranosyl units from sucrose without any mediation of sugar nucleotides. Using the high energy of the sucrose bond to catalyze condensation reaction, they stand among the most efficient transglucosidases in the glycoside-hydrolase family. Depending on regiospecificity of the enzyme, distinct types of glucosidic linkage are found in the polymer formed. Notably, polymerization reaction can be redirected toward the glucosylation of exogenous acceptors, when the latter are well recognized (Figure 3). In addition, glucansucrases generally possess a broad acceptor spectrum, what indicates a certain plasticity of the acceptor recognition at the acceptor binding site. However, none of them had yet been tested for the glucosylation of the starting acceptor of interest. Glucosylation of methyl α-L-rhamnopyranoside (formula I) was thus attempted with four recombinant glucansucrases, which were selected for their very distinct specificities. Enzymes specific for α-1,6 and α-1,3, α-1,2 or α-1,4 glucosidic bond formation were tested in the presence of the target acceptor. None of them was able to glucosylate this acceptor with good yields and to achieve the expected regiospecificity.

Consequently, to overcome the limited substrate recognition by the enzymes, it was opted for the engineering of novel transglucosidases with altered regio and stereospecificities. The combination of combinatorial and rational enzyme engineering in the form of focused small size libraries was used. Further, the three-dimensional structure of AS (ref. 52 and 81) was available in complex with either the substrate or the natural reaction product.

### Engineering of amylosucrases able to glucosylate the target building blocks

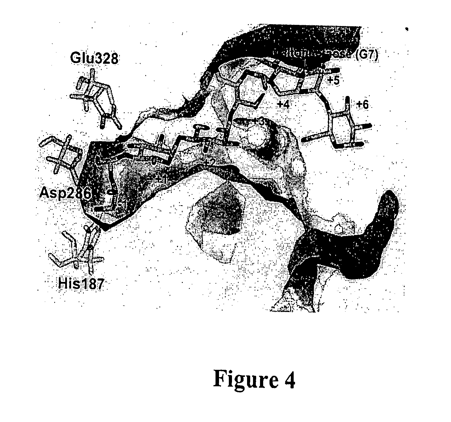

To modify enzyme specificity, an approach based on site-directed evolution targeted at the binding pocket was followed. The catalytic site pocket is defined by the subsites (-1) and (+1) according to the nomenclature earlier described for glycoside hydrolases (Figure 4). The subsite (-1) is responsible for the specificity towards sucrose and is occupied by the glucosyl unit which will be transferred whereas the subsite (+1) ensures a correct positioning of the acceptor and is also responsible for specificity of synthesis of the (α-1→4) glucan linkage (ref. 46).

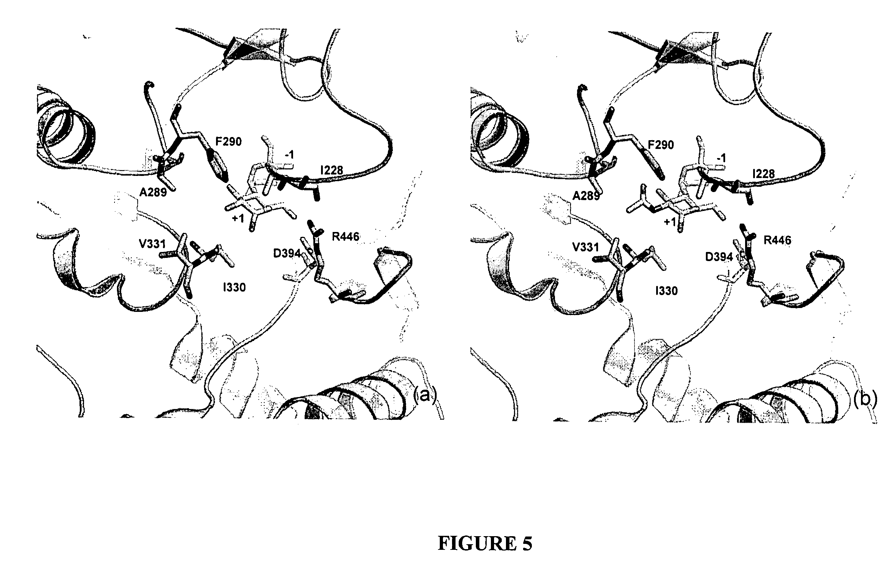

It was combined both the rational selection of mutation targets at the acceptor binding site (noted +1 subsite in Figure 4) and for each of the identified positions, a systematic modification of the residue by all 19 remaining possible amino acids. As (+1) subsite is responsible for the enzyme specificity toward acceptors, the approach consisted in (*i*) mapping the binding site residues important for functional plasticity and *(ii)* identifying the most promising positions to be modified to favour acceptor recognition. Starting from the crystallographic structure of AS in complex with sucrose (PDB: 1JGI) (ref. 53) and maltoheptaose (PDB: 1MW0) (ref. 54), the target disaccharide, α-D-Glc*p*-(1→3)-a-L-Rha*p*O-Me was docked in the AS active site (Figure 5).

### Structural analysis of α-D-Glcp-(1→3)-α-L-Rhap-OMe [EA-OMe] :

### AS complex

The desired disaccharide α-D-Glc*p*-(1→3)-α-L-Rha*p*-OMe was docked into the AS active site using the crystallographic maltose glucosyl units (ie α-D-Glc*p*-(1→4)-D-Glc*p*: native product) bound at (-1) and (+1) subsites (PDB: 1MW0) as a template for the starting location. The conformation of the disaccharide α-D-Glc*p*-(1→3)-α-L-Rhap-OMe in the enzyme pocket with best binding energy was considered out of all docking solutions. Comparison of the docking modes adopted by a-D-Glc*p*-(1→4)-D-Glcp and α-D-Glcp-(1→3)-α-L-Rhap-OMe, in particular at subsite (+1), revealed a 'flip' of the pyranose ring, due to the change in the chair ring conformation adopted by the α-L-Rhap-OMe unit (⁴C₁ vs ¹C₄, respectively) as well as a 'rotation' of the pyranose ring due to the altered osidic linkage (α-*1→4* vs α*-1→3,* respectively). As a result of these main structural differences, the network of interactions with the wild type enzyme was drastically impaired. A detailed comparison of both disaccharides bound in the enzyme pocket allowed to highlight the key amino acid side-chains in close contact with α-D-Glcp-(1→3)-α-L-Rha*p*-OMe that could be selected to facilitate the binding of the acceptor in a catalytically productive position and to provide a new network of interactions specific to α-L-Rhap-OMe (Figure 5). In particular, the corresponding amino acids Ile228, Ile330, Asp394 and Arg446 were proposed for mutation.

Out of the 18 residues identified as surrounding the (+1) subsite, 7 positions that were presumed to be not critical for sucrose binding but beneficial for target acceptor glucosylation have been selected for mutagenesis: Ile228, Ala289, Phe290, Ile330, Val331, Asp394 and Arg446. It was systematically mutated the selected amino acids by the 19 other possible residues to create a small size library focused on +1 subsite.

### Detection of mutants able to glucosylate the target acceptor

For each of the 7 selected positions, 19 single mutants were generated (corresponding to each possible amino acid change). Site-directed mutagenesis has been performed. A first library of 133 monomutants (7x19) was thus obtained and stored in cryotubes and 96-wells microplates. The mutants were tested for the glucosylation of the target acceptor α-L-Rhap-OMe in microtiter format experiments. HPLC screening was performed to identify those able to form the desired disaccharide. Both sucrose consumption and disaccharide formation were determined in order to calculate the glucosylation rate defined as the molar ratio of monoglucosylated acceptor versus/sucrose consumed.

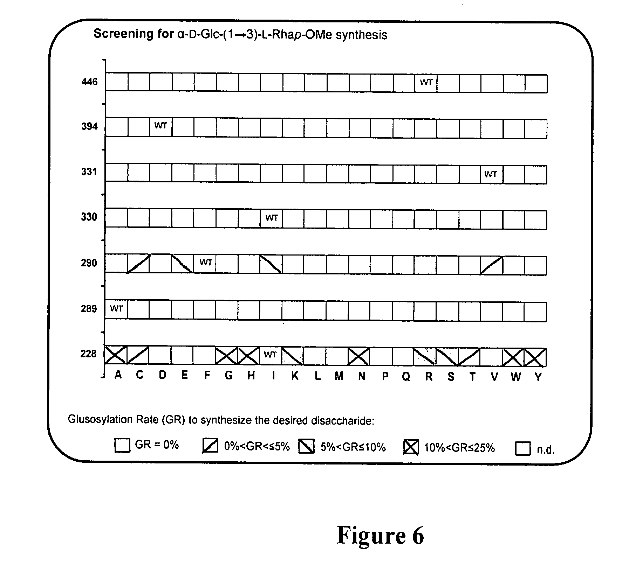

The wild type AS does not recognize the α-L-Rha*p*O-Me as an acceptor. As shown in Figure 6, the strategy enabled to isolate 15 mutants that display a totally new acceptor substrate specificity. Analysis of the acceptor reaction products further revealed that position 228 is crucial for α-D-Glc*p*-(1→3)-α-L-Rhap-OMe synthesis. Of the 19 mutants at position 228, 11 are able to form the desired disaccharide. Notably, I228Y, I228H and I228W yielded the desired product with a glucosylation rate of more than 15%. (respectively 23, 18 and 17%) Mutations at position 290 also improved the α-L-Rha*p*O-Me recognition but to a lesser extent. Only 4 of the 19 mutants are able to form the desired disaccharide but with lower yield (<5%). No positive results have been obtained for the positions 289, 330, 331, 394, and 446.

In overall, the most improved mutant of interest for the chemo-enzymatic pathway was retained and further characterized: I228Y which is specific for the production of α-D-Glc*p*-(1→3)-α-L-Rha*p*O-Me.

### Characterization of I228Y

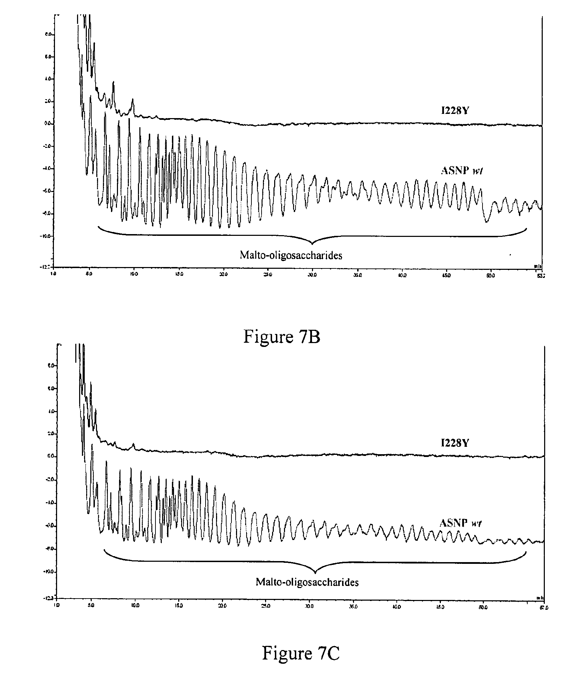

I228Y was produced in a larger amount and purified to homogeneity for further characterization. Glucosylation reactions with I228Y and wild type AS were performed using sucrose and in the absence or presence of α-L-Rha*p*O-Me. Distributions of the acceptor reaction products obtained with I228Y and wild type AS are shown in Figures 7A-c. Unlike wild type AS, I228Y lost its ability to elongate maltooligosaccharide chains (the highest Dp observed is 5) and to synthesize insoluble polymers in the sole presence of sucrose (Figure 7B). Hydrolysis is the major reaction occurring with I228Y in the sole presence of sucrose. When α-L-Rha*p*O-Me is added into the reaction medium, the HPAEC profile remains very similar (Figure 7C). However, HPLC profiles given in Figure 7A indicate that I228Y also recognizes α-L-RhapO-Me as an acceptor to form in majority a Dp2 corresponding to α-D-Glc*p-*(1→3)-α-L-Rha*p*O-Me and traces of higher Dp derivatives, that could not be detected by HPAEC analysis. Meanwhile, wild type AS does not recognize at all α-L-Rha*p*O-Me as an acceptor, as shown in Figure 7A These data also show that the presence of α-L-Rha*p*O-Me in the reaction medium does not affect the product profile obtained for wild type AS.

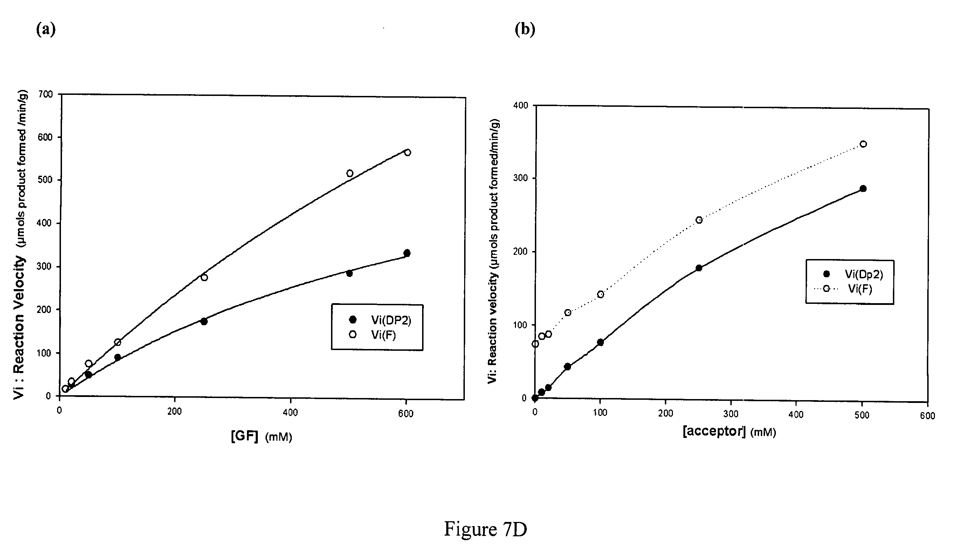

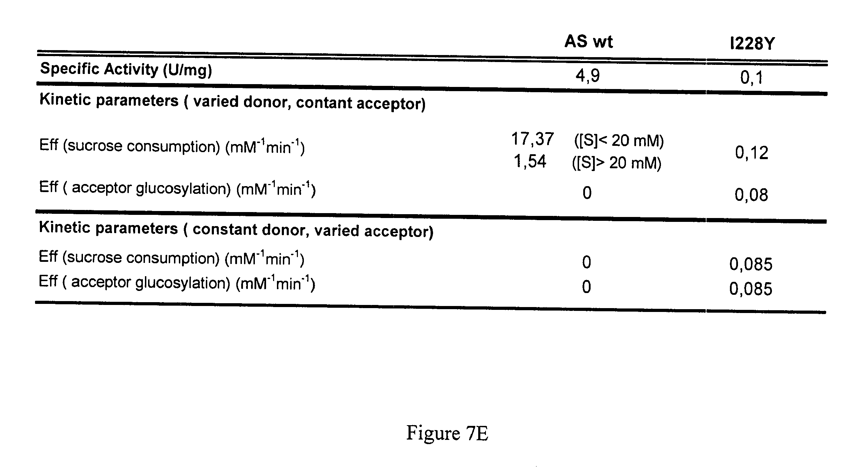

Comparison of kinetic parameters determined using sucrose as variable donor and α-L-Rha*p*O-Me as constant acceptor showed that catalytic efficiency towards sucrose is 13 to 150 times less active for I228Y variant than for wild type AS depending on sucrose concentration (Figures 7D (a) and 7E). However, the catalytic efficiency towards the desired reaction (acceptor glucosylation) clearly indicates that an enzyme with novel specificity has been obtained, adapted to sucrose usage for methyl α-L-rhamnopyranoside glucosylation. Noteworthy, Figure 7D (b) shows a clear activation of I228Y in the presence of α-L-Rha*p*O-Me. Activation of I228Y is induced by increasing concentration of acceptor and leading to an increase of Vi(Dp2) and therefore of Vi(F) in the same order of magnitude (Figure 7D (b)). Conversely, no activation of wild type AS is detected in the presence of the acceptor, as shown in Figure 7E where Vi(Dp2)=0 and Vi(F) remains constant, leading then to a null catalytic efficiency for reaction conditions corresponding to a sucrose concentration fixed at a constant 250mM and varied acceptor concentration.

### EXEMPLE 2: Enzymatic glucosylation of L-Rhap and α-L-Rhap-OAll

The seven 1G5A mutants I228A, I228G, I228H, I228N, I228S, I228W and I228Y were tested on other rhamnose derivatives (L-Rhap, α-L-Rha*p-*OAll) used as potential acceptors. The enzymatic reaction was carried out using sucrose as donor and rhamnose derivative as acceptor in a 1:1 ratio (146 mM for L-Rha*p*, 50 mM for α-L-Rha*p*-OAll).

Regarding glucosylation with L-rhamnose, α-(1→3) linkage is observed for all mutants except I228Y. All mutants except I228W are also able to glucosylate α-L-Rha*p*-OAll. This is an additional demonstration of the potency of the strategy considering that the wt enzyme lacks this newly described specific activity.

Efficiency of the glucosylation reaction was evaluated by HPLC analysis of the acceptor reaction product synthesized using a reversed phase analytical column (Synergi C18RPFusion, 4µm, 30x4.6 mm) (when testing L-Rha*p*) and a Biorad HPLC Carbohydrate Analysis column (HPX-87K column (300x7.8 mm)) when testing α-L-Rha*p*-OAll. (cf. Example 1/Materials and Methods/Screening of mutant library)

### EXAMPLE 3: Chemo-enzymatic synthesis of the disaccharide building block XX₅, compatible with the synthesis of oligosaccharide fragments incorporating R→2)-[α-D-Glcp-(1→3)]-α-L-Rhap (R = glycosyl, H) as for exemple in S. flexneri 2b, 3a, 5a, 5b, and X, O-antigens (cf fig 2E)

**General methods.** TLC were performed on precoated slides of Silica Gel 60 F₂₅₄ (Merck). Detection was effected when applicable, with UV light, and/or by charring in orcinol (35mM) in 4N aqueous sulfuric acid and ethanol (95/5). Preparative chromatography was performed by elution from columns of Silica Gel 60 (particle size 0.040-0.063 mm). NMR spectra were recorded at 25°C for solutions in CDCl₃ or MeOD (400 MHz for ¹H, 100 MHz for ¹³C). Residual CHCl₃ (7.28 ppm for ¹H and 77.0 ppm for ¹³C), MeOH (3.31 ppm for ¹H and 49.0 ppm for ¹³C), and HOD (4.79 ppm) were used as internal references for solutions in CDCl₃, MeOD, and D₂O, respectively. Proton-signal assignments were made by first-order analysis of the spectra, as well as analysis of 2D ¹H-¹H correlation maps (COSY). Of the two magnetically non-equivalent geminal protons at C-6, the one resonating at lower field is denoted H-6a, and the one at higher field is denoted H-6b. The ¹³C NMR assignments were supported by 2D ¹³C-¹H correlations maps (HMBC and HSQC). Interchangeable assignments are marked with an asterisk in the listing of signal assignments. Sugar residues in disaccharides are serially lettered according to the lettering of the repeating unit of the *S. flexneri* 3a O-antigen (glucopyranosyl: E, and rhamnopyranosyl: A) and identified by a subscript in the listing of signal assignments. Electrospray Ionisation-Time of flight (ESI-TOF) mass spectra were recorded in the positive-ion mode using a 1/1 acetonitrile (CH₃CN)/water containing 0.1% formic acid ESI-TOF spectrometer-solution. Anhydrous dichloromethane (DCM) and dichloroethane (DCE) sold on molecular sieves were used as such. 4 Å powder molecular sieves was activated before use by heating at 250°C under vacuum.

**Methyl (2,3,4,6-tetra-*O*-acetyt-α-D-gtucopyranosyl)-(1→3)-2,4-di-*O*-acetyl-α-L-rhamnopyranoside (XX₁) (ref. 74).** The crude mixture issued from the enzymatic glucosylation of methyl α-L-rhamnopyranoside (9.06 g, 50.9 mmol) was purified by column chromatography (CH₃CN-H₂O, 9:1) to give a mixture of methyl α-D-glucopyranosyl-(1→3)-α-L-rhamnopyranoside (formula I) and fructose (17.5 g).

Acetic anhydride (100 mL) was added dropwise to a solution of the whole mixture (17.5 g) in anhydrous pyridine (100 mL) stirred at 0°C. The solution was stirred overnight at room temperature. TLC (Toluene-EtOAc, 6:4) showed the complete disappearance of the starting materials and the presence of three less polar products. The mixture was concentrated under reduced pressure, and volatiles were eliminated by repeated coevaporation with toluene. The residue was purified by column chromatography (Toluene-EtOAc, 7:3) to give **XX₁** (4.9 g, 17% from methyl α-L-rhamnopyranoside) as a colourless amorphous solid. Analytical data for compound **XX₁** were as described in Example 1.

**(2,3,4,6-Tetra-*O*-acetyl-α-D-glucopyranosyl)-(1→3)-1,2,4-tri*-O-*acetyl-α/β-L-rhamnopyranose (XX₂).** 4% Sulphuric acid solution in acetic anhydride (20 mL) was added dropwise to a solution of **XX₁** (4.0 g, 6.7 mmol) in anhydride acetic (40 mL) stirred at 0°C under argon. After one hour at room temperature, TLC (Cyclohexane-EtOAc, 6:4) showed the complete transformation of **XX₁** into a more polar product. The reaction mixture was cooled to 0°C, diluted with DCM, then washed with saturated NaHCO₃ and water. The organic layer was dried on Na₂SO₄, filtered, and concentrated under reduced pressure. The residue was purified by column chromatography (Cyclohexane-EtOAc, 6:4) to give a mixture of α/β: 90/10 **XX₂** (2.8 g, 90%) as a colourless amorphous solid. Compound **XX₂** had Rf = 0.4 (6:4 Toluene-EtOAc); ¹H NMR (CDCl₃) δ 5.99 (d, 1H, J_{1,2} = 2.0 Hz, H-1_{A}), 5.31 (dd, 1H, J_{3,4} = 9.4 Hz, H-3_{E}), 5.19 (dd, 1H, J_{2,3} = 3.1 Hz, H-2_{A}), 5.16-5.11 (m, 2H, H-1_{E}, H-4_{A}), 5.06 (dd, 1H, J_{4,5} = 10.0 Hz, H-4_{E}), 4.93 (dd, 1H, J_{1,2} = 3.4 Hz, J_{2,3} = 10.4 Hz, H-2_{E}), 4.17 (m, 2H, H-6a_{E}, H-6b_{E}), 4.10-4.05 (m, 2H, H-5_{E}, H-3_{A}), 3.81 (dq, 1H, J_{4,5} = 9.7 Hz, H-5_{A}), 2.15, 2.12, 2.06, 2.03, 1.98, 1.95, 1.93 (7s, 21H, 7Ac), 1.21 (d, 3H, J_{5,6} = 6.2 Hz, H-6_{A}); ¹³C NMR (CDCl₃) δ 170.4, 170.0, 169.9, 169.8, 169.5, 169.4, 169.3 (7C, 7C=O), 93.7 (C-1_{E}, ¹J_{CH} = 176.0 Hz), 90.8 (C-1_{A}, ¹J_{CH} = 176.3 Hz), 71.8 (C-3_{A}), 71.3 (C-4_{A}), 69.7 (2C, C-2_{E}, C-3_{E}), 69.1 (C-5_{A}), 68.4 (C-4_{E}), 67.8 (C-5_{E}), 67.0 (C-2_{A}), 61.6 (C-6_{E}), 20.8, 20.7, 20.6, 20.6, 20.5, 20.5, 20.4 (7C, 7Ac), 17.5 (C-6_{A}). HRMS (ESI⁺) of C₂₆H₃₆O₁₇Na (M+Na, 643.1850) m/z 643.1910 ([M+Na]⁺); HRMS (ESI⁺) of C₂₆H₃₆O₁₇NH₄ (M+NH₄, 638.2296) m/z 638.2353 ([M+NH₄]⁺).

**(2,3,4,6-Tetra-*O*-acetyt-α-D-gtucopyranosyt)-(1→**3**)-2,4-di-*O-*acetyl-α-L-rhamnopyranosyl Bromide (XX_{2B}).** A 33% HBr solution in acetic acid (15 mL) was added dropwise to a solution of **XX₂** (2.5 g, 4.0 mmol) in DCM (50 mL) stirred at room temperature. After 5h, TLC (Toluene-EtOAc, 6:4), volatiles were evaporated under reduced pressure. The resulting mixture was extracted with DCM (2 x 100 mL). The organic layer was washed with cold saturated NaHCO₃ (2 x 40 mL) and water (40 mL), dried on Na₂SO₄, filtered, and concentrated to dryness to provide crude **XX_{2B}** (R₂ = Br) (2.5 g) as a light yellow syrup. This material was used directly for the next step without further purification. Compound **XX_{2B}** (R₂ = Br) had Rf = 0.5 (Toluene-EtOAc, 6:4); ¹H NMR (CDCl₃) δ 6.29 (d, 1H, J_{1,2} = 1.2 Hz, H-1_{A}), 5.39 (dd, 1H, H-2_{A}), 5.30 (dd, 1H, J_{3,4} = 9.4 Hz, H-3_{E}), 5.18 (pt, 1H, H-4_{A}), 5.13 (d, 1H, J_{1,2} = 3.4 Hz, H-1_{E}), 5.03 (dd, 1H, J_{4,5} = 10.2 Hz, H-4_{E}), 4.92 (dd, 1H, J_{2,3} = 10.2 Hz, H-2_{E}), 4.47 (dd, 1H, J_{2,3} = 3.3 Hz, J₃,₄ = 10.0 Hz, H-3_{A}), 4.17 (m, 2H, H-6a_{E}, H-6b_{E}), 4.07 (ddd, 1H, J₅,_{6aE} = 3.1 Hz, J₅,_{6bE} = 7.0 Hz, H-5_{E}), 3.97 (dq, 1H, J_{4,5} = 9.8 Hz, H-5_{A}), 2.17, 2.16, 2.11, 2.04, 2.00, 1.96 (6s, 18H, 6Ac), 1.21 (d, 3H, J_{5,6} = 6.2 Hz, H-6_{A}); ¹³C NMR (CDCl₃) δ 170.5, 169.9, 169.8, 169.5, 169.4, 169.3 (6C, 6C=O), 94.5 (C-1_{E}, ¹J_{CH} = 174.3 Hz), 84.8 (C-1_{A}, ¹J_{CH} = 185.3 Hz), 71.7 (C-3_{A}), 71.5 (C-5_{A}), 71.2 (C-2_{A}), 71.1 (C-4_{A}), 69.9 (C-2_{E}), 69.6 (C-3_{E}), 68.4 (C-4_{E}), 68.1 (C-5_{E}), 61.7 (C-6_{E}), 20.7, 20.5, 20.4 (6C, 6Ac), 17.0 (C-6_{A}).

**(2,3,4,6-Tetra-*O*-acetyl-α-D-gtueopyranosyl)-(1→3)-4-**O**-acetyl-1,2-*O*-allyloxyethylidene-β-L-rhamnopyranose (XX₃).** 2,6-Lutidine (5 mL) was added to a stirred suspension of crude **XX_{2B}** (R₂ = Br, 1.7 g, 2.7 mmol) and allyl alcohol (5 mL) in anhydrous DCM (10 mL) containing 4 Å molecular sieves (1 g). The mixture was stirred at room temperature for 12h, at which time TLC (Toluene-EtOAc, 6:4) indicated the complete disappearance of **XX_{2B}** (R₂ = Br). The mixture was diluted with DCM (100 mL), washed with cold citric acid (3 x 20 mL), cold saturated NaHCO₃ (2 x 20 mL) and water (50 mL), dried on Na₂SO₄, filtered, and concentrated under reduced pressure to provide crude orthoester **XX₃** (1.6 g) as a light yellow syrup. This material was used directly for the next step without further purification. Compound **XX₃** had Rf = 0.45 (Toluene-EtOAc, 6:4); ¹H NMR (CDCl₃) δ 5.88 (m, 1H, CH=), 5.41 (pt, 1H, J_{3,4} = 10.4 Hz, H-3_{E}), 5.40 (d_{overlaped}, 1H, H-1_{E}), 5.38 (d, 1H, J_{1,2} = 2.4 Hz, H-1_{A}), 5.30 (m, 1H, Jₜᵣₐₙₛ = 17.2 Hz, =CH₂), 5.14 (m, 1H, J_{cis} = 13.2 Hz, =CH₂), 5.05 (dd, 1H, J_{4,5} = 9.5 Hz, H-4_{E}), 5.04 (pt, 1H, H-4_{A}), 4.74 (dd, 1H, J_{1,2} = 3.8 Hz, J_{2,3} = 10.2 Hz, H-2_{E}), 4.48 (dd, 1H, J_{2,3} = 4.1 Hz, H-2_{A}), 4.20 (dd_{overlaped}, 1H, J_{5,6aE} = 4.1 Hz, J_{6aE,6bE} = 12.4 Hz, H-6a_{E}), 4.16 (dd_{overlaped}, 1H, J₅,_{6bE} = 2.6 Hz, H-6b_{E}), 4.06-3.97 (m, 3H, OCH₂, H-5_{E}), 3.90 (dd, 1H, J_{2,3} = 4.2 Hz, J_{3,4} = 9.7 Hz, H-3_{A}), 3.39 (dq, 1H, J₄,₅ = 9.5 Hz, H-5_{A}), 2.10, 2.08, 2.06, 2.01, 2.00 (5s, 15H, 5Ac), 1.72 (s, 3H, CH₃ₒᵣₜₕₒ), 1.21 (d, 3H, J₅,₆ = 6.2 Hz, H-6_{A}); ¹³C NMR (CDCl₃) δ 170.8, 170.5, 169.7, 169.5, 169.4, (5C, 5C=O), 134.0 (=CH), 124.1 (Cₒᵣₜₕₒ), 116.8 (=CH₂), 97.4 (C-1_{A}, ¹J_{CH} = 172.6 Hz), 91.9 (C-1_{E}, ¹J_{CH} = 172.7 Hz), 75.0 (C-2_{A}), 73.3 (C-3_{A}), 71.2 (C-4_{E}), 70.8 (C-2_{E}), 69.7 (C-3_{E}), 69.1 (C-5_{A}), 68.5 (C-4_{A}), 67.7 (C-5_{E}), 63.3 (OCH₂), 61.6 (C-6_{E}), 25.3 (CH₃ₒᵣₜₕₒ), 20.7, 20.6, 20.5 (5C, 5Ac), 17.5 (C-6_{A}). HRMS (ESI⁺) of C₂₇H₃₈O₁₆Na (M+Na, 641.2057) m/z 641.2024 ([M+Na]⁺); HRMS (ESI⁺) of C₂₇H₃₈O₁₆NH₄ (M+NH₄, 636.2504) m/z 636.2498 ([M+NH₄]⁺).

**α-D-Glueopyranosyl-(1→3)-1,2-*O*-aHytoxyethylidene-β-L-rhamnopyranose (XX_{3B}, R₃ = All).** Anhydrous K₂CO₃ (200 mg, 1.5 mmol) was added to a stirred solution of crude **XX₃** (R₃ = All, 1.6 g, 2.7 mmol) in dry MeOH (20 mL). The mixture was stirred at room temperature for 6h, at which time TLC (DCM-MeOH, 8.5:1.5) indicated total conversion of **XX₃** (R₃ = All) into a single product. Volatiles were removed under reduced pressure to give crude **XX_{3B}** (R₃ = All, 1.1 g) as a pale foam. This material was used directly for the next step without further purification. Compound **XX_{3B}** (R₃ = All) had Rf = 0.35 (DCM-MeOH, 8.5:1.5); ¹H NMR (MeOD) δ 5.92 (m, 1H, CH=), 5.45 (d, 1H, J_{1,2} = 2.2 Hz, H-1_{A}), 5.28 (m, 1H, Jₜᵣₐₙₛ = 17.3 Hz, =CH₂), 5.10 (m, 2H, =CH₂, H-1_{E}), 4.68 (dd, 1H, J_{2,3} = 3.6 Hz, H-2_{A}), 4.11 (m, 2H, OCH₂), 3.95 (m, 1H, J_{4,5} = 9.8 Hz, H-5_{E}), 3.89 (dd, 1H, J₃,₄ = 9.4 Hz, H-3_{A}), 3.83 (dd, 1H, 1₅,_{6aE} = 2.3 Hz, J_{6aE},_{6bE} = 11.9 Hz, H-6a_{E}), 3.76 (dd, 1H, J_{3,4} = 9.2 Hz, H-3_{E}), 3.72 (dd, 1H, J₅,_{6bE} = 4.7 Hz, H-6b_{E}), 3.51 (dd, 1H, J_{1,2} = 3.6 Hz, J_{2,3} = 9.7 Hz, H-2_{E}), 3.46 (pt, 1H, J_{4,5} = 9.3 Hz, H-4_{A}), 3.40-3.35 (m, 2H, H-5_{A}, H-4_{E}), 1.70 (s, 3H, CH₃ₒᵣₜₕₒ), 1.21 (d, 3H, J_{5,6} = 6.2 Hz, H-6_{A}); ¹³C NMR (MeOD) δ 134.4 (=CH), 123.4 (Cₒᵣₜₕₒ), 115.2 (=CH₂), 97.5 (C-1_{A}, ¹J_{CH} = 175.7 Hz), 95.9 (C-1_{E}, ¹J_{CH} = 170.5 Hz), 76.5 (C-2_{A}), 75.6 (C-3_{A}), 73.5 (C-3_{E}), 72.6 (C-5_{E}), 72.1 (C-2_{E}), 70.8 (C-5_{A}), 70.5 (C-4_{E}), 70.1 (C-4_{A}), 63.1 (OCH₂), 61.2 (C-6_{E}), 24.4 (CH₃ₒᵣₜₕₒ), 16.8 (C-6_{A}). HRMS (ESI⁺) of C₁₇H₂₈O₁₁Na (M+Na, 431.1529) *m*/*z* 431.1534 ([M+Na]⁺).

**(2,3,4,6-Tetra-*O*-benzyl-α-D-glucopyranosyl)-(1→3)-1,2-*O-*allyloxyethylidene-4-*O*-benzyl-β-L-rhamnopyranose (XX₄, R₃ = All).** Sodium hydride (60% suspension in oil, 1.6 g, 40.9 mmol, 15 eq.) was added portionwise to a solution of the crude pentaol **XX_{3B}** (R₃ = All) (1.2 g, 2.7 mmol) in dry DMF (20 mL), while the temperature was maintained below 5 °C. Stirring was continued for 1h at rt, then benzyl bromide (3.2 mL, 27.3 mmol, 10 eq.) was added dropwise to the reaction mixture kept under strong stirring below 10°C. The solution was then left stirring at rt for 48 h at room temperature when TLC (Cyclohexane-EtOAc, 7:3) indicated the presence of a single less polar product. The mixture was cooled to 0°C, and MeOH (2 mL) was added dropwise. After 2 h, EtOAc (200 mL) was added, and the organic phase was washed with water (3 x 40 mL), dried on Na₂SO₄, filtered, and concentrated to dryness. Chromatography of the residue (Cyclohexane-EtOAc, 9:1) gave orthoester **XX₄** (R₃ = All, 1.7 g, 74% over 4 steps, ie from per-*O*-acetylated **XX₂**) as a colourless syrup. Compound **XX₄** (R₃ = All) had Rf = 0.5 (Cyclohexane-EtOAc, 7:3); ¹H NMR (CDCl₃) δ 7.49-7.09 (m, 25H, Ph), 5.96 (m, 1H, CH=), 5.38 (d, 1H, J_{1,2} = 2.3 Hz, H-1_{A}), 5.37 (d, 1H, J_{1,2} = 3.5 Hz, H-1_{E})_{,} 5.30 (m, 1H, Jₜᵣₐₙₛ = 17.2 Hz, =CH₂), 5.18 (m, 1H, J_{cis} = 10.4 Hz, =CH₂), 5.06 (d, 1H, J = 11.0 Hz, OCH₂Ph), 4.98 (d, 1H, J = 10.1 Hz, OCH₂Ph), 4.93-4.87 (m, 3H, 3OCH₂Ph), 4.77 (d, 1H, J = 11.8 Hz, OCH₂Ph), 4.72 (dd, 1H, H-2_{A}), 4.66 (d, 1H, J = 10.1 Hz, OCH₂Ph), 4.58 (d, 1H, J = 12.1 Hz, OCH₂Ph), 4.51 (d, 1H, J = 11.0 Hz, OCH₂Ph), 4.38 (d, 1H, J = 12.1 Hz, OCH₂Ph), 4.17 (pt_{overlaped}, 1H, J_{3,4} = 9.3 Hz, H-3_{E}), 4.12 (m_{overlaped}, 2H, 2OCH₂), 4.06 (M_{overlaped}, 1H, H-5_{E}), 4.02 (dd_{overiaped}, 1H, J₂,₃ = 4.0 Hz, J_{3,4} = 9.2 Hz, H-3_{A}), 3.76-3.72 (m, 2H, H-2_{E}, H-4_{E}), 3.67 (dd_{overlaped}, 1H, J₅,_{6aE} = 4.0 Hz, H-6a_{E}), 3.65 (dd, 1H, H-4_{A}), 3.58 (dd, 1H, J₅,_{6bE} = 1.8 Hz, J_{6aE},_{6bE} = 10.8 Hz, H-6b_{E}), 3.36 (dq, 1H, J_{4,5} = 9.3 Hz, H-5_{A}), 1.83 (s, 3H, CH₃ₒᵣₜₕₒ), 1.42 (d, 3H, J_{5,6 =} 6.3 Hz, H-6_{A}); ¹³C NMR (CDCl₃) δ 138.7-137.6 (CPh), 134.2 (CH=), 128.8-127.5 (CHPh), 124.0 (Cₒᵣₜₕₒ), 116.5 (CH₂=), 97.6 (C-1_{A}, ¹J_{CH} = 173.6 Hz), 92.1 (C-1_{E}, ¹J_{CH} = 168.2 Hz), 82.2 (C-3_{E}), 78.9 (C-2_{E}), 78.6 (C-4_{A}), 77.5 (C-4_{E}), 76.2, 75.7, 75.0 (3C, 3OCH₂Ph), 74.9 (C-2_{A}), 73.9 (C-3_{A}), 73.3, 72.1 (2C, 2OCH₂Ph), 70.3 (2C, C-5_{E}, C-5_{A}), 68.3 (C-6_{E}), 63.6 (OCH₂), 25.1 (CH₃ₒᵣₜₕₒ), 17.9 (C-6_{A}). HRMS (ESI⁺) of C₅₂H₅₈O₁₁Na (M+Na, 881.3877) *m*/*z* 881.3655 ([M+Na]⁺).

**Allyl (2,3,4,6-tetra-*O*-benzyl-α-D-glucopyranosyl)-(1→3)-2-*O-*acetyl-4-*O*-benzyl-α-L-rhamnopyranoside (XX_{5B}, R₃ = All)** (ref. 83) and Allyl **(2,3,4,6-tetra-*O*-benzyt-α-D-glucopyranosyl)-(1→3)-2-*O*-acetyl-4-*O*-benzyt-β-L-rhamnopyranoside (XX_{5A}, R₃ = All):** TMSOTf (41 µL, 0.23 mmol, 0.3 eq.) was added to a solution of **XX₄** (R₃ = All) (640 mg, 0.75 mmol) in toluene (12 mL) containing 4 Å molecular sieves (650 mg), stirred at -78°C. The reaction mixture was stirred for 15 minutes. TLC (Cyclohexane-EtOAc, 7.3:2.7) showed the complete disappearance of the starting material and the presence of a major less polar product. Et₃N (0.3 mL) was added and the mixture was filtered, and concentrated to dryness. Chromatography of the residue (Cyclohexane-EtOAc, 85:15) gave firstly known **XX_{5B}** (R₃ = All) (540 mg, 85%) as a colourless syrup and secondly, Compound **XX_{5A}** (R₃ = All) the corresponding β anomer (Rf : 0.45, 65 mg, 10%). Compound **XX_{5B}** (R₃ = All) had Rf = 0.5 (Cyclohexane-EtOAc, 7.3:2.7); ¹H NMR (CDCl₃) δ 7.46-7.15 (m, 25H, Ph), 5.88 (m, 1H, CH=), 5.43 (dd, 1H, H-2_{A}), 5.31 (m, 1H, Jₜᵣₐₙₛ = 17.3 Hz, =CH₂), 5.24-5.21 (m, 2H, H-1_{E}, =CH₂), 5.06 (d, 1H, J = 11.0 Hz, OCH₂Ph), 4.99 (d, 1H, J = 10.2 Hz, OCH₂Ph), 4.92 (d, 1H, OCH₂Ph), 4.89 (d, 1H, OCH₂Ph), 4.82 (d, 1H, J_{1,2} = 1.6 Hz, H-1_{A}), 4.77 (d, 1H, J = 11.9 Hz, OCH₂Ph), 4.72 (d, 1H, OCH₂Ph), 4.65 (d, 1H, OCH₂Ph), 4.63 (d, 1H, OCH₂Ph), 4.52 (d, 1H, J = 11.0 Hz, OCH₂Ph), 4.40 (d, 1H, J = 12.1 Hz, OCH₂Ph), 4.37 (dd, 1H, J_{2,3} = 3.3 Hz, J_{3,4} = 9.6 Hz, H-3_{A}), 4.19 (M_{overlaped}, 1H, OCH₂), 4.14 (pt_{overlaped}, 1H, J_{3,4} = 9.5 Hz, H-3_{E}), 4.10 (M_{overlaped}, 1H, H-5_{E}), 4.00 (m, 1H, OCH₂), 3.84 (dq, 1H, J_{4,5} = 9.4 Hz, H-5_{A}), 3.85 (pt, 1H, J_{4,5} = 9.4 Hz, H-4_{E}), 3.68-3.61 (m, 3H, H-2_{E}, H-6a_{E}, H-4_{A}), 3.57 (dd, 1H, J₅,_{6bE} = 1.8 Hz, J_{6aE,6bE} = 10.9 Hz, H-6b_{E}), 1.99 (s, 3H, Ac), 1.43 (d, 3H, J_{5,6} = 6.3 Hz, H-6_{A}); ¹³C NMR (CDCl₃) δ 170.5 (C=O), 138.9-137.7 (CPh), 133.6 (CH=), 128.6-127.4 (CHPh), 117.5 (CH₂=), 96.7 (C-1_{A}, ¹J_{CH} = 170.4 Hz), 92.7 (C-1_{E}, ¹J_{CH} = 167.0 Hz), 82.2 (C-3_{E}), 79.9 (C-4_{A}), 79.4 (C-2_{E}), 77.9 (C-4_{E}), 76.2, 75.5, 75.0, 73.3, 73.0 (5C, 5OCH₂Ph), 72.2 (C-3_{A}), 70.2 (C-5_{E}), 68.3 (C-6_{E}), 68.1 (OCH₂), 68.0 (C-5_{A}), 67.9 (C-2_{A}), 20.9 (Ac), 18.0 (C-6_{A}). HRMS (ESI⁺) of C₅₂H₅₈O₁₁Na (M+Na, 881.3877) m/z 881.3854 ([M+Na]⁺); HRMS (ESI⁺) of C₅₂H₅₈O₁₁NH₄ (M+NH₄, 876.4323) m/z 876.4322 ([M+NH₄]⁺).

The β anomer **XX_{5A}**, (R₃ = All) had Rf = 0.45 (Cyclohexane-EtOAc, 7.3:2.7); ¹H NMR (CDCl₃) δ 7.43-7.05 (m, 25H, Ph), 5.88 (m, 1H, CH=), 5.66 (d, 1H, H-2_{A}), 5.33 (d, 1H, J_{1,2} = 3.5 Hz, H-1_{E}), 5.28 (m, 1H, 1ₜᵣₐₙₛ = **17.2** Hz, =CH₂), 5.25 (m, 1H, J_{cis} = 10.2 Hz, =CH₂), 5.03 (d, 1H, J = 11.0 Hz, OCH₂Ph), 4.96 (d, 1H, J = 10.2 Hz, OCH₂Ph), 4.94 (d, 1H, OCH₂Ph), 4.90-4.85 (m, 2H, OCH₂Ph), 4.78 (d, 1H, J = 11.4 Hz, OCH₂Ph), 4.72-4.56 (m, 3H, OCH₂Ph), 4.82 (bs, 1H, H-1_{A}), 4.77 (d, 1H, J = 12.1 Hz, OCH₂Ph), 4.31 (m, 1H, OCH₂), 4.14-4.06 (m, 3H, H-3_{E}, OCH₂, H-5_{E}), 3.95 (dd, 1H, J_{2,3} = 3.2 Hz, J_{3,4} = 9.6 Hz, H-3_{A}), 3.69 (pt, 1H, J_{4,5} = 9.6 Hz, H-4_{E}), 3.65-3.55 (m, 4H, H-2_{E}, H-6a_{E}, H-6b_{E}, H-4_{A}), 3.35 (dq, 1H, J_{4,5} = 9.2 Hz, H-5_{A}), 1.98 (s, 3H, Ac), 1.45 (d, 3H, J_{5,6} = 6.2 Hz, H-6_{A}); ¹³C NMR (CDCl₃) δ 170.7 (C=O), 138.8-137.5 (CPh), 133.6 (CH=), 128.9-127.4 (CHPh), 117.5 (CH₂=), 97.5 (C-1_{A}, ¹J_{CH} = 156.3 Hz), 92.0 (C-1_{E}, ¹J_{CH} = 169.0 Hz), 82.0 (C-3_{E}), 79.4 (C-4_{A}), 79.3 (C-2_{E}), 77.9 (C-4_{E}), 76.3, 75.6, 75.0, (3C, 2OCH₂Ph), 73.8 (C-3_{A}), 73.1, 72.9 (2C, 2OCH₂Ph), 71.9 (C-5_{A}), 70.0 (C-5_{E}), 69.8 (OCH₂), 68.4 (C-6_{E}), 66.7 (C-2_{A}), 20.8 (Ac), 17.9 (C-6_{A}). HRMS (ESI⁺) of C₅₂H₅₈O₁₁Na (M+Na, 881.3877) *m*/*z* 881.3854 ([M+Na]⁺); HRMS (ESI⁺) of C₅₂H₅₈O₁₁NH₄ (M+NH₄, 876.4323) m/z 876.4322 ([M+NH₄]⁺).

**Allyl (2,3,4,6-tetra-*O*-benzyl-α-D-glucopyranosyl)-(1→3)-4-*O-*benzyl-α-L-rhamnopyranoside (XX_{5D}, R₃ = All) (ref. 61):** To a solution of 0.5 M MeONa (1.5 mL, 0.75 mmol, 1.1 eq.) in DCM and MeOH (8:11), compound (**XX_{5B}**, R₃ = All, 565 mg, 0.66 mmol) was added, and the mixture was heated at 50°C for 12h. TLC (Cyclohexane-EtOAc, 7.3:2.7) showed the complete disappearance of the starting material and the presence of a single less polar product. The mixture was neutralized by addition of Dowex X8-200 ion exchange resin (H⁺), and filtered. Evaporation of the filtrate gave a syrup which was purified by chromatography (Cyclohexane-EtOAc, 80:20) to give known **XX_{5D}** (R₃ = All, 510 mg, 95%) as a colorless oil. Compound **XX_{5D}** (R₃ = All) had Rf = 0.45 (Cyclohexane-EtOAc, 7.3:2.7); ¹H NMR (CDCl₃) δ 7.25-7.02 (m, 25H, Ph), 5.78 (m, 1H, CH=), 5.18 (m, 1H, Jₜᵣₐₙₛ = 17.3 Hz, =CH₂), 5.10 (m, 1H, J_{cis}=10.4 Hz, =CH₂), 4.85 (d, 1H, J = 11.0 Hz, OCH₂Ph), 4.81 (m, 2H, H-1_{E}, OCH₂Ph), 4.80 (d, 1H, J_{1,2} = 1.2 Hz, H-1_{A}), 4.73 (d, 2H, J = 11.5 Hz, OCH₂Ph), 4.67-4.53 (m, 3H, OCH₂Ph), 4.43-4.37 (d, 2H, OCH₂Ph), 4.20 (d, 1H, J = 12.0 Hz, OCH₂Ph), 4.10 (m, 1H, OCH₂), 3.99 (pt, 1H, J_{3,4} = 9.3 Hz, H-3_{E}), 3.95-3.83 (m, 4H, H-3_{A}, OCH₂CH, H-2_{A}, H-5_{E}), 3.68 (dq, 1H, J_{4,5} = 9.3 Hz, H-5_{A}), 3.63 (pt, 1H, J_{4,5} = 9.7 Hz, H-4_{E}), 3.51 (dd, 1H, J_{1,2} = 3.6 Hz, J_{2,3} = 9.6 Hz, H-2_{E}), 3.41 (pt, 1H, J₃,₄ = 9.4 Hz, H-4_{A}), 3.37 (dd, 1H, J_{5,6aE} = 2.7 Hz, J_{6aE,6bE} = 10.9 Hz, H-6a_{E}), 3.30 (bs, 1H, OH), 3.24 (dd, 1H, J_{5,6bE} = 1.8 Hz, H-6b_{E}), 1.27 (d, 3H, J_{5,6} = 6.2 Hz, H-6_{A}); ¹³C NMR (CDCl₃) δ 139.0-137.9 (CPh), 134.2 (CH=), 129.4-127.9 (CHPh), 117.7 (CH₂=), 98.6 (C-1_{A}, ¹J_{CH} = 166.3 Hz), 94.4 (C-1_{E,} ¹J_{CH} = 166.9 Hz), 82.8 (C-3_{E}), 79.7 (C-4_{A}), 79.3 (C-2_{E}), 78.1 (C-4_{E}), 77.7 (C-3_{A}), 77.0, 76.0, 75.3, 74.7, 73.8 (5C, 5OCH₂Ph), 71.1 (C-5_{E}), 68.3 (C-6_{E}), 68.2 (OCH₂), 67.8 (C-2_{A}), 67.6 (C-5_{A}), 18.3 (C-6_{A}). HRMS (ESI⁺) of C₅₀H₅₆O₁₀Na (M+Na, 839.3771) m/z 839.3796 ([M+Na]⁺); HRMS (ESI⁺) of C₅₀H₅₆O₁₀NH₄ (M+NH₄, 834.4217) *m*/*z* 834.4251 ([M+NH₄]⁺).

**(2,3,4,6-Tetra-*O*-benzyl-α-D-glucopyranosyl)-(1→3)-2-*O*-acetyl-4-*O*-benzyl-α/β-L-rhamnopyranose (XX₅):** 1,5-Cyclooctadiene-bis(methyldiphenylphosphine)-iridium hexafluorophosphate (30 mg) was dissolved in THF (10 mL) and the resulting red solution was degassed under an argon stream. Hydrogen was then bubbled through the solution, causing the colour to change to yellow. The solution was then degassed again under an argon stream. A solution of compound **XX_{5B}** (R = All, 240 mg, 0.29 mmol) in THF (4 mL) was added. The mixture was stirred overnight at rt and TLC (Cyclohexane-EtOAc, 7.3:2.7) showed the complete disappearance of starting material and the presence of a single less polar product. The mixture was treated with a solution of iodine (150 mg, 0.58 mmol) in THF/water (10 mL, 4:1 v/v) and stirred for 1h at rt. TLC (Cyclohexane-EtOAc, 6:4) showed the complete disappearance of the intermediate and the presence of a single more polar product. Excess iodine was destroyed by adding a solution of freshly prepared 5% aqueous sodium bisulphite. DCM (50 mL) was added, and the organic phase was washed with brine (3 x 20 mL), water (3 x 20 mL), dried on Na₂SO₄, filtered, and concentrated to dryness. Chromatography of the residue (Cyclohexane-EtOAc, 7:3) gave hemiacetal **XX₅** (220 mg, 96%) as a white foam. Compound **XX₅** had Rf = 0.4 (Cyclohexane-EtOAc, 6:4); ¹H NMR (CDCl₃) δ 7.45-7.10 (m, 25H, Ph), 5.43 (dd, 1H, H-2_{A}), 5.24 (d, 1H, J_{1,2} = 3.5 Hz, H-1_{E}), 4.82 (d, 1H, J_{1,2} = 1.4 Hz, H-1_{A}), 5.06 (d, 1H, J = 11.0 Hz, OCH₂Ph), 4.99 (d, 1H, J = 10.3 Hz, OCH₂Ph), 4.93 (d, 1H, OCH₂Ph), 4.88 (d, 1H, OCH₂Ph), 4.77 (d, 1H, J = 11.8 Hz, OCH₂Ph), 4.72 (d, 1H, OCH₂Ph), 4.65 (d, 1H, J = 10.4 Hz, OCH₂Ph), 4.62 (d, 1H, J = 12.1 Hz, OCH₂Ph), 4.51 (d, 1H, OCH₂Ph), 4.38 (d, 1H, OCH₂Ph), 4.35 (dd, 1H, J_{2,3} = 3.8 Hz, J_{3,4} = 9.6 Hz, H-3_{A}), 4.16 (pt_{overlaped}, 1H, J_{3,4} = 9.3 Hz, H-3_{E}), 4.10 (m_{overlaped}, 1H, H-5_{E}), 4.04 (dq, 1H, J_{4,5} = 9.4 Hz, H-5_{A}), 3.75 (pt, 1H, J_{4,5} = 9.4 Hz, H-4_{E}), 3.67 (dd, 1H, J_{2,3} = 9.6 Hz, H-2_{E}), 3.65-3.60 (m, 2H, H-6a_{E}, H-4_{A}), 3.56 (dd, 1H, J_{5,6bE} = 1.8 Hz, J_{6aE,6bE} = 10.8 Hz, H-6b_{E}), 3.44 (d, 1H, J_{1,OH} = 3.9 Hz, OH), 1.99 (s, 3H, Ac), 1.41 (d, 3H, J_{5,6} = 6.2 Hz, H-6_{A}); ¹³C NMR (CDCl₃) δ 170.7 (C=O), 138.8-137.5 (CPh), 128.9-127.4 (CHPh), 92.5 (C-1_{E}, ¹J_{CH} = 170.0 Hz), 92.2 (C-1_{A}, ¹J_{CH} = 170.8 Hz), 82.1 (C-3_{E}), 79.8 (C-4_{A}), 79.4 (C-2_{E}), 77.9 (C-4_{E}), 76.2, 75.6, 75.0, 73.2, 73.0 (5C, 5OCH₂Ph), 71.7 (C-3_{A}), 70.1 (C-5_{E}), 68.3 (C-6_{E}), 68.2 (C-5_{A}), 68.0 (C-2_{A}), 20.9 (Ac), 18.0 (C-6_{A}). HRMS (ESI⁺) of C₄₉H₅₄O₁₁Na (M+Na, 811.3564) *m*/*z* 841.3602 ([M+Na]⁺); HRMS (ESI⁺) of C₄₉H₅₄O₁₁NH₄ (M+NH₄, 836.4010) m/z 836.4057 ([M+NH₄]⁺).

Compound **XX₅** may be obtained according to the same protocole when using Compound **XX₄** (R = All) as starting material. Alternatively, Compound **XX₅** can also result from conventional acidic hydrolysis of Compounf **XX₄** (X = All, Me, Pent).

**(2,3,4,6-Tetra-*O*-benzyi-α-D-gtucopyranosyl)-(1→3)-2-*O*-acetyl-4-*O*-benzyl-α/β-L-rhamnopyranosyl Trichloracetimidate (XX_{5c}):** The hemiacetal **XX₅** (130 mg, 0.16 mmol) was dissolved in DCE (10 mL), placed under argon, and cooled at -5°C. Trichloroacetonitrile (79 µL, 0.79 mmol) then DBU (6.6 µL, 44 µmol) were added. The mixture was stirred at -5°C for 10 min. TLC (Cyclohexane-EtOAc, 8:2) showed the complete disappearance of the starting material and the presence of a single less polar product. The mixture was directly chromatographied (Cyclohexane-EtOAc, 8:2) to give compound **XX_{5c}** (141 mg, 92%) as a white foam. Compound **XX_{5c}** had Rf = 0.45 (Cyclohexane-EtOAc, 8:2); ¹H NMR (CDCl₃) δ 8.75 (s, 1H, NH), 7.46-7.12 (m, 25H, Ph), 6.28 (d, 1H, J_{1,2} = 1.6 Hz, H-1_{A}), 5.66 (dd, 1H, H-2_{A}), 5.31 (d, 1H, J_{1,2} = 3.4 Hz, H-1_{E}), 5.09-4.91 (m, 4H, OCH₂Ph), 4.81 (d, 1H, J = 11.9 Hz, OCH₂Ph), 4.76 (d, 1H, OCH₂Ph), 4.68 (d, 1H, J = 10.1 Hz, OCH₂Ph), 4.63 (d, 1H, J = 12.1 Hz, OCH₂Ph), 4.56 (d, 1H, OCH₂Ph), 4.45 (d, 1H, OCH₂Ph), 4.38 (dd, 1H, J_{2,3} = 3.1 Hz, J_{3,4} = 9.6 Hz, H-3_{A}), 4.17 (pt, 1H, J_{2,3} = J_{3,4} = 9.3 Hz, H-3_{E}), 4.11 (m, 1H, H-5_{E}), 4.05 (dq, 1H, J_{4,5} = 9.6 Hz, H-5_{A}), 3.83 (pt, 1H, J_{4,5} = 9.6 Hz, H-4_{E}), 3.75 (pt, 1H, J_{4,5} = 9.6 Hz, H-4_{A}), 3.72-3.69 (m, 2H, H-6a_{E}, H-2_{E}), 3.55 (dd, 1H, J_{5,6bE} = 1.2 Hz, J_{6aE,6bE} = 10.7 Hz, H-6b_{E}), 2.05 (s, 3H, Ac), 1.49 (d, 3H, J_{5,6} = 6.2 Hz, H-6_{A}); ¹³C NMR (CDCl₃) δ 170.1(C=O), 160.2 (O-C=NH), 138.7-137.5 (CPh), 129.1-127.5 (CHPh), 95.1 (C-1_{A}, ¹J_{CH} = 178.6 Hz), 92.8 (C-1_{E}, ¹J_{CH} = 168.5 Hz), 90.9 (CCl₃), 82.2 (C-3_{E}), 79.3 (C-2_{E}), 79.1 (C-4_{A}), 77.8 (C-4_{E}), 76.5, 75.6, 75.1, 73.4, 73.0 (5C, 5OCH₂Ph), 71.8 (C-3_{A}), 71.0 (C-5_{A}), 70.3 (C-5_{E}), 68.2 (C-6_{E}), 66.1 (C-2_{A}), 20.7 (Ac), 18.0 (C-6_{A}).

**(2,3,4,6-tetra-*O*-aeetyt-α-D-gtucopyranosyl)-(1→3)-4-*O*-acetyl-1,2-*O*-pentenyloxyethylidene-β-L-rhamnopyranose (XX₃, R₃ = Pent)**: 2,6-Lutidine (2 mL) was added to a stirred suspension of crude **XX₂** (R₂ = Br, 750 mg, 1.2 mmol) and pentenyl alcohol (3 mL) in anhydrous DCM (5 mL) containing 4 Å molecular sieves (0.5 g). The mixture was stirred at room temperature for 12h, at which time TLC (Toluene-EtOAc, 6:4) indicated the complete disappearance of **XX₂** (R₂ = Br). The mixture was diluted with DCM (50 mL), washed with cold citric acid (3 x 10 mL), cold saturated NaHCO₃ (2 x 10 mL), and water (30 mL). The organic phase was dried on Na₂SO₄, filtered, and concentrated under reduced pressure to provide crude orthoester **XX₃** (R₃ = Pent, 760 mg) as a light yellow syrup. This material was used directly for the next step without further purification. Compound **XX₃** (R₃ = Pent) had Rf = 0.5 (Toluene-EtOAc, 6:4); ¹H NMR (CDCl₃) δ 5.80 (m, 1H, CH=), 5.41 (dd, 1H, J_{3,4} = 9.6 Hz, H-3_{E}), 5.38 (d, 1H, H-1_{E}), 5.3 (d, 1H, J_{1,2} = 2.4 Hz, H-1_{A}), 5.06-4.92 (m, 4H, H-4_{E}, H-4_{A}, =CH₂), 4.74 (dd, 1H, J_{1,2} = 3.7 Hz, J_{2,3} = 10.2 Hz, H-2_{E}), 4.46 (dd, 1H, H-2_{A}), 4.19 (dd_{overlaped}, 1H, J_{5,6aE} = 4.0 Hz, J_{6aE,6bE} = 12.4 Hz, H-6a_{E}), 4.16 (dd_{overlaped}, 1H, J_{5,6bE} = 2.7 Hz, H-6b_{E}), 4.00 (ddd, 1H, J_{4,5} = 10.3 Hz, H-5_{E}), 3.88 (dd, 1H, J_{2,3} = 4.2 Hz, J_{3,4} = 9.7 Hz, H-3_{A}), 3.46 (m, 2H, OCH₂), 3.38 (dq, 1H, J_{4,5} = 9.6 Hz, H-5_{A}), 2.15 (m, 2H, CH₂), 2.10, 2.08, 2.05, 2.01, 1.99 (5s, 15H, 5Ac), 1.71 (s, 3H, CH₃ₒᵣₜₕₒ), 1.65 (m, 2H, OCH₂C**H**₂), 1.20 (d, 3H, J_{5,6} = 6.2 Hz, H-6_{A}); ¹³C NMR (CDCl₃) δ 170.6, 170.5, 169.7, 169.5, 169.4 (5C, 5C=O), 138.2 (=CH), 124.1 (Cₒᵣₜₕₒ), 114.8 (=CH₂), 97.4 (C-1_{A}, ¹J_{CH} = 174.2 Hz), 91.9 (C-1_{E}, ¹J_{CH} = 173.6 Hz), 74.8 (C-2_{A}), 73.3 (C-3_{A}), 71.3 (C-4_{A}), 70.7 (C-2_{E}), 69.7 (C-3_{E}), 69.1 (C-5_{A}), 68.5 (C-4_{E}), 67.7 (C-5_{E}), 61.6 (OCH₂), 61.5 (C-6_{E}), 30.2 (CH₂), 28.7 (OCH₂CH₂), 25.0 (CH₃ₒᵣₜₕₒ), 20.7, 20.6, 20.5 (5C, 5Ac), 17.5 (C-6_{A}). HRMS (ESI⁺) of C₂₉H₄₂O₁₆Na (M+Na, 669.2371) *mlz* 669.2332 ([M+Na]⁺); HRMS (ESI⁺) of C₂₉H₄₂O₁₆NH₄ (M+NH₄, 664.2817) *mlz* 664.2789 ([M+NH_{4]}⁺).

**α-D-Glucopyranosyl-(1→3)-1,2-*O*-pentenyloxyethytidene-β-L-rhamnopyranose** (**XX_{3B}, R₃ = Pent):** Anhydrous K₂CO₃ (83 mg, 0.6 mmol) was added to a stirred solution of crude **XX₃** (R₃ = Pent, 760 mg, 1.2 mmol) in dry MeOH (10 mL). The mixture was stirred at room temperature for 6h, at which time TLC (DCM-MeOH, 8.5:1.5) indicated total conversion of **XX₃** (R₃ = Pent) into a single product. Volatiles were removed under reduced pressure to give crude **XX_{3B}** (R₃ = Pent, 510 mg) as a pale foam. This material was used directly for the next step without further purification. Compound **XX_{3B}** (R₃ = Pent) had Rf = 0.4 (DCM-MeOH, 8.5:1.5); ¹H NMR (MeOD) δ 5.82 (m, 1H, CH=), 5.43 (d, 1H, J_{1,2} = 2.2 Hz, H-1_{A}), 5.10 (m_{overlaped}, 1H, H-1_{E}), 5.03 (m_{overlaped}, 1H, Jₜᵣₐₙₛ = 17.3 Hz, =CH₂), 4.96 (m, 1H, J_{cis} = 10.2 Hz, =CH₂), 4.66 (dd, 1H, H-2_{A}), 3.94 (ddd, 1H, J_{4,5} = 9.8 Hz, H-5_{E}), 3.89 (dd, 1H, J_{2,3} = 4.0 Hz, J_{3,4} = 9.3 Hz, H-3_{A}), 3.84 (dd, 1H, J_{5,6aE} = 2.4 Hz, J_{6aE,6bE} = 12.0 Hz, H-6a_{E}), 3.77 (pt, 1H, J_{3,4} = 9.4 Hz, H-3_{E}), 3.72 (dd, 1H, J_{5,6bE} = 5.2 Hz, H-6b_{E}), 3.59 (m, 2H, OCH₂), 3.51 (dd, 1H, 1_{1,2} = 3.7 Hz, J_{2,3} = 9.7 Hz, H-2_{E}), 3.46 (pt, 1H, J_{4,5} = 9.3 Hz, H-4_{A}), 3.35 (m, 2H, H-5_{A}, H-4_{E}), 2.11 (m, 2H, CH₂), 1.67 (s, 3H, CH₃ₒᵣₜₕₒ), 1.63 (m, 2H, OCH₂C**H**₂), 1.31 (d, 3H, J_{5,6} = 6.0 Hz, H-6_{A}); ¹³C NMR (MeOD) δ 137.9 (=CH), 123.4 (Cₒᵣₜₕₒ), 113.9 (=CH₂), 97.5 (C-1_{A}, ¹J_{CH} = 174.0 Hz), 95.6 (C-1_{E,} ¹J_{CH} = 168.7 Hz), 76.4 (C-2_{A}), 75.4 (C-3_{A}), 73.4 (C-3_{E}), 72.6 (C-5_{E}), 72.1 (C-2_{E}), 70.8 (C-5_{A}), 70.4 (C-4_{E}), 70.0 (C-4_{A}), 61.3 (OCH₂), 61.2 (C-6_{E}), 30.0 (CH₂), 28.6 (OCH₂CH₂), 24.3 (CH₃ₒᵣₜₕₒ), 16.8 (C-6_{A}). HRMS (ESI⁺) of C₁₉H₃₂O₁₁Na (M+Na, 459.1842) *m*/*z* 459.1881 ([M+Na]⁺).

**(2,3,4,6-Tetra-*O*-benzyl-α-D-glucopyranosyl)-(1 →3)-1,2-*O-*pentenyloxyethylidene-4-*O*-benzyl-β-L-rhamnopyranose (XX₄, R₃ = Pent):** Sodium hydride (60% suspension in oil, 700 mg, 17.5 mmol) was added portionwise to a solution of the crude pentaol **XX_{3B}** (R₃ = Pent, 510 mg, 1.2 mmol) in dry DMF (15 mL), while the temperature was maintained below 5 °C. Stirring was continued for 1h at rt, then benzyl bromide (1.4 mL, 11.7 mmol) was added dropwise to the reaction mixture kept under strong stirring below 10°C. The solution was then left stirring for 48 h at room temperature when TLC (Cyclohexane-EtOAc, 7:3) indicated the presence of a single less polar product. The mixture was cooled to 0°C, and MeOH (1 mL) was added dropwise. After 2 h, EtOAc (100 mL) was added, and the organic phase was washed with water (3 x 20 mL), dried on Na₂SO₄, filtered, and concentrated to dryness. Chromatography of the residue (Cyclohexane-EtOAc, 9:1) gave orthoester **XX₄** (R₃ = Pent, 675 mg, 65% over 4 steps, ie from per-*O*-acetylated **XX₂**) as a colourless syrup. Compound **XX₄** (R₃ = Pent) had Rf = 0.55 (Cyclohexane-EtOAc, 7:3); ¹H NMR (CDCl₃) δ 7.51-7.10 (m, 25H, Ph), 5.83 (m, 1H, CH=), 5.42 (d, 1H, J_{1,2} = 3.6 Hz, H-1_{E}), 5.39 (d, 1H, J_{1,2} = 2.3 Hz, H-1_{A}), 5.09-4.88 (m, 7H, =CH₂, 5OCH₂Ph), 4.77 (d, 1H, J = 11.6 Hz, OCH₂Ph), 4.73 (dd, 1H, H-2_{A}), 4.68 (d, 1H, J = 10.1 Hz, OCH₂Ph), 4.60 (d, 1H, J = 12.1 Hz, OCH₂Ph), 4.53 (d, 1H, J = 11.0 Hz, OCH₂Ph), 4.39 (d, 1H, J = 12.0 Hz, OCH₂Ph), 4.19 (pt, 1H, J_{3,4} = 9.2 Hz, H-3_{E}), 4.08 (m, 1H, H-5_{E}), 4.04 (dd, 1H, J_{2,3} = 4.0 Hz, J_{3,4} = 9.4 Hz, H-3_{A}), 3.77 (dd_{overlaped}, 1H, J_{2,3} = 9.6 Hz, H-2_{E}), 3.76 (pt_{overlaped}, 1H, J_{4,5} = 9.6 Hz, H-4_{E}), 3.69 (dd_{overlaped}, 1H, J_{5,6aE} = 3.7 Hz, H-6a_{E}), 3.67 (pt_{overlaped}, 1H, H-4_{A}), 3.63-3.53 (m, 3H, H-6b_{E}, OCH₂), 3.37 (dq, 1H, J_{4,5} = 9.3 Hz, H-5_{A}), 2.13 (m, 2H, CH₂), 1.82 (s, 3H, CH₃ₒᵣₜₕₒ), 1.72 (m, 2H, OCH₂C**H**₂), 1.44 (d, 3H, J_{5,6}= 6.1 Hz, H-6_{A}); ¹³C NMR (CDCl₃) δ 138.8-137.6 (CPh), 138.0 (CH=), 128.8-127.5 (CHPh), 124.0 (Cₒᵣₜₕₒ), 115.0 (CH₂=), 97.6 (C-1_{A}, ¹J_{CH} = 174.9 Hz), 91.9 (C-1_{E}, ¹J_{CH} = 167.7 Hz), 82.1 (C-3_{E}), 79.0 (C-2_{E}), 78.7 (C-4_{A}), 77.5 (C-4_{E}), 76.2, 75.7, 75.0 (3C, 3OCH₂Ph), 74.9 (C-2_{A}), 73.9 (C-3_{A}), 73.3, 72.0 (2C, 2OCH₂Ph), 70.3 (2C, C-5_{E}, C-5_{A}), 68.4 (C-6_{E}), 61.8 (OCH₂), 30.3 (CH₂), 28.8 (OCH₂CH₂), 25.0 (CH₃ₒᵣₜₕₒ), 17.9 (C-6_{A}). HRMS (ESI⁺) of C₅₄H₆₂O₁₁Na (M+Na, 909.4190) *m*/*z* 909.4165 ([M+Na]⁺).

**Pentenyl (2,3,4,6-tetra-*O*-benzyl-α-D-glucopyranosyl)-(1→3)-2-*O*-acetyl-4-*O*-benzyl-α-L-rhamnopyranoside (XX_{5B}, R = Pent):** TMSOTf (27 µL, 0.15 mmol, 0.3 eq.) was added to a solution of **XX₄** (R₃ = Pent , 446 mg, 0.50 mmol) in toluene (12 mL) containing 4 A molecular sieves (450 mg), stirred at -78°C. The reaction mixture was stirred for 15 minutes. TLC (Cyclohexane-EtOAc, 7.3:2.7) showed the complete disappearance of the starting material and the presence of a major less polar product. Et₃N (0.2 mL) was added and the mixture was filtered, and concentrated to dryness. Chromatography of the residue (Cyclohexane-EtOAc, 85:15) gave compound **XX_{5B}** (R₃ = Pent, 330 mg, 74%) as a colourless syrup. Compound **XX_{5B}** (R₃ = Pent) had Rf = 0.55 (Cyclohexane-EtOAc, 7.3:2.7); ¹H NMR (CDCl₃) δ 7.43-7.10 (m, 25H, Ph), 5.85 (m, 1H, CH=), 5.41 (dd, 1H, J_{1,2} = 2.0 Hz, H-2_{A}), 5.24 (d, 1H, J_{1,2} = 3.5 Hz, H-1_{E}), 5.00-4.88 (m, 6H, =CH₂, 5OCH₂Ph), 4.78 (d, 1H, J = 11.5 Hz, OCH₂Ph), 4.77 (d, 1H, H-1_{A}), 4.72 (d, 1H, J = 11.9 Hz, OCH₂Ph), 4.65 (d, 1H, J = 10.2 Hz, OCH₂Ph), 4.64 (d, 1H, J = 12.0 Hz, OCH₂Ph), 4.53 (d, 1H, OCH₂Ph), 4.40 (d, 1H, OCH₂Ph), 4.28 (dd, 1H, J_{2,3} = 3.3 Hz, J_{3,4} = 9.6 Hz, H-3_{A}), 4.15 (pt, 1H, J_{3,4} = J_{2,3} = 9.3 Hz, H-3_{E}), 4.10 (m, 1H, J_{4,5} = 10.1 Hz, H-5_{E}), 3.83 (dq, 1H, J_{4,5} = 9.6 Hz, H-5_{A}), 3.79 (pt, 1H, H-4_{E}), 3.72-3.64 (m, 3H, OCH₂, H-2_{E}, H-6a_{E}), 3.79 (pt, 1H, H-4_{A}), 3.58 (dd, 1H, J_{5,6bE} = 1.9 Hz, J_{6aE,6bE} = 10.9 Hz,H-6b_{E}), 3.45 (m, 1H, OCH₂), 2.15 (m, 2H, CH₂), 1.99 (s, 3H, Ac), 1.71 (m, 2H, OCH₂C**H**₂), 1.44 (d, 3H, J_{5,6}= 6.2 Hz, H-6_{A}); ¹³C NMR (CDCl₃) δ 170.6 (C=O), 138.8-137.7 (CPh), 138.0 (CH=), 128.7-127.4 (CHPh), 115.0 (CH₂=), 97.6 (C-1_{A}, ¹J_{CH} = 170.7 Hz), 92.7 (C-1_{E}, ¹J_{CH} = 169.1 Hz), 82.2 (C-3_{E}), 79.9 (C-4_{A}), 79.4 (C-2_{E}), 77.9 (C-4_{E}), 76.3, 75.6, 75.0, 73.3, 73.0 (5C, 5OCH₂Ph), 72.3 (C-3_{A}), 70.1 (C-5_{E}), 68.3 (C-6_{E}), 68.0, 67.9 (2C, C-2_{A}, C-5_{A}), 67.3 (OCH₂), 30.3 (CH₂), 28.6 (OCH₂CH₂), 20.9 (Ac), 18.0 (C-6_{A}). HRMS (ESI⁺) of C₅₄H₆₂O₁₁Na (M+Na, 909.4190) m/z 909.4150 ([M+Na]⁺); HRMS (ESI⁺) of C₅₄H₆₂O₁₁NH₄ (M+NH₄, 904.4636) m/z 904.4603 ([M+NH₄]⁺).

**Pentenyl (2,3,4,6-tetra-*O*-benzyl-α-D-glucopyranosyl)-(1→3)-4-*O*-benzyl-α-L-rhamnopyranoside (XX_{5D}, R₃ =** Pent): To a solution of 0.5 M MeONa (0.55 mL, 0.28 mmol, 1.1 eq.) in DCM and MeOH (8:11), compound **XX_{5B}** (R₃ = Pent, 210 mg, 0.24 mmol) was added, and the mixture was heated at 50°C for 12h. TLC (Cyclohexane-EtOAc, 7.3:2.7) showed the complete disappearance of the starting material and the presence of a single less polar product. The mixture was neutralized by addition of Dowex X8-200 ion exchange resin (H⁺), and filtered. Evaporation of the filtrate gave a syrup which was purified by chromatography (Cyclohexane-EtOAc, 80:20) to give compound **XX_{5D}** (R₃ = Pent, 165 mg, 83%) as a colorless oil. Compound **XX_{5D}** (R₃ = Pent) had Rf = 0.5 (Cyclohexane-EtOAc, 7.3:2.7); ¹H NMR (CDCl₃) δ 7.38-7.13 (m, 25H, Ph), 5.83 (m, 1H, CH=), 5.05 (m, 1H, Jₜᵣₐₙₛ = 17.1 Hz, =CH₂), 5.00 (m, 1H, J_{cis} = 10.2 Hz, =CH₂), 4.97 (d, 1H, J = 11.0 Hz, OCH₂Ph), 4.95 (d, 1H, J_{1,2} = 3.0 Hz, H-1_{E}), 4.92 (d, 1H, J = 11.1 Hz, OCH₂Ph), 4.84 (d, 1H, J_{1,2} = 1.2 Hz, H-1_{A}), 4.86-4.83 (m, 2H, OCH₂Ph), 4.76 (d, 1H, J = 10.6 Hz, OCH₂Ph), 4.72 (d, 1H, J = 11.6 Hz, OCH₂Ph), 4.65 (d, 1H, J = 10.6 Hz, OCH₂Ph), 4.53 (d, 1H, J = 12.0 Hz, OCH₂Ph), 4.50 (d, J = 12.0 Hz, 1H, OCH₂Ph), 4.31 (d, 1H, OCH₂Ph), 4.06 (pt, 1H, J_{3,4} = 9.3 Hz, H-3_{E}), 4.01 (dd, 1H, h₃ = 3.4 Hz, J_{3,4}= 9.2 Hz, H-3_{A}), 3.95 (m_{overlaped}, 1H, H-5_{E}), 3.94 (m_{overlaped}, 1H, H-2_{A}), 3.77 (dq, 1H, J_{4,5} = 9.7 Hz, H-5_{A}), 3.74 (pt_{overlaped}, 1H, H-4_{E}), 3.61 (dd, 1H, J_{2,3}= 9.6 Hz, H-2_{E}), 3.50 (pt_{overlaped}, 1H, H-4_{A}), 3.47 (m_{overlaped}, 1H, H-6a_{E}), 3.42 (m, 1H, OCH₂), 3.36 (dd, 1H, J_{5,6bE} = 1.5 Hz, J_{6aE,6bE} = 10.7 Hz, H-6b_{E}), 2.12 (m, 2H, CH₂), 1.67 (m, 2H, OCH₂CH₂), 1.38 (d, 3H, J_{5,6}= 6.2 Hz, H-6_{A}); ¹³C NMR (CDCl₃) δ 138.7-137.6 (CPh), 138.1 (CH=), 128.7-127.0 (CHPh), 115.0 (CH₂=), 99.1 (C-1_{A}, ¹J_{CH} = 171.0 Hz), 94.0 (C-1_{E}, ¹J_{CH} = 166.8 Hz), 82.4 (C-3_{E}), 79.4 (C-4_{A}), 79.1 (C-2_{E}), 77.9 (C-4_{E}), 76.7 (C-3_{A}), 75.7, 75.6, 75.0, 74.3, 73.4 (5C, 5OCH₂Ph), 70.7 (C-5_{E}), 68.1 (C-6_{E}), 67.6 (C-2_{A}), 67.2 (C-5_{A}), 66.9 (OCH₂), 30.4 (CH₂), 28.7 (OCH₂CH₂), 18.0 (C-6_{A}). HRMS (ESI⁺) of C₅₂H₆₀O₁₁Na (M+Na, 867.4084) m/z 867.4130 ([M+Na]⁺); HRMS (ESI⁺) of C₅₂H₆₀O₁₁NH₄ (M+NH₄, 862.4530) *m*/*z* 862.4578 ([M+NH₄]⁺).

### REFERENCES

1. Gabius, H.J. et al., Chembiochem 5, 740-764 (2004).
2. Ruffing, A. & Chen, R.R., Microbial cell factories 5, 25 (2006).
3. Persidis, A., Nature biotechnology 15, 479-480 (1997).
4. Seeberger, P.H. & Werz, D.B., Nature 446, 1046-1051 (2007).
5. Kilcoyne, M. & Joshi, L., Cardiovascular & hematological agents in medicinal chemistry 5, 186-197 (2007).
6. Lee, J.C. et al., Nature protocols 1, 3143-3152 (2006).
7. Plante, O.J. et al., Science 291, 1523-1527 (2001).
8. Wang, C.C. et al., Nature 446, 896-899 (2007).
9. Joe, M. et al., Journal of the American Chemical Society 129, 9885-9901 (2007).
10. Drouillard, S. et al., Angewandte Chemie (International ed.) 45, 1778-1780 (2006).
11. Lindberg, A.A. Vaccine 17 Suppl 2, S28-36 (1999).
12. Ada, G. & Isaacs, D., Clin Microbiol Infect 9, 79-85 (2003).
13. Pirofski, L.A., Trends in microbiology 9, 445-451 (2001).
14. Nyame, A.K., et al., Archives of biochemistry and biophysics 426, 182-200 (2004).
15. Ragupathi, G., Cancer Immunol Immunother 43, 152-157 (1996).
16. Pozsgay, V., Advances in carbohydrate chemistry and biochemistry 56, 153-199 (2000).
17. Verez-Bencomo, V. et al., Science 305, 522-525 (2004).
18. Thiem, J., FEMS microbiology reviews 16, 193-211 (1995).
19. Koeller, K.M. & Wong, C.H., Nature 409, 232-240 (2001).
20. Yan, F., et al., Carbohydrate research 328, 3-16 (2000).
21. Hanson, S., et al., Trends Biochem Sci 29, 656-663 (2004).
22. Crout, D.H. & Vic, G., Current opinion in chemical biology 2, 98-111 (1998).
23. Schroven, A., et al., Chemistry 13, 9012-21 (2007).
24. Schmidt, D. et al., J Org Chem 65, 8518-8526 (2000).
25. Yoon, J.H. & Ajisaka, K., Carbohydrate research 292, 153-163 (1996).
26. Hancock, S.M. et al., Current opinion in chemical biology 10, 509-519 (2006).
27. Jank, T. et al., The Journal of biological chemistry 280, 37833-37838 (2005).
28. Zhang, J.H. et al., Proceedings of the National Academy of Sciences of the United States of America 94, 4504-4509 (1997).
29. Parikh, M.R. & Matsumura, I., Journal of molecular biology 352, 621-628 (2005).
30. Osanjo, G. et al., Biochemistry 46, 1022-1033 (2007).
31. Hancock, S.M. et al.,. Chembiochem 6, 866-875 (2005).
32. Kim, Y.W. et al., The Journal of biological chemistry 279, 42787-42793 (2004).
33. Honda, Y. & Kitaoka, M., The Journal of biological chemistry 281, 1426-1431 (2006).
34. Niggemann, J. et al., Bioorg Med Chem 6, 1605-1612 (1998).
35. Eichler, E. et al., Carbohydrate research 319, 1-16 (1999).
36. Mehta, S. et al., Org Lett 2, 751-753 (2000).
37. Yan, F. et al., J Org Chem 68, 2426-2431 (2003).
38. Yan, F. et al., Org Lett 3, 3265-3268 (2001).
39. Kotloff, K.L. et al., Bulletin of the World Health Organization 77, 651-666 (1999).
40. Levine, M.M. et al., Nature reviews 5, 540-553 (2007).
41. Lindberg, A.A. et al., Reviews of infectious diseases 13 Suppl 4, S279-284 (1991).
42. Jennison, A.V. & Verma, N.K., FEMS microbiology reviews 28, 43-58 (2004).
43. Phalipon, A. et al., J Immunol 176, 1686-1694 (2006).
44. Simmons, D.A., Biochemical Society transactions 18, 1271-1272 (1990).
45. De Montalk, G.P. et al., Journal of bacteriology 181, 375-381 (1999).
46. Albenne, C. et al., The Journal of biological chemistry 279, 726-734 (2004).
47. Yoon, S.H. & Robyt, J.F., Carbohydrate research 337, 2427-2435 (2002).
48. Cote, G.L. et al., Carbohydrate research 340, 257-262 (2005).
49. Richard, G. Carbohydrate research 340, 395-401 (2005).
50. Demuth, K. et al., Carbohydrate research 337, 1811-1820 (2002).
51. Coutinho, P.M. & Henrissat, B. in Recent Advances in Carbohydrate Bioengineering. (eds. H.J. Gilbert, G. Davies, B. Henrissat & B. Svensson) 3-12 (The Royal Society of Chemistry, Cambridge; 1999).
52. Skov, L.K. et al., Biol Crystallogr 56, 203-205 (2000).
53. Mirza, O. et al., Biochemistry 40, 9032-9039 (2001).
54. Skov, L.K. et al., The Journal of biological chemistry 277, 47741-47747 (2002).
55. Bradford, M.M., Analytical biochemistry 72, 248-254 (1976).
56. Sumner, J. & Howell, S.A., J Biol. Chem 108, 51-54 (1935).
57. Clement, M.J. et al., The Journal of biological chemistry 278, 47928-47936 (2003).
58. West, N.P. et al., Science 307, 1313-1317 (2005).
59. Allison, G.E. & Verma, N.K., Trends in microbiology 8, 17-23 (2000).
60. Ovodov, Y.S. et al., Biochemistry 71, 937-954 (2006).
61. Mulard, L. A. et al., Tetrahedron 58, 2593-604 (2002).
62. Lipkind G.M., et al., Carbohydr. Res. 181, 1-12 (1988).
63. Mulard, L. A., et al., J Carbohydr Chem. 19, 849-77 (2000).
64. Mulard, L. A. et al., J Carbohydr Chem. 18, 721-53 (1999).
65. Mulard, L. A. et al., Eur J Org Chem. 15, 2486-98 (2002).
66. Davies, G. & Henrissat, B., Structure 3, 853-9 (1995).
67. Henrissat, B., Biochem J. 280, 309-16 (1991).
68. Henrissat, B. & Bairoch, A., Biochem J 293, 781-8 (1993).
69. Stam, M. R. et al., Protein Eng Des Sel. 19, 555-62 (2006).
70. Janecek, S., Biologia 57, 29-41 (2002).
71. Nakajima, R. et al. Applied Microbiology and Biotechnology 23, 355-360 ( 1986).
72. Takata, H. et al. J Biol. Chem. 267, 18447-52 (1992).
73. Janecek, S. FEBS Lett. 377, 6-8 (1995).
74. Bakinovskii, L.V. et al., Bioorg. Khim. 11, 254-263 (1984).
76. Pozsgay, V., Org. Lett. 1, 477-479 (1999).
77. Manzoni, L. & Castelli R., Org. Lett. 8, 955-957 (2006).
78. Binkley, R. et al., Org. Chem. 49, 992-996 (1984).
79. Okuyama, M. et al., Biosci. Biotechnol. Biochem. 66, 928-933 (2002).
80. Rye, C.S.& Withers, S.G., Curr Opin Chem Biol. 4, 573-80 (2000).
81. Skov, L. et al., JBiol Chem. 276, 25273-8 (2001).
82. Gigg, C.D., J. Chem. Soc. C, 1903-1911 (1968).
83. Zhang K.Q., et al., Chem. J Chin. Univ. 18, 1469-73 (1997).

## Claims

1. A method for preparing the disaccharide [α-D-Glc*p*(1→3)]-α-L-Rha*p*-YR of formula (Ia), wherein Y is selected from -O- and -S- and R is selected from the group consisting of: C₁-C₆ alkyl, C₁-C₆ alkenyl, aryl, allyl, -CO-alkyl (C₁-C₆), -CO-alkenyl (C₁-C₆), -CO-aryl: 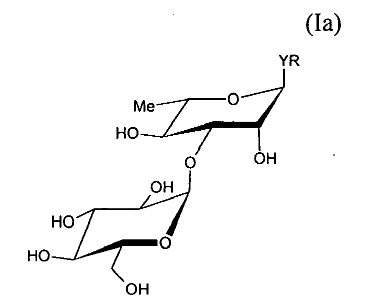 said method being **characterized in that** it comprises the step of reacting:
A) a mutant of a wild type glycoside hydrolase,
• wherein said wild type glycoside hydrolase has 450 to 850 amino acids and comprises from the N- to C-terminus eleven motifs defined by the following consensus sequences:
(1) the amino acid sequence LGVNYLHLMPL (SEQ ID NO: 1), which is located in the β-strand 2 of said wild type glycoside hydrolase;
(2) the amino acid sequence DGGYAV (SEQ ID NO: 2), which is located in the loop 2 of the (β/α)₈-barrel of said wild type glycoside hydrolase;
(3) the amino acid sequence DFVFNH (SEQ ID NO: 3) which is located in the β-strand 3 of said wild type glycoside hydrolase;
(4) the amino acid sequence LREIFPDTAPGNF (SEQ ID NO: 4), which is located in the domain B of said wild type glycoside hydrolase;
(5) the amino acid sequence FNSYQWDLN (SEQ ID NO: 5), which is located in the C-terminal part of the domain B of said wild type glycoside hydrolase;
(6) the amino acid sequence ILRLDAVAFLWK (SEQ ID NO: 6), which is located in the β-strand 4 of said wild type glycoside hydrolase;
(7) the amino acid sequence EAIV (SEQ ID NO: 7), which is located in the β-strand 5 of said wild type glycoside hydrolase;
(8) the amino acid sequence YVRCHDDI (SEQ ID NO: 8), which is located in the β-strand 7 of said wild type glycoside hydrolase;
(9) the amino acid sequence RISGTLASLAG (SEQ ID NO: 9), which is located in the domain B' of said wild type glycoside hydrolase;
(10) the amino acid sequence GIPLIYLGDE (SEQ ID NO: 10), which is located in the β-strand 8 of said wild type glycoside hydrolase;
(11) the amino acid sequence RWVHRP (SEQ ID NO: 11), which is located in the loop 8 of the (β/α)₈-barrel,
and the sequence formed by said eleven motifs joined end-to-end from motif (1) to motif (11) of said wild type glycoside hydrolase has at least 65% sequence identity or at least 80% sequence similarity with the amino acid sequence SEQ ID NO: 12;
• wherein said mutant has a mutation consisting of:
- the substitution of the amino acid residue at position 4 in said motif (4) with any amino acid selected from the group consisting of alanine (A), cysteine (C), glycine (G), histidine (H), lysine (K), asparagine (N), arginine (R), serine (S), threonine (T), tryptophan (W) and tyrosine (Y), or
- the substitution of the amino acid residue at position 9 in said motif (6) with any amino acid selected from the group consisting of cysteine (C), glutamic acid (E), isoleucine (I) and valine (V),
B) with the acceptor of formula (IIa): 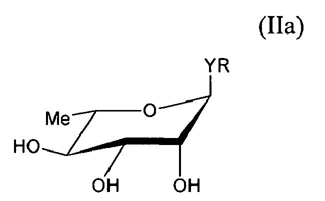 wherein Y and R are defined as above, and
C) with a donor of formula (IIIa) : 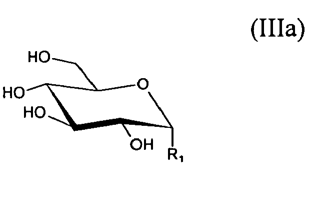
wherein R₁ represents a group selected from:

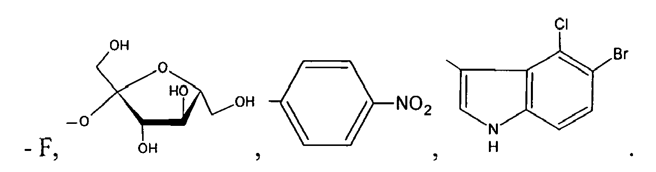

2. A method according to claim 1, **characterized in that** said wild type glycoside hydrolase is an amylosucrase (EC 2.4.1.4) or sucrose hydrolase (EC 3.2.1.-).

3. A method according to claim 2, **characterized in that** said wild type glycoside hydrolase is an amylosucrase from *Neisseria polysaccharea,* and is preferably selected from the group consisting of 1G5A (SEQ ID NO: 13), 1ZS2, 1MVY, 1MW0, 1S46, 1JGI, 1MW2, 1MW3, 1MW1 and 1JG9 proteins.

4. A method according to anyone of claims 1 to 3 for preparing the disaccharide [α-D-Glc*p*(1→3)]-α-L-Rha*p*-OMe of formula (I): 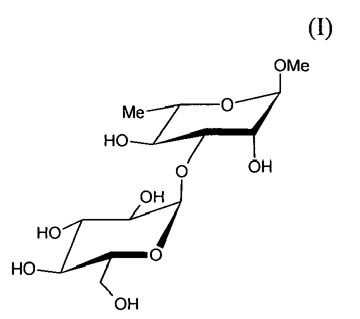 comprising the step of reacting a mutant of a wild type glycoside hydrolase as defined in anyone of claims 1 to 3, with the acceptor of formula (II) 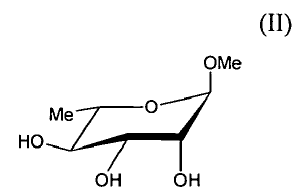 and with a donor of formula (IIIa) :

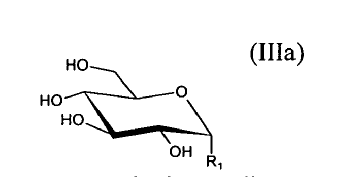

5. A method according to anyone of claims 1 to claim 4, wherein the acceptor of formula (IIIa) is sucrose.

6. A method for the preparation of the building block corresponding to the disaccharide of formula (XX₁): 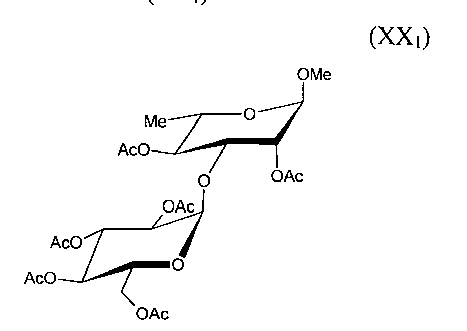 **characterized in that** it comprises at least one step according to anyone of claim 1 to claim 5.

7. A method for preparing any of the disaccharide of formula (XX₁), the disaccharide of formula (XX₂), the disaccharide of formula (XX_{2B}), , the disaccharide of formula (XX₃), the disaccharide of formula (XX_{3A}), the disaccharide of formula (XX_{3B}), the disaccharide of formula (XX₄), the disaccharide of formula (XX₅), the disaccharide of formula (XX_{5A}), the disaccharide of formula (XX_{5B}), the disaccharide of formula (XX_{5C}), the disaccharide of formula (XX_{5D}), or the disaccharide of formula (XX_{5E}): 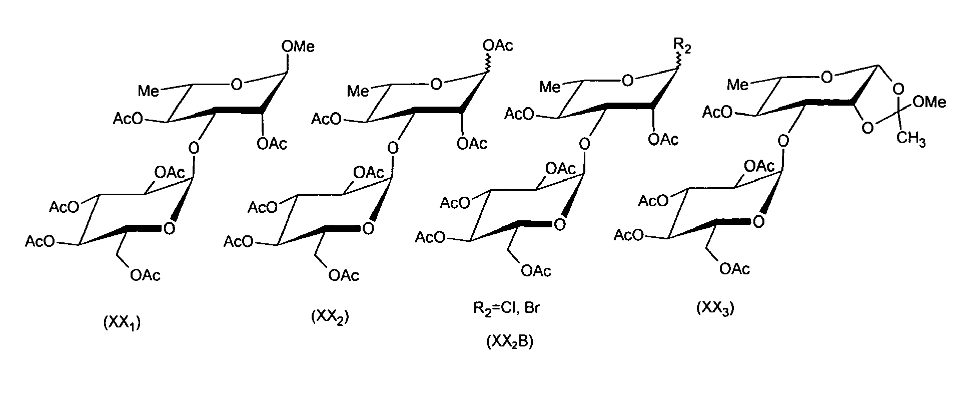 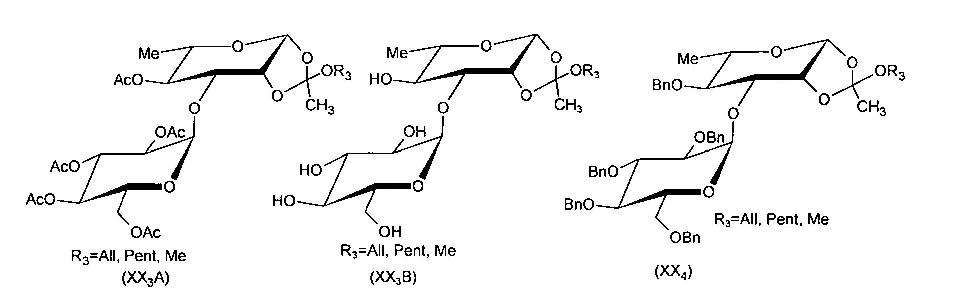 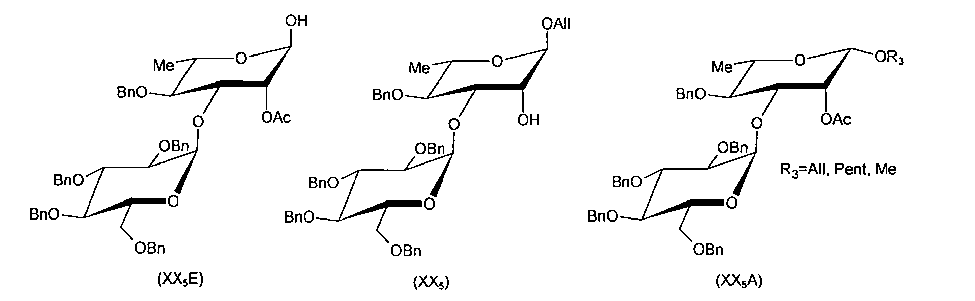 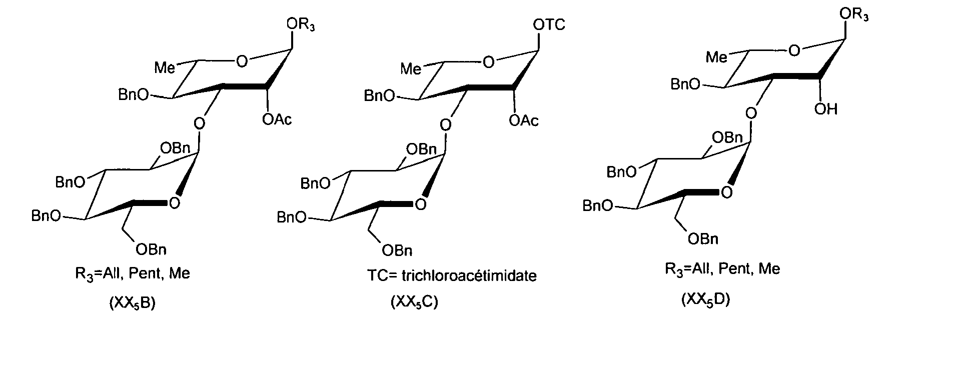 **characterized in that** it comprises at least one step according to anyone of claims 1 to 3.

8. A method for the preparation of the building block corresponding to the disaccharide of formula (XX₅): 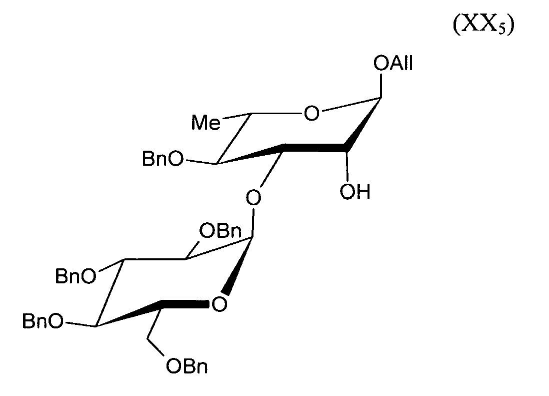 wherein it follows the steps according to scheme 1: 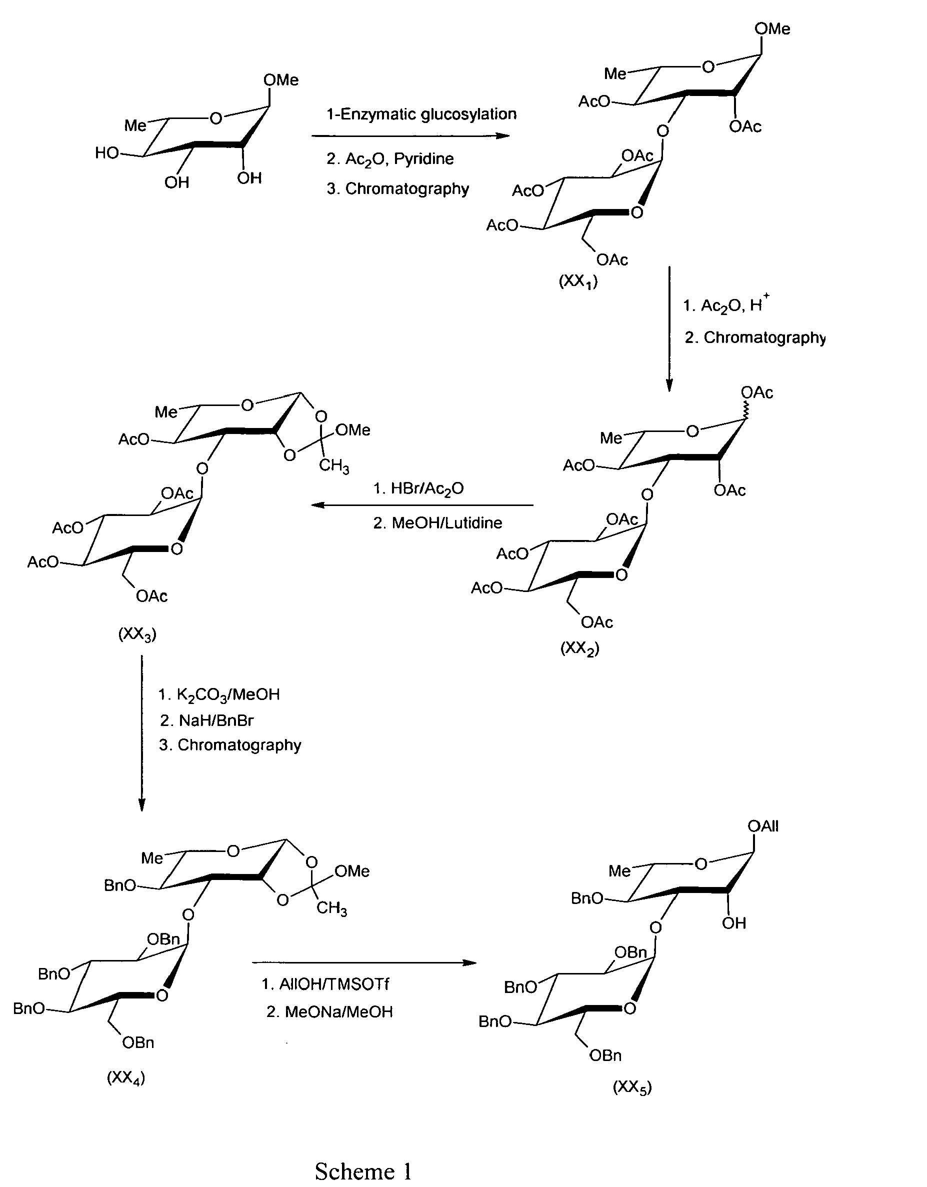
- methyl glycosidation of L-Rhamnose,
- enzymatic glucosylation by treatment of methyl α-L-rhamnopyranoside used as an acceptor with a mutant of amylosucrase
- advantageously rough purification, preferably by flash chromatography
- acetylation
- advantageously purification
- acetolysis
- advantageously purification
- anomeric bromination
- cyclization into an orthoester preferably with allyl alcohol,
- deacetylation, possibly under basic conditions
- protection of the hydroxyl groups, preferentially as ethers
- purification preferably by crystallisation
- orthoester opening under acid catalysis to give a glycoside or hemiacetal T
- selective deacetylation

9. A mutant of a wild type glycoside hydrolase,
wherein said wild type glycoside hydrolase has 450 to 850 amino acids and comprises from the N- to C-terminus eleven motifs defined by the following consensus sequences:
(1) the amino acid sequence LGVNYLHLMPL (SEQ ID NO: 1), which is located in the β-strand 2 of said wild type glycoside hydrolase;
(2) the amino acid sequence DGGYAV (SEQ ID NO: 2), which is located in the loop 2 of the (β/α)₈-barrel of said wild type glycoside hydrolase;
(3) the amino acid sequence DFVFNH (SEQ ID NO: 3) which is located in the β-strand 3 of said wild type glycoside hydrolase;
(4) the amino acid sequence LREIFPDTAPGNF (SEQ ID NO: 4), which is located in the domain B of said wild type glycoside hydrolase;
(5) the amino acid sequence FNSYQWDLN (SEQ ID NO: 5), which is located in the C-terminal part of the domain B of said wild type glycoside hydrolase;
(6) the amino acid sequence ILRLDAVAFLWK (SEQ ID NO: 6), which is located in the β-strand 4 of said wild type glycoside hydrolase;
(7) the amino acid sequence EAIV (SEQ ID NO: 7), which is located in the β-strand 5 of said wild type glycoside hydrolase;
(8) the amino acid sequence YVRCHDDI (SEQ ID NO: 8), which is located in the β-strand 7 of said wild type glycoside hydrolase;
(9) the amino acid sequence RISGTLASLAG (SEQ ID NO: 9), which is located in the domain B' of said wild type glycoside hydrolase;
(10) the amino acid sequence GIPLIYLGDE (SEQ ID NO: 10), which is located in the β-strand 8 of said wild type glycoside hydrolase;
(11) the amino acid sequence RWVHRP (SEQ ID NO: 11), which is located in the loop 8 of the (β/α)₈-barrel,
and the sequence formed by said eleven motifs joined end-to-end from motif (1) to motif (11) of said wild type glycoside hydrolase has at least 65% sequence identity or at least 80% sequence similarity with with the amino acid sequence SEQ ID NO: 12;
**characterized in that** said mutant has a mutation consisting of:
- the substitution of the amino acid residue at position 4 in said motif (4) with any amino acid selected from the group consisting of lysine (K) and arginine (R), or
- the substitution of the amino acid residue at position 9 in said motif (6) with any amino acid selected from the group consisting of cysteine (C), glutamic acid (E), isoleucine (I) and valine (V).

10. A mutant according to claim 9, **characterized in that** said wild type glycoside hydrolase is an amylosucrase (EC 2.4.1.4) or sucrose hydrolase (EC 3.2.1.-).

11. A mutant according to claim 10, **characterized in that** said wild type glycoside hydrolase is an amylosucrase from *Neisseria polysaccharea,* and is preferably selected from the group consisting of 1G5A, 1ZS2, 1 MVY, 1MW0, 1S46, 1JGI, 1MW2, 1MW3, 1MW1 and 1JG9 proteins.

12. A polynucleotide encoding a mutant of wild type glycoside hydrolase of anyone of claims 9 to 11.

13. A recombinant vector comprising a polynucleotide of claim 12.

14. Molecule belonging to the following list:
